# EUROPEAN PATENT APPLICATION

(11) **EP 4 546 456 A1**
(43) Date of publication of application: **30.04.2025**
(21) Application number: 23827186.0
(22) Date of filing: 20.06.2023
(51) Int. Cl.: H01M 4/36, C07D 213/89, H01M 4/13, H01M 4/139, H01M 4/62, H01M 4/66

(54) **SURFACE TREATMENT AGENT FOR ELECTRODE MATERIAL, POSITIVE ELECTRODE ACTIVE MATERIAL, CURRENT COLLECTOR FOIL, NEGATIVE ELECTRODE ACTIVE MATERIAL, CONDUCTIVE AID, ELECTRODE, BATTERY, METHOD FOR MANUFACTURING POSITIVE ELECTRODE ACTIVE MATERIAL, METHOD FOR MANUFACTURING CURRENT COLLECTOR FOIL, METHOD FOR MANUFACTURING NEGATIVE ELECTRODE ACTIVE MATERIAL, METHOD FOR MANUFACTURING CONDUCTIVE AID, AND METHOD FOR MANUFACTURING ELECTRODE**

(30) Priority: 24.06.2022 JP 2022101883
(71) Applicant: DAIKIN INDUSTRIES, LTD., Osaka-shi, Osaka 530-0001 (JP)
(72) Inventor: FUJIWARA, Kae, Osaka-Shi, Osaka 530-0001 (JP); YAMAZAKI, Shigeaki, Osaka-Shi, Osaka 530-0001 (JP); TERADA, Junpei, Osaka-Shi, Osaka 530-0001 (JP); JAKELSKI, Rene, 40211 Dusseldorf (DE); BRESSER, Dominic, 76131 Karlsruhe (DE); KUNZEL, Matthias, 76131 Karlsruhe (DE); KAZZAZI, Arefeh, 76131 Karlsruhe (DE); KUMAR, Vidur, 76131 Karlsruhe (DE); PASSERINI, Stefano, 76131 Karlsruhe (DE)
(74) Representative: Hoffmann Eitle
(86) International application number: PCT/JP2023/022714
(87) International publication number: WO 2023/249003

(57) **Abstract**

The disclosure aims to provide a surface-treating agent for an electrode material, a positive electrode active material, a current collector foil, a negative electrode active material, an electrode, a battery, a method for producing a positive electrode active material, a method for producing a current collector foil, a method for producing a negative electrode active material, a method for producing a conductive aid, and a method for producing an electrode which are capable of improving the cycle characteristics of batteries and preventing or reducing corrosion of current collector foils. The disclosure relates to a surface-treating agent for an electrode material, including a pyridine skeleton and fluorine.

## Description

### TECHNICAL FIELD

The disclosure relates to surface-treating agents for electrode materials, positive electrode active materials, current collector foils, negative electrode active materials, conductive aids, electrodes, batteries, methods for producing positive electrode active materials, methods for producing current collector foils, methods for producing negative electrode active materials, methods for producing conductive aids, and methods for producing electrodes.

### BACKGROUND ART

Current electric appliances demonstrate a tendency to have a reduced weight and a smaller size, which leads to development of electrochemical devices such as lithium-ion secondary batteries having a high energy density. Further, electrochemical devices such as lithium-ion secondary batteries are desired to have improved characteristics as they are applied to more various fields. Improvement in battery characteristics will become more and more important particularly when lithium-ion secondary batteries are put in use for automobiles.

As a technique for improving battery characteristics, for example, Patent Literature 1 discloses a current collector for an electrode, made of aluminum and with fluorine present on its surface.

### CITATION LIST

### - Patent Literature

Patent Literature 1: JP 2017-183256 A

### SUMMARY OF INVENTION

### - Technical Problem

The disclosure aims to provide a surface-treating agent for an electrode material, a positive electrode active material, a current collector foil, a negative electrode active material, a conductive aid, an electrode, a battery, a method for producing a positive electrode active material, a method for producing a current collector foil, a method for producing a negative electrode active material, a method for producing a conductive aid, and a method for producing an electrode, which are capable of improving the cycle characteristics of batteries and preventing or reducing corrosion of current collector foils.

### - Solution to Problem

The disclosure (1) relates to a surface-treating agent for an electrode material, including a pyridine skeleton and fluorine.

The disclosure (2) relates to the surface-treating agent for an electrode material according to the disclosure (1), which is an N-fluoropyridinium compound or a fluorinated halogen-pyridine-HF complex.

The disclosure (3) relates to the surface-treating agent for an electrode material according to the disclosure (2), wherein the N-fluoropyridinium compound is 1,1'-difluoro-2,2'-bipyridinium bis(tetrafluoroborate) and the fluorinated halogen-pyridine-HF complex is an IF₅-pyridine-HF complex.

The disclosure (4) relates to the surface-treating agent for an electrode material according to any one of the disclosures (1) to (3), which is used for a positive electrode active material, a current collector foil, a negative electrode active material, a conductive aid, or an electrode.

The disclosure (5) relates to a positive electrode active material treated with the surface-treating agent for an electrode material according to any one of the disclosures (1) to (4).

The disclosure (6) relates to a current collector foil treated with the surface-treating agent for an electrode material according to any one of the disclosures (1) to (4).

The disclosure (7) relates to a negative electrode active material treated with the surface-treating agent for an electrode material according to any one of the disclosures (1) to (4).

The disclosure (8) relates to a conductive aid treated with the surface-treating agent for an electrode material according to any one of the disclosures (1) to (4).

The disclosure (9) relates to an electrode including at least one selected from the group consisting of the positive electrode active material according to the disclosure (5), the current collector foil according to the disclosure (6), and the conductive aid according to the disclosure (8).

The disclosure (10) relates to an electrode including at least one selected from the group consisting of the negative electrode active material according to the disclosure (7), the current collector foil according to the disclosure (6), and the conductive aid according to the disclosure (8).

The disclosure (11) relates to a battery including the electrode according to the disclosure (9) or (10).

The disclosure (12) relates to a method for producing the positive electrode active material according to the disclosure (5), the method including bringing a positive electrode active material into contact with a solution containing the surface-treating agent for an electrode material according to any one of the disclosures (1) to (4).

The disclosure (13) relates to a method for producing the current collector foil according to the disclosure (6), the method including bringing a current collector foil into contact with a solution containing the surface-treating agent for an electrode material according to any one of the disclosures (1) to (4).

The disclosure (14) relates to a method for producing the negative electrode active material according to the disclosure (7), the method including bringing a negative electrode active material into contact with a solution containing the surface-treating agent for an electrode material according to any one of the disclosures (1) to (4).

The disclosure (15) relates to a method for producing the conductive aid according to the disclosure (8), the method including bringing a conductive aid into contact with a solution containing the surface-treating agent for an electrode material according to any one of the disclosures (1) to (4).

The disclosure (16) relates to a method for producing an electrode, the method including bringing an electrode including a current collector foil and a positive electrode active material or a negative electrode active material into contact with a solution containing the surface-treating agent for an electrode material according to any one of the disclosures (1) to (4).

### - Advantageous Effects of Invention

The disclosure can provide a surface-treating agent for an electrode material, a positive electrode active material, a current collector foil, a negative electrode active material, a conductive aid, an electrode, a battery, a method for producing a positive electrode active material, a method for producing a current collector foil, a method for producing a negative electrode active material, a method for producing a conductive aid, and a method for producing an electrode, which are capable of improving the cycle characteristics of batteries and preventing or reducing corrosion of current collector foils.

### DESCRIPTION OF EMBODIMENTS

The disclosure will be specifically described hereinbelow.

### <Surface-treating agent for an electrode material>

The disclosure relates to a surface-treating agent for an electrode material, including a pyridine skeleton and fluorine.

The surface of an electrode active material or a current collector, for example, is fluorinated by treatment with the surface-treating agent for an electrode material. Fluorine on the surface and the surface-treating agent for an electrode material remaining on the surface, whether reacted or unreacted, together form a stable protective film, which is believed to exhibit an effect of improving cycle characteristics of batteries (e.g., capacity retention after cycles) and an effect of preventing or reducing corrosion of current collector foils.

Moreover, the remaining surface-treating agent for an electrode material is expected to give an effect of improving ion conductivity.

Thus, high-performance batteries with high energy densities can be provided.

The surface-treating agent for an electrode material is advantageously excellent in safety and handleability, as compared with the method of fluorinating an object with fluorine gas as in examples of Patent Literature 1.

The surface-treating agent for an electrode material includes a pyridine skeleton and fluorine.

The number of pyridine skeletons in the surface-treating agent for an electrode material may be one or two or more. The number of pyridine skeletons is preferably 5 or less, more preferably 3 or less, still more preferably 2 or less.

The number of fluorine atoms in the surface-treating agent for an electrode material may be one or two or more, and is preferably 2 or more. The number of fluorine atoms is preferably 20 or less, more preferably 12 or less, still more preferably 8 or less.

The surface-treating agent for an electrode material used is suitably an N-fluoropyridinium compound or a fluorinated halogen-pyridine-HF complex.

The N-fluoropyridinium compound used is suitably a compound represented by the following formula (A1): 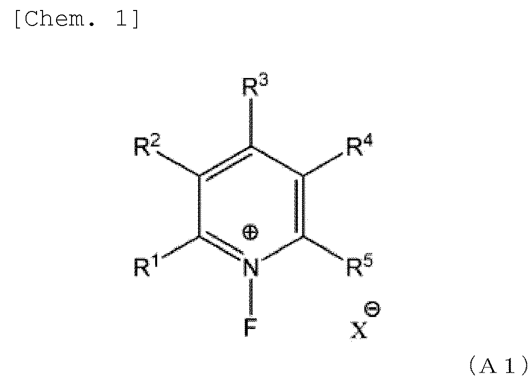 or a compound represented by the following formula (A2):

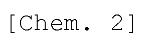

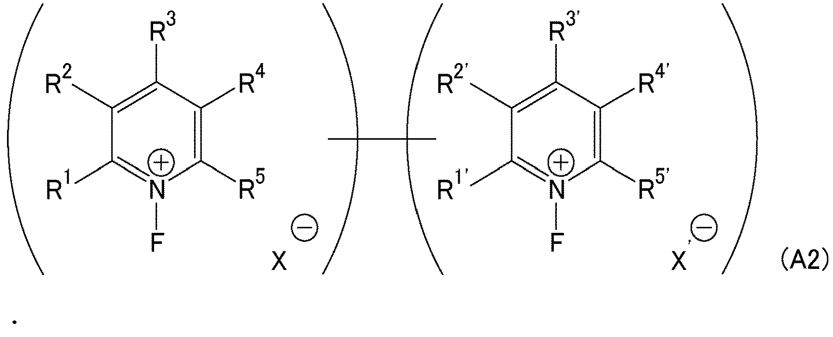

In the formulas (A1) and (A2),
R¹ and R² adjacent to each other, R² and R³ adjacent to each other, R³ and R⁴ adjacent to each other, or R⁴ and R⁵ adjacent to each other may be linked to form -CR⁶=CR⁷-CR⁸=CR⁹-, and
R¹' and R²', R²' and R³', R³' and R⁴', or R⁴' and R⁵' may be linked to form -CR⁶'=CR⁷'-CR⁸'=CR⁹'-.
R¹, R², R³, R⁴, R⁵, R⁶, R⁷, R⁸, R⁹, R¹', R²', R³', R⁴', R⁵', R⁶', R⁷', R⁸', and R⁹' may be the same as or different from each other and are each a hydrogen atom; a halogen atom; a nitro group; a hydroxy group; a cyano group; a carbamoyl group; a C1-C15 alkyl group optionally substituted with at least one selected from the group consisting of a halogen atom, a hydroxy group, a C1-C5 alkoxy group, a C6-C10 aryloxy group, a C2-C5 acyl group (e.g., alkanoyl group), a C2-C5 acyloxy group (e.g., alkanoyloxy group), and a C6-C10 aryl group; a C1-C15 alkenyl group optionally substituted with at least one selected from the group consisting of a halogen atom and a C6-C10 aryl group; a C1-C15 alkynyl group optionally substituted with at least one selected from the group consisting of a halogen atom and a C6-C10 aryl group; a C6-C15 aryl group optionally substituted with at least one selected from the group consisting of a halogen atom and a C1-C5 alkyl group; a C1-C15 acyl group (e.g., alkanoyl group) optionally substituted with at least one halogen atom; a C2-C15 alkoxy carbonyl group optionally substituted with at least one selected from the group consisting of a halogen atom and a C6-C10 aryl group; a C7-C15 aryloxy carbonyl group optionally substituted with at least one selected from the group consisting of a halogen atom and a C1-C5 alkyl group; a C1-C15 alkylsulfonyl group optionally substituted with at least one selected from the group consisting of a halogen atom and a C6-C10 aryl group; a C6-C15 arylsulfonyl group optionally substituted with at least one selected from the group consisting of a halogen atom and a C1-C5 alkyl group; a C1-C15 alkylsulfinyl group optionally substituted with at least one selected from the group consisting of a halogen atom and a C6-C10 aryl group; a C6-C15 arylsulfinyl group optionally substituted with at least one selected from the group consisting of a halogen atom and a C1-C5 alkyl group; a C1-C15 alkoxy group optionally substituted with at least one selected from the group consisting of a halogen atom and a C6-C10 aryl group; a C6-C15 aryloxy group optionally substituted with at least one selected from the group consisting of a halogen atom and a C1-C5 alkyl group; a C1-C15 acyloxy group (e.g., alkanoyloxy group) optionally substituted with at least one halogen atom; a C1-C15 acylthio group (e.g., alkanoylthio group) optionally substituted with at least one halogen atom; a C1-C15 alkanesulfonyloxy group optionally substituted with at least one selected from the group consisting of a halogen atom and a C6-C10 aryl group; a C6-C15 arylsulfonyloxy group optionally substituted with at least one selected from the group consisting of a halogen atom and a C1-C5 alkyl group; a carbamoyl group optionally substituted with at least one selected from the group consisting of a C1-C5 alkyl group and a C6-C10 aryl group; an amino group optionally substituted with at least one selected from the group consisting of a C1-C5 acyl group (e.g., alkanoyl group) and a halogen atom; a C6-C15 N-alkylpyridinium salt group optionally substituted with at least one selected from the group consisting of a halogen atom, a C6-C10 aryl group, and a C1-C5 alkyl group; a C11-C15 N-arylpyridinium salt group optionally substituted with at least one selected from the group consisting of a halogen atom, a C6-C10 aryl group, and a C1-C5 alkyl group; or an organic polymer chain. R¹, R², R³, R⁴, R⁵, R⁶, R⁷, R⁸, R⁹, R¹', R²', R³', R⁴', R⁵', R⁶', R⁷', R⁸', and R⁹' may form, in various combinations, a ring structure with or without a hetero atom.

One of R¹, R², R³, R⁴, R⁵, R⁶, R⁷, R⁸, and R⁹ and one of R¹', R²', R³', R⁴', R⁵', R⁶', R⁷', R⁸', and R⁹' in the formula (A2) are bonded to each other via a single bond to form a bonding chain.

Moreover,

[Chem. 3] X^{⊝} and X'^{⊝}

are the same as or different from each other and each are a conjugate base of a Brønsted acid.

Examples of the Brønsted acid include: sulfonic acids such as methanesulfonic acid, butanesulfonic acid, benzenesulfonic acid, toluenesulfonic acid, nitrobenzenesulfonic acid, dinitrlbenzenesulfonic acid, trinitrobenzenesulfonic acid, trifluoromethanesulfonic acid, trifluoroethanesulfonic acid, perfluorobutanesulfonic acid, perfluorooctanesulfonic acid, perfluoro(2-ethoxyehane)sulfonic acid, perfluoro(4-ethylcyclohexane)sulfonic acid, trichloromethanesulfonic acid, difluoromethanesulfonic acid, trifluoroethanesulfonic acid, fluorosulfonic acid, chlorosulfonic acid, camphorsulfonic acid, bromocamphorsulfonic acid, Δ⁴-cholesten-3-one-6-sulfonic acid, 1-hydroxy-p-methane-2-sulfonic acid, p-styrenesulfonic acid, β-styrenesulfonic acid, vinylsulfonic acid, perfluoro-3,6-dioxa-4-methyl-7-octenesulfonic acid; mineral acids such as sulfuric acid, phosphoric acid, and nitric acid; halogenated acids such as perchloric acid, perbromic acid, periodic acid, chloric acid, and bromic acid; monoalkylsulfuric acids such as monomethyl sulfuric acid and monoethyl sulfuric acid; carboxylic acids such as acetic acid, formic acid, trichloroacetic acid, trifluoroacetic acid, pentafluoropropionic acid, dichloroacetic acid, and acrylic acid; compounds of Lewis acids and hydrogen halides such as HAlF₄, HBF₄, HB₂F₇, HPF₆, HSbF₄, HSbF₆, HSb₂F₁₁, HAsF₆, HAlCl₄, HAlCl₃F, HAlF₃Cl, HBCl₄, HBCl₃F, HBBr₃F, HSbCl₆, and HSbCl₅F; aryl-substituted boron compounds such as HBPh₄ (Ph represents a phenyl group; the same applies hereinbelow) and 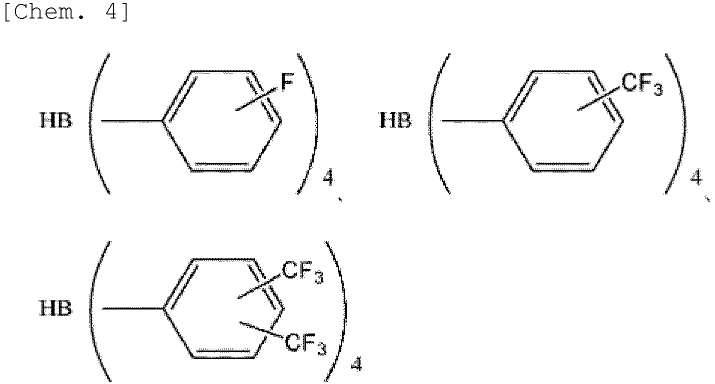 ; acid amide compounds such as (FSO₂)₂NH, (PhSO₂)₂NH, (CF₃SO₂)₂NH, (C₂F₅SO₂)₂NH, (C₄F₉SO₂)₂NH, (HCF₂CF₂SO₂)₂NH, CF₃SO₂NHSO₂C₆F₁₃, and 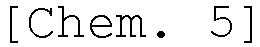 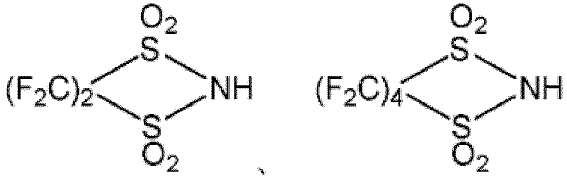 ; and carbonate compounds such as (FSO₂)₃CH, (CF₃SO₂)₃CH, (PhOSO₂)₃CH (Ph represents a phenyl group), (CF₃SO₂)₂CH₂, (CF₃SO₂)₃CH, (C₄F₉SO₂)₃CH, and (C₈F₁₇SO₂)₃CH.

The Brønsted acid is particularly preferably a Brønsted acid with a stronger acidity than acetic acid (pKa: 4.56) in terms of compound stability.

The conjugate base of the Brønsted acid is particularly preferably, for example, ⁻BF₄, ⁻PF₆, ⁻AsF₆, ⁻ SbF₆, ⁻AlF₄, ⁻AlCl₄, ⁻SbCl₆, ⁻SbCl₅F, ⁻Sb₂F₁₁, ⁻B₂F₇, ⁻OClO₃, ⁻ OSO₂F, ⁻OSO₂Cl, ⁻OSO₂OH, ⁻OSO₂OCH₃, ⁻OSO₂CH₃, ⁻OSO₂CF₃, ⁻ OSO₂CCl₃, ⁻OSO₂C₄F₉, ⁻OSO₂C₆H₅, ⁻OSO₂C₆H₄CH₃, ⁻OSO₂C₆H₄NO₂, or ⁻ N(SO₂CF₃)₂. Preferred among these is tetrafluoroborate (⁻ BF₄) or a perfluoroalkanesulfonate (e.g., ⁻OSO₂CF₃, ⁻ OSO₂C₄F₉), and more preferred is tetrafluoroborate (⁻BF₄).

The compound represented by the formula (A1) is preferably a compound represented by the following formula (A1a): 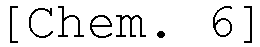 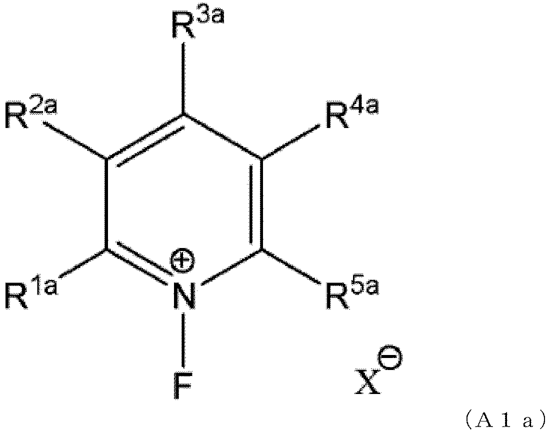 wherein R^{1a}, R^{2a}, R^{3a}, R^{4a}, and R^{5a} are the same as or different from each other and each represent a hydrogen atom, a C1-C4 alkyl group, a C1-C4 haloalkyl group, a halogen atom, a phenyl group optionally substituted with a methyl group or a halogen atom, a C1-C4 alkoxy group, a C2-C4 acyl group (e.g., alkanoyl group), a C2-C4 acyloxy group (e.g., alkanoyloxy group), a C2-C4 alkoxycarbonyl group, a cyano group, or a nitro group; and

[Chem. 7] X^{⊝}

represents a conjugate base of a Brønsted acid with a pKa of 4.56 or lower.

The compound represented by the formula (A2) is preferably a compound represented by the following formula (A2a): 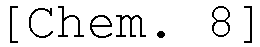 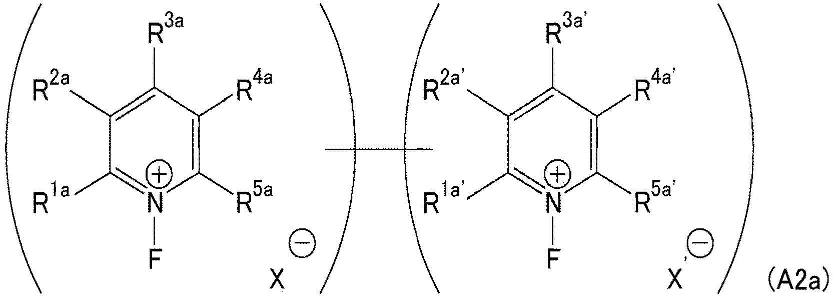 wherein R^{1a}, R^{2a}, R^{3a}, R^{4a}, R^{5a}, R^{1a}', R^{2a}', R^{3a}', R^{4a}', and R^{5a}' are the same as or different from each other and each represent a hydrogen atom, a C1-C4 alkyl group, a C1-C4 haloalkyl group, a halogen atom, a phenyl group optionally substituted with a methyl group or a halogen atom, a C1-C4 alkoxy group, a C2-C4 acyl group (e.g., alkanoyl group), a C2-C4 acyloxy group (e.g., alkanoyloxy group), a C2-C4 alkoxycarbonyl group, a cyano group, or a nitro group, and one of R^{1a}, R^{2a}, R^{3a}, R^{4a}, and R^{5a} and one of R^{1a}', R^{2a}', R^{3a}', R^{4a}', and R^{5a}' are bonded to each other via a single bond to form a bonding chain; and

[Chem. 9] X^{⊝} and/or X'^{⊝}

represents a conjugate base of a Brønsted acid with a pKa of 4.56 or lower.

In the compounds represented by the formulas (A1a) and (A2a), the higher the electron-donating property of R^{1a}, R^{2a}, R^{3a}, R^{4a}, R^{5a}, R^{1a}', R^{2a}', R^{3a}', R^{4a}', and R^{5a}', the higher the stability, and the higher the electron-withdrawing property of R^{1a}, R^{2a}, R^{3a}, R^{4a}, R^{5a}, R^{1a}', R^{2a}', R^{3a}', R^{4a}', and R^{5a}', the higher the reactivity. From this standpoint, the compound can be appropriately selected in accordance with the desired properties.

For a higher effect of improving the cycle characteristics of batteries and for a higher effect of preventing or reducing corrosion of current collector foils, the N-fluoropyridinium compound is preferably a compound represented by the formula (A2), more preferably a compound represented by the formula (A2a), still more preferably 1,1'-difluoro-2,2'-bipyridinium bis(tetrafluoroborate).

The fluorinated halogen used in the fluorinated halogen-pyridine-HF complex may be, for example, a compound represented by the formula XFₙ wherein X represents chlorine, bromine, or iodine and n is a natural number of 1 to 5. X is preferably iodine or bromine, more preferably iodine. n is preferably 3 to 5, particularly preferably 5.

The number of fluorinated halogens in the fluorinated halogen-pyridine-HF complex may be one or two or more, and is preferably one. The same applies to pyridine and HF.

For a higher effect of improving the cycle characteristics of batteries and for a higher effect of preventing or reducing corrosion of current collector foils, the fluorinated halogen-pyridine-HF complex is preferably an IF₅-pyridine-HF complex.

One type of the surface-treating agent for an electrode material may be used, or two or more types thereof may be used in combination.

The surface-treating agent for an electrode material can be used for a positive electrode active material, a current collector foil, a negative electrode active material, a conductive aid, or an electrode.

The disclosure relates to a positive electrode active material, a current collector foil, a negative electrode active material, a conductive aid, and an electrode each treated with the surface-treating agent for an electrode material.

### <Positive electrode active material>

The surface of the positive electrode active material is fluorinated by treatment with the surface-treating agent for an electrode material. The percentage of fluorine on the surface of the positive electrode active material is preferably 2.5 atom% or higher, more preferably 3.2 atom% or higher, still more preferably 3.6 atom% or higher, while preferably 6.5 atom% or lower, more preferably 5.5 atom% or lower, still more preferably 4.5 atom% or lower.

The percentage of fluorine on the surface of the positive electrode active material can be measured by energy dispersive X-ray spectroscopy using a scanning electron microscope (SEM-EDX).

The unreacted/reacted surface-treating agent for an electrode material remaining on the surface of the positive electrode active material forms a stable protective film. The amount (total amount) of the unreacted/reacted surface-treating agent for an electrode material remaining on the surface of the positive electrode active material is preferably 0.01% by mass or more, more preferably 0.1% by mass or more, still more preferably 0.15% by mass or more, while preferably 5.0% by mass or less, more preferably 1.0% by mass or less, still more preferably 0.3% by mass or less.

The amount of the unreacted/reacted surface-treating agent for an electrode material remaining on the surface of the positive electrode active material can be calculated from the difference in mass of the positive electrode active material before and after the treatment as weighed by a precision electronic balance.

The positive electrode active material may be any material that can electrochemically occlude and release lithium ions. Examples thereof include lithium-containing transition metal complex oxides, lithium-containing transition metal phosphoric acid compounds, sulfur-based materials, and conductive polymers. Preferred among these as the positive electrode active material are lithium-containing transition metal complex oxides and lithium-containing transition metal phosphoric acid compounds. Particularly preferred is a lithium-containing transition metal complex oxide that generates high voltage.

In use of a high-voltage material, occurrence of oxidative decomposition of the electrolyte solution, elution of metal ions from the positive electrode active material, and corrosion of current collector foils are concerned. Use of the surface-treating agent for an electrode material can prevent or reduce occurrence of these. In other words, the surface-treating agent for an electrode material is particularly suitable for high-voltage materials.

The term "high voltage" herein means a voltage of 4.2 V or higher. The upper limit of the "high voltage" is preferably 4.9 V.

The transition metal of the lithium-containing transition metal complex oxide is preferably V, Ti, Cr, Mn, Fe, Co, Ni, Cu, or the like. Specific examples thereof include lithium-cobalt complex oxides such as LiCoO₂, lithium-nickel complex oxides such as LiNiO₂, lithium-manganese complex oxides such as LiMnO₂, LiMn₂O₄, and Li₂MnO₄, and those obtained by substituting some of transition metal atoms as main components of these lithium transition metal complex oxides with another element such as Na, K, B, F, Al, Ti, V, Cr, Mn, Fe, Co, Li, Ni, Cu, Zn, Mg, Ga, Zr, Si, Nb, Mo, Sn, or W. Specific examples of those obtained by substitution include LiNi_{0.5}Mn_{0.5}O₂, LiNi_{0.85}Co_{0.10}Al_{0.05}O₂, LiNi_{0.5}Co_{0.2}Mn_{0.3}O₂, LiNi_{0.6}Co_{0.2}Mn_{0.2}O₂, LiNi_{0.33}Co_{0.33}Mn_{0.33}O₂, LiNi_{0.8}Co_{0.1}Mn_{0.1}O₂, LiNi_{0.45}Co_{0.10}Al_{0.45}O₂, LiMn_{1.8}Al_{0.2}O₄, and LiNi_{0.5}Mn_{1.5}O₄.

The lithium-containing transition metal complex oxide is preferably any of LiNi_{0.5}Mn_{1.5}O₄, LiNi_{0.5}Co_{0.2}Mn_{0.3}O₂, and LiNi_{0.6}Co_{0.2}Mn_{0.2}O₂ each of which has a high energy density even at high voltage. In particular, LiNi_{0.5}Mn_{1.5}O₄ is preferred at a high voltage of 4.4 V or higher.

In order to provide a high-capacity lithium-ion secondary battery, the lithium-containing transition metal complex oxide is preferably LiNi_{0.6}Co_{0.2}Mn_{0.2}O₂, LiNi_{0.8}Co_{0.1}Mn_{0.1}O₂, or LiNi_{0.85}Co_{0.10}Al_{0.05}O₂.

The transition metal of the lithium-containing transition metal phosphoric acid compound is preferably V, Ti, Cr, Mn, Fe, Co, Ni, Cu, or the like. Specific examples thereof include iron phosphates such as LiFePO₄, Li₃Fe₂(PO₄)₃, and LiFeP₂O₇, cobalt phosphates such as LiCoPO₄, and those obtained by substituting some of transition metal atoms as main components of these lithium transition metal phosphoric acid compounds with another element such as Al, Ti, V, Cr, Mn, Fe, Co, Li, Ni, Cu, Zn, Mg, Ga, Zr, Nb, or Si.

Examples of the lithium-containing transition metal complex oxide include
lithium-manganese spinel complex oxides represented by the formula: LiₐMn_{2-b}M¹_{b}O₄ (wherein 0.9 ≤ a; 0 ≤ b ≤ 1.5; and M¹ is at least one metal selected from the group consisting of Fe, Co, Ni, Cu, Zn, Al, Sn, Cr, V, Ti, Mg, Ca, Sr, B, Ga, In, Si, and Ge),
lithium-nickel complex oxides represented by the formula: LiNi_{1-c}M²_{c}O₂ (wherein 0 ≤ c ≤ 0.5; and M² is at least one metal selected from the group consisting of Fe, Co, Mn, Cu, Zn, Al, Sn, Cr, V, Ti, Mg, Ca, Sr, B, Ga, In, Si, and Ge), and
lithium-cobalt complex oxides represented by the formula: LiCo_{1-d}M³_{d}O₂ (wherein 0 ≤ d ≤ 0.5; and M³ is at least one metal selected from the group consisting of Fe, Ni, Mn, Cu, Zn, Al, Sn, Cr, V, Ti, Mg, Ca, Sr, B, Ga, In, Si, and Ge).

In order to provide a high-power lithium-ion secondary battery having a high energy density, preferred is LiCoO₂, LiMnO₂, LiNiO₂, LiMn₂O₄, LiNi_{0.8}Co_{0.15}Al_{0.05}O₂, or LiNi_{1/3}Co_{1/3}Mn_{1/3}O₂.

Other examples of the positive electrode active material include LiFePO₄, LiNi_{0.8}Co_{0.2}O₂, Li_{1.2}Fe_{0.4}Mn_{0.4}O₂, LiNi_{0.5}Mn_{0.5}O₂, LiV₃O₆, and Li₂MnO₃.

An example of the sulfur-based material is a sulfur-containing material. The sulfur-based material preferably includes at least one selected from the group consisting of an elemental sulfur, a metallic sulfide, and an organic sulfur compound. The sulfur-based material is more preferably an elemental sulfur. The metallic sulfide may be a metal polysulfide. The organic sulfur compound may be an organic polysulfide.

Examples of the metal sulfides include compounds represented by LiSₓ (0 < x ≤ 8), compounds represented by Li₂Sₓ (0 < x ≤ 8), compounds having a 2D lamellar structure such as TiS₂ and MoS₂, and Chevrel compounds having a strong 3D skeletal structure such as those represented by the formula: MeₓMo₆S₈ (wherein Me is a transition metal such as Pb, Ag, or Cu).

An example of the organic sulfur compound is a carbon sulfide compound.

The organic sulfur compound may be carried on a porous material such as carbon and used as a carbon composite material. In order to achieve further excellent cycle performance and further reduce overvoltage, the carbon composite material preferably contains sulfur in an amount of 10 to 99% by mass, more preferably 20% by mass or more, still more preferably 30% by mass or more, particularly preferably 40% by mass or more, and preferably 85% by mass or less relative to the carbon composite material.

When the positive electrode active material is an elemental sulfur, the amount of sulfur in the positive electrode active material equals the amount of the elemental sulfur.

Examples of the conductive polymers include p-doped conductive polymers and n-doped conductive polymers. Examples of the conductive polymers include polyacetylene-based polymers, polyphenylene-based polymers, heterocyclic polymers, ionic polymers, ladder-shaped polymers, and network polymers.

In order to improve the continuous charge characteristics, the positive electrode active material preferably contains lithium phosphate. Lithium phosphate may be used in any manner, and is preferably used in admixture with the positive electrode active material. The lower limit of the amount of lithium phosphate used is preferably 0.1% by mass or more, more preferably 0.3% by mass or more, still more preferably 0.5% by mass or more, relative to the sum of the amounts of the positive electrode active material and lithium phosphate. The upper limit thereof is preferably 10% by mass or less, more preferably 8% by mass or less, still more preferably 5% by mass or less.

To a surface of the positive electrode active material may be attached a substance having a composition different from the positive electrode active material. Examples of the substance attached to the surface include oxides such as aluminum oxide, silicon oxide, titanium oxide, zirconium oxide, magnesium oxide, calcium oxide, boron oxide, antimony oxide, and bismuth oxide; sulfates such as lithium sulfate, sodium sulfate, potassium sulfate, magnesium sulfate, calcium sulfate, and aluminum sulfate; carbonates such as lithium carbonate, calcium carbonate, and magnesium carbonate; and carbon.

Such a substance may be attached to a surface of the positive electrode active material by, for example, a method of dissolving or suspending the substance in a solvent, impregnating the solution or suspension into the positive electrode active material, and drying the impregnated material; a method of dissolving or suspending a precursor of the substance in a solvent, impregnating the solution or suspension into the positive electrode active material, and heating the material and the precursor to cause a reaction therebetween; or a method of adding the substance to a precursor of the positive electrode active material and simultaneously sintering the materials. In the case of attaching carbon, for example, a carbonaceous material in the form of activated carbon may be mechanically attached to the surface afterward.

For the amount of the substance attached to the surface in terms of the mass relative to the amount of the positive electrode active material, the lower limit thereof is preferably 0.1 ppm or more, more preferably 1 ppm or more, still more preferably 10 ppm or more, while the upper limit thereof is preferably 20% or less, more preferably 10% or less, still more preferably 5% or less. The substance attached to the surface can reduce oxidation of the electrolyte solution on the surface of the positive electrode active material, improving the battery life. Too small an amount of the substance may fail to sufficiently provide this effect. Too large an amount thereof may hinder the entrance and exit of lithium ions, increasing the resistance.

Particles of the positive electrode active material may have any shape conventionally used, such as a bulky shape, a polyhedral shape, a spherical shape, an ellipsoidal shape, a plate shape, a needle shape, or a pillar shape. The primary particles may agglomerate to form secondary particles.

The positive electrode active material commonly has a tap density of preferably 1.5 g/cm³ or higher, more preferably 2.0 g/cm³ or higher, still more preferably 2.5 g/cm³ or higher, most preferably 3.0 g/cm³ or higher. The positive electrode active material having a tap density below the lower limit may cause an increased amount of a dispersion medium required and increased amounts of a conductive material and a binder required in formation of the positive electrode active material layer, as well as limitation on the packing fraction of the positive electrode active material in the positive electrode active material layer, resulting in limitation on the battery capacity. A metal complex oxide powder having a high tap density enables formation of a positive electrode active material layer with a high density. The tap density is preferably as high as possible and has no upper limit. The upper limit of the tap density is commonly 4.5 g/cm³ or lower, preferably 4.3 g/cm³ or lower.

In the disclosure, the tap density is determined as a powder packing density (tap density) g/cm³ when 5 to 10 g of the positive electrode active material powder is packed into a 10-ml glass graduated cylinder and the cylinder is tapped 200 times with a stroke of about 20 mm.

The particles of the positive electrode active material have a median size d50 (or a secondary particle size when the primary particles agglomerate to form secondary particles) of preferably 0.3 µm or greater, more preferably 0.5 µm or greater, still more preferably 0.8 µm or greater, most preferably 1.0 µm or greater, while preferably 30 µm or smaller, more preferably 27 µm or smaller, still more preferably 25 µm or smaller, most preferably 22 µm or smaller. The particles having a median size below the lower limit may fail to provide a product with a high tap density. The particles having a median size greater than the upper limit may cause prolonged diffusion of lithium in the particles, impairing the battery performance and generating streaks in formation of the positive electrode for a battery, i.e., when the active material and components such as a conductive material and a binder are formed into slurry by adding a solvent and the slurry is applied in the form of a film, for example. Mixing two or more positive electrode active materials having different median sizes d50 can further improve the easiness of packing in formation of the positive electrode.

In the disclosure, the median size d50 is determined using a known laser diffraction/scattering particle size distribution analyzer. In the case of using LA-920 (Horiba, Ltd.) as the particle size distribution analyzer, the dispersion medium used in the measurement is a 0.1% by mass sodium hexametaphosphate aqueous solution and the measurement refractive index is set to 1.24 after 5-minute ultrasonic dispersion.

When the primary particles agglomerate to form secondary particles, the average primary particle size of the positive electrode active material is preferably 0.05 µm or greater, more preferably 0.1 µm or greater, still more preferably 0.2 µm or greater. The upper limit thereof is preferably 5 µm or smaller, more preferably 4 µm or smaller, still more preferably 3 µm or smaller, most preferably 2 µm or smaller. The primary particles having an average primary particle size greater than the upper limit may have difficulty in forming spherical secondary particles, adversely affecting the powder packing. Further, such primary particles may have a greatly reduced specific surface area, highly possibly impairing the battery performance such as output characteristics. In contrast, the primary particles having an average primary particle size below the lower limit may usually be insufficiently grown crystals, causing poor charge and discharge reversibility, for example.

In the disclosure, the primary particle size is measured by scanning electron microscopic (SEM) observation. Specifically, the primary particle size is determined as follows. A photograph at a magnification of 10000x is first taken. Any 50 primary particles are selected and the maximum length between the left and right boundary lines of each primary particle is measured along the horizontal line. Then, the average value of the maximum lengths is calculated, which is defined as the primary particle size.

The positive electrode active material has a BET specific surface area of preferably 0.1 m²/g or larger, more preferably 0.2 m²/g or larger, still more preferably 0.3 m²/g or larger. The upper limit thereof is preferably 50 m²/g or smaller, more preferably 40 m²/g or smaller, still more preferably 30 m²/g or smaller. The positive electrode active material having a BET specific surface area smaller than the above range may easily impair the battery performance. The positive electrode active material having a BET specific surface area larger than the above range may less easily have an increased tap density, easily causing a difficulty in applying the material in formation of the positive electrode active material layer.

In the disclosure, the BET specific surface area is defined by a value determined by single point BET nitrogen adsorption utilizing a gas flow method using a surface area analyzer (e.g., fully automatic surface area measurement device, Ohkura Riken Co., Ltd.), a sample pre-dried in nitrogen stream at 150°C for 30 minutes, and a nitrogen-helium gas mixture with the nitrogen pressure relative to the atmospheric pressure being accurately adjusted to 0.3.

In the case of a large-size lithium-ion secondary battery for hybrid vehicles or distributed generation, it needs to achieve high output. Thus, the particles of the positive electrode active material are preferably mainly composed of secondary particles.

The particles of the positive electrode active material preferably include 0.5 to 7.0% by volume of fine particles having an average secondary particle size of 40 µm or smaller and having an average primary particle size of 1 µm or smaller. The presence of fine particles having an average primary particle size of 1 µm or smaller enlarges the contact area with the electrolyte solution and enables more rapid diffusion of lithium ions between the electrode and the electrolyte solution, improving the output performance of the battery.

The positive electrode active material may be produced by any usual method of producing an inorganic compound. In particular, a spherical or ellipsoidal active material can be produced by various methods. For example, a material substance of transition metal is dissolved or crushed and dispersed in a solvent such as water, and the pH of the solution or dispersion is adjusted under stirring to form a spherical precursor. The precursor is recovered and, if necessary, dried. Then, a Li source such as LiOH, Li₂CO₃, or LiNO₃ is added thereto and the mixture is sintered at high temperature, thereby providing an active material.

One positive electrode active material may be used alone or two or more thereof having different compositions may be used in any combination at any ratio. Preferred examples of the combination in this case include a combination of LiCoO₂ and LiMn₂O₄ in which part of Mn may optionally be replaced by a different transition metal (e.g., LiNi_{0.33}Co_{0.33}Mn_{0.33}O₂), and a combination with LiCoO₂ in which part of Co may optionally be replaced by a different transition metal.

### <Current collector foil>

The surface of the current collector foil is fluorinated by treatment with the surface-treating agent for an electrode material. The percentage of fluorine on the surface of the current collector foil is preferably 0.5 atom% or higher, more preferably 0.7 atom% or higher, still more preferably 0.9 atom% or higher, while preferably 3.5 atom% or lower, more preferably 2.5 atom% or lower, still more preferably 2.0 atom% or lower.

The percentage of fluorine on the surface of the current collector foil can be measured by energy dispersive X-ray spectroscopy using a scanning electron microscope (SEM-EDX).

The unreacted/reacted surface-treating agent for an electrode material remaining on the surface of the current collector foil forms a stable protective film. The amount of the unreacted/reacted surface-treating agent for an electrode material remaining on the surface of the current collector foil is normally very small and is hardly measurable.

Examples of the material of the current collector foil include metal materials such as aluminum, titanium, tantalum, stainless steel, nickel, and copper, and alloys thereof; and carbon materials such as carbon cloth and carbon paper. Preferred among these are metal materials. Aluminum or an alloy thereof is preferred for a positive electrode, and copper or an alloy thereof is preferred for a negative electrode.

In the case of the current collector foil made of aluminum, corrosion at high voltage is concerned. However, use of the surface-treating agent for an electrode material can prevent or reduce corrosion of aluminum.

The current collector foil may have any thickness, and the thickness is usually 1 µm or greater, preferably 3 µm or greater, more preferably 5 µm or greater, while usually 1 mm or smaller, preferably 100 µm or smaller, more preferably 50 µm or smaller. The film having a thickness smaller than the above range may have insufficient strength as a current collector. In contrast, the film having a thickness greater than the above range may have poor handleability.

In order to reduce the electric contact resistance between the current collector foil and the active material layer, the current collector foil also preferably has a conductive aid applied on the surface thereof. Examples of the conductive aid include carbon and noble metals such as gold, platinum, and silver.

The current collector foil of the disclosure is suitable for electrodes of batteries such as lithium-ion secondary batteries, and is also usable for electrodes of electrochemical devices other than batteries, such as electric double-layer capacitors.

### <Negative electrode active material>

The surface of the negative electrode active material is fluorinated by treatment with the surface-treating agent for an electrode material. The percentage of fluorine on the surface of the negative electrode active material is preferably 2.5 atom% or higher, more preferably 3.2 atom% or higher, still more preferably 3.6 atom% or higher, while preferably 8.5 atom% or lower, more preferably 7.5 atom% or lower, still more preferably 6.5 atom% or lower.

The percentage of fluorine on the surface of the negative electrode active material can be measured by energy dispersive X-ray spectroscopy using a scanning electron microscope (SEM-EDX).

The unreacted/reacted surface-treating agent for an electrode material remaining on the surface of the negative electrode active material forms a stable protective film. The amount (total amount) of the unreacted/reacted surface-treating agent for an electrode material remaining on the surface of the negative electrode active material is preferably 0.01% by mass or more, more preferably 0.05% by mass or more, still more preferably 0.1% by mass or more, while preferably 5.0% by mass or less, more preferably 2.0% by mass or less, still more preferably 0.5% by mass or less.

The amount of the unreacted/reacted surface-treating agent for an electrode material remaining on the surface of the negative electrode active material can be calculated from the difference in mass of the negative electrode active material before and after the treatment as weighed by a precision electronic balance.

The negative electrode material may be any material that can electrochemically occlude and release lithium ions. Specific examples of the negative electrode active material include carbonaceous materials that can occlude and release lithium such as pyrolysates of organic matter under various pyrolysis conditions, artificial graphite, and natural graphite; metal oxide materials that can occlude and release lithium such as tin oxide and silicon oxide; lithium metals; various lithium alloys; lithium-containing metal complex oxide materials; and conductive polymers. Two or more of these negative electrode active materials may be used in admixture with each other.

Fluorination of the surface of the negative electrode active material by the surface-treating agent for an electrode material and formation of a protective film on the surface of the negative electrode active material can prevent or reduce reductive decomposition of the electrolyte solution in each cycle of the battery, leading to an effect of preventing or reducing an increase in resistance.

The carbonaceous material that can occlude and release lithium is preferably artificial graphite produced by high-temperature treatment of easily graphitizable pitch from various materials, purified natural graphite, or a material obtained by surface treatment on such graphite with pitch or other organic matter and then carbonization of the surface-treated graphite. In order to achieve a good balance between the initial irreversible capacity and the high-current-density charge and discharge characteristics, the carbonaceous material is more preferably selected from carbonaceous materials obtained by heat-treating natural graphite, artificial graphite, artificial carbonaceous substances, or artificial graphite substances at 400°C to 3200°C once or more; carbonaceous materials which allow the negative electrode active material layer to include at least two or more carbonaceous matters having different crystallinities and/or have an interface between the carbonaceous matters having the different crystallinities; and carbonaceous materials which allow the negative electrode active material layer to have an interface between at least two or more carbonaceous matters having different orientations. One of these carbonaceous materials may be used alone or two or more thereof may be used in any combination at any ratio.

Examples of the carbonaceous materials obtained by heat-treating artificial carbonaceous substances or artificial graphite substances at 400°C to 3200°C once or more include coal-based coke, petroleum-based coke, coal-based pitch, petroleum-based pitch, and those prepared by oxidizing these pitches; needle coke, pitch coke, and carbon materials prepared by partially graphitizing these cokes; pyrolysates of organic matter such as furnace black, acetylene black, and pitch-based carbon fibers; carbonizable organic matter and carbides thereof; and solutions prepared by dissolving carbonizable organic matter in a low-molecular-weight organic solvent such as benzene, toluene, xylene, quinoline, or n-hexane, and carbides thereof.

The metal material (excluding lithium-titanium complex oxides) to be used as the negative electrode active material may be any compound that can occlude and release lithium, and examples thereof include simple lithium, simple metals and alloys that constitute lithium alloys, and oxides, carbides, nitrides, silicides, sulfides, and phosphides thereof. The simple metals and alloys constituting lithium alloys are preferably materials containing any of metal and semi-metal elements in Groups 13 and 14, more preferably simple metal of aluminum, silicon, and tin (hereinafter, referred to as "specific metal elements"), and alloys and compounds containing any of these atoms. One of these materials may be used alone or two or more thereof may be used in any combination at any ratio.

Examples of the negative electrode active material containing at least one atom selected from the specific metal elements include simple metal of any one specific metal element, alloys of two or more specific metal elements, alloys of one or two or more specific metal elements and one or two or more other metal elements, compounds containing one or two or more specific metal elements, and composite compounds such as oxides, carbides, nitrides, silicides, sulfides, and phosphides of the compounds. Such a simple metal, alloy, or metal compound used as the negative electrode active material can lead to a high-capacity battery.

Examples thereof further include compounds in which any of the above composite compounds are complexly bonded with several elements such as simple metals, alloys, and nonmetal elements. Specifically, in the case of silicon or tin, for example, an alloy of this element and a metal that does not serve as a negative electrode may be used. In the case of tin, for example, a composite compound including a combination of five or six elements, including tin, a metal (excluding silicon) that serves as a negative electrode, a metal that does not serve as a negative electrode, and a nonmetal element, may be used.

Specific examples thereof include simple Si, SiB₄, SiB₆, Mg₂Si, Ni₂Si, TiSi₂, MoSi₂, CoSi₂, NiSi₂, CaSi₂, CrSi₂, Cu₆Si, FeSi₂, MnSi₂, NbSi₂, TaSi₂, VSi₂, WSi₂, ZnSi₂, SiC, Si₃N₄, Si₂N₂O, SiOᵥ (0 < v ≤ 2), LiSiO, simple tin, SnSiO₃, LiSnO, Mg₂Sn, and SnO_{w} (0 < w ≤ 2).

Examples thereof further include composite materials of Si or Sn used as a first constitutional element, and second and third constitutional elements. The second constitutional element is at least one selected from the group consisting of cobalt, iron, magnesium, titanium, vanadium, chromium, manganese, nickel, copper, zinc, gallium, and zirconium, for example. The third constitutional element is at least one selected from the group consisting of boron, carbon, aluminum, and phosphorus, for example.

In order to achieve a high battery capacity and excellent battery characteristics, the metal material is preferably simple silicon or tin (which may contain trace impurities), SiOᵥ (0 < v ≤ 2), SnO_{w} (0 ≤ w ≤ 2), a Si-Co-C composite material, a Si-Ni-C composite material, a Sn-Co-C composite material, or a Sn-Ni-C composite material.

The lithium-containing metal complex oxide material to be used as the negative electrode active material may be any material that can occlude and release lithium. In order to achieve good high-current-density charge and discharge characteristics, materials containing titanium and lithium are preferred, lithium-containing metal complex oxide materials containing titanium are more preferred, and complex oxides of lithium and titanium (hereinafter, abbreviated as "lithium titanium complex oxides") are still more preferred. In other words, use of a spinel-structured lithium titanium complex oxide in the negative electrode active material for an electrolyte battery is particularly preferred because this can markedly reduce the output resistance.

Preferred examples of the lithium titanium complex oxides include compounds represented by the following formula:

LiₓTi_{y}M_{z}O₄

wherein M is at least one element selected from the group consisting of Na, K, Co, Al, Fe, Ti, Mg, Cr, Ga, Cu, Zn, and Nb.

In order to achieve a good balance of the battery performance, particularly preferred among the above compositions are those satisfying any of the following:
(i) 1.2 ≤ x ≤ 1.4, 1.5 ≤ y ≤ 1.7, z = 0
(ii) 0.9 ≤ x ≤ 1.1, 1.9 ≤ y ≤ 2.1, z = 0
(iii) 0.7 ≤ x ≤ 0.9, 2.1 ≤ y ≤ 2.3, z = 0.

Particularly preferred representative composition of the compound is Li_{4/3}Ti_{5/3}O₄ corresponding to the composition (i), Li₁Ti₂O₄ corresponding to the composition (ii), and Li_{4/5}Ti_{11/5}O₄ corresponding to the composition (iii). Preferred examples of the structure satisfying Z ≠ 0 include Li_{4/3}Ti_{4/3}Al_{1/3}O₄.

### <Conductive aid (conductive material)>

The surface of the conductive aid is fluorinated by treatment with the surface-treating agent for an electrode material. The percentage of fluorine on the surface of the conductive aid is preferably 3.5 atom% or higher, more preferably 4.0 atom% or higher, still more preferably 4.5 atom% or higher, while preferably 9.5 atom% or lower, more preferably 8.5 atom% or lower, still more preferably 7.5 atom% or lower.

The percentage of fluorine on the surface of the conductive aid can be measured by energy dispersive X-ray spectroscopy using a scanning electron microscope (SEM-EDX).

The unreacted/reacted surface-treating agent for an electrode material remaining on the surface of the conductive aid forms a stable protective film. The amount (total amount) of the unreacted/reacted surface-treating agent for an electrode material remaining on the surface of the conductive aid is preferably 0.01% by mass or more, more preferably 0.05% by mass or more, still more preferably 0.1% by mass or more, while preferably 5.0% by mass or less, more preferably 2.0% by mass or less, still more preferably 0.5% by mass or less.

The amount of the unreacted/reacted surface-treating agent for an electrode material remaining on the surface of the conductive aid can be calculated from the difference in mass of the conductive aid before and after the treatment as weighed by a precision electronic balance.

Specific examples of the conductive aid include metal materials such as copper and nickel, and carbon materials such as graphite, including natural graphite and artificial graphite, carbon black, including acetylene black, Ketjen black, channel black, furnace black, lamp black, and thermal black, and amorphous carbon, including needle coke, carbon nanotube, fullerene, and VGCF. Preferred among these are carbon materials.

One of these materials may be used alone or two or more thereof may be used in any combination at any ratio.

### <Electrode>

The electrode used includes at least one of the positive electrode active material, the current collector foil, the negative electrode active material, or the conductive aid. For the positive electrode active material and the negative electrode active material, the electrode includes only one of them.

The surface of the electrode is fluorinated by treatment with the surface-treating agent for an electrode material. The percentage of fluorine on the surface of the electrode is preferably 2.5 atom% or higher, more preferably 3.3 atom% or higher, still more preferably 3.6 atom% or higher, while preferably 6.5 atom% or lower, more preferably 5.5 atom% or lower, still more preferably 4.5 atom% or lower.

The percentage of fluorine on the surface of the electrode can be measured by energy dispersive X-ray spectroscopy using a scanning electron microscope (SEM-EDX).

The unreacted/reacted surface-treating agent for an electrode material remaining on the surface of the electrode forms a stable protective film. The amount of the unreacted/reacted surface-treating agent for an electrode material remaining on the surface of the electrode is normally very small and is hardly measurable.

In the case where the electrode is a positive electrode containing the positive electrode active material, the positive electrode includes a current collector foil and a positive electrode active material layer containing the positive electrode active material. The current collector foil may be the current collector foil of the disclosure, which is treated with the surface-treating agent for an electrode material, or another current collector foil, and is preferably the current collector foil of the disclosure.

In order to achieve a high battery capacity, the amount of the positive electrode active material is preferably 50 to 99.5% by mass, more preferably 80 to 99% by mass, of the positive electrode mixture. The amount of the positive electrode active material in the positive electrode active material layer is preferably 80% by mass or more, more preferably 82% by mass or more, particularly preferably 84% by mass or more. The upper limit thereof is preferably 99% by mass or less, more preferably 98% by mass or less. Too small an amount of the positive electrode active material in the positive electrode active material layer may lead to an insufficient electric capacity. In contrast, too large an amount thereof may lead to insufficient strength of the positive electrode.

The positive electrode mixture contains the surface-treating agent for an electrode material in an amount of preferably 0.01% by mass or more, more preferably 0.05% by mass or more, still more preferably 0.1% by mass or more, while preferably 10% by mass or less, more preferably 5% by mass or less, still more preferably 3% by mass or less.

In the case where the electrode is a negative electrode containing the negative electrode active material, the negative electrode includes a current collector foil and a negative electrode active material layer containing the negative electrode active material. The current collector foil may be the current collector foil of the disclosure, which is treated with the surface-treating agent for an electrode material, or another current collector foil, and is preferably the current collector foil of the disclosure.

In order to achieve a high battery capacity, the amount of the negative electrode active material is preferably 50 to 99.5% by mass, more preferably 80 to 99% by mass, in the negative electrode mixture. The amount of the negative electrode active material in the negative electrode active material layer is preferably 80% by mass or more, more preferably 82% by mass or more, particularly preferably 84% by mass or more. The upper limit of the amount is preferably 99% by mass or less, more preferably 98% by mass or less. Too small an amount of the negative electrode active material in the negative electrode active material layer may lead to an insufficient electric capacity. In contrast, too large an amount thereof may lead to insufficient strength of the negative electrode.

The amount of the surface-treating agent for an electrode material in the negative electrode mixture is preferably 0.01% by mass or more, more preferably 0.05% by mass or more, still more preferably 0.1% by mass or more, while preferably 10% by mass or less, more preferably 5% by mass or less, still more preferably 3% by mass or less.

The amount of the surface-treating agent for an electrode material in the electrode mixture described above is a total amount of the unreacted/reacted surface-treating agent for an electrode material and is a value (estimated value) calculated from the amounts of the materials charged.

The positive electrode mixture and the negative electrode mixture each preferably contain a conductive aid. The conductive aid may be the conductive aid of the disclosure, which is treated with the surface-treating agent for an electrode material, or another conductive aid, and is preferably the conductive aid of the disclosure. One of these may be used alone or two or more thereof may be used in any combination at any ratio.

The conductive aid is used in an amount of usually 0.01% by mass or more, preferably 0.1% by mass or more, more preferably 1% by mass or more, while usually 50% by mass or less, preferably 30% by mass or less, more preferably 15% by mass or less, in each of the positive electrode mixture and the negative electrode mixture. The conductive aid in an amount less than the above range may cause insufficient conductivity. In contrast, the conductive aid in an amount more than the above range may cause a low battery capacity.

The positive electrode mixture and the negative electrode mixture each preferably further contain a binder and a thickening agent.

The binder may be any material that is safe against a solvent to be used in production of the electrode and the electrolyte solution. Examples thereof include resin polymers such as polyethylene, polypropylene, polyethylene terephthalate, polymethyl methacrylate, aromatic polyamide, chitosan, alginic acid, polyacrylic acid, polyimide, cellulose, and nitro cellulose; rubbery polymers such as SBR (styrene-butadiene rubber), isoprene rubber, butadiene rubber, fluoroelastomers, NBR (acrylonitrile-butadiene rubber), and ethylene-propylene rubber; styrene-butadienestyrene block copolymers and hydrogenated products thereof; thermoplastic elastomeric polymers such as EPDM (ethylene-propylene-diene terpolymers), styrene-ethylene-butadienestyrene copolymers, and styrene-isoprene-styrene block copolymers and hydrogenated products thereof; soft resin polymers such as syndiotactic-1,2-polybutadiene, polyvinyl acetate, ethylene-vinyl acetate copolymers, and propylene-α-olefin copolymers; fluoropolymers such as polyvinylidene fluoride, polytetrafluoroethylene, vinylidene fluoride copolymers, and tetrafluoroethylene-ethylene copolymers; and polymer compositions having ion conductivity of alkali metal ions (especially, lithium ions). One of these may be used alone or two or more thereof may be used in any combination at any ratio.

The amount of the binder in each of the positive electrode mixture and the negative electrode mixture is usually 0.1% by mass or more, preferably 1% by mass or more, more preferably 1.5% by mass or more, while usually 80% by mass or less, preferably 60% by mass or less, more preferably 40% by mass or less, most preferably 10% by mass or less. Too small an amount of the binder may fail to sufficiently hold the electrode active material and cause insufficient mechanical strength of the electrode, impairing the battery performance such as cycle characteristics. In contrast, too large an amount thereof may cause a reduction in battery capacity and conductivity.

Examples of the thickening agent include carboxymethyl cellulose, methyl cellulose, hydroxymethyl cellulose, ethyl cellulose, polyvinyl alcohol, oxidized starch, monostarch phosphate, casein, polyvinylpyrrolidone, and salts thereof. One of the thickening agents may be used alone or two or more thereof may be used in any combination at any ratio.

The amount of the thickening agent in each of the positive electrode mixture and the negative electrode mixture is within a range of usually 0.1% by mass or more, preferably 0.2% by mass or more, more preferably 0.3% by mass or more and usually 5% by mass or less, preferably 3% by mass or less, more preferably 2% by mass or less. The thickening agent in an amount smaller than the above range may cause significantly poor easiness of application. The thickening agent in an amount greater than the above range may cause a low proportion of the active material in the electrode layer, resulting in a low capacity of the battery and high resistance between the electrode active materials.

The ratio between the thicknesses of the current collector foil and the electrode active material layer in the electrode may be any value, and the ratio {(thickness of electrode active material layer on one side immediately before injection of electrolyte solution)/(thickness of current collector)} is preferably 20 or lower, more preferably 15 or lower, most preferably 10 or lower. The ratio is also preferably 0.5 or higher, more preferably 0.8 or higher, most preferably 1 or higher. The current collector and the electrode active material layer showing a ratio higher than the above range may cause the current collector to generate heat due to Joule heating during high-current-density charge and discharge. The current collector and the electrode active material layer showing a ratio lower than the above range may cause an increased ratio by volume of the current collector to the positive electrode active material, reducing the battery capacity.

The electrode may be produced by a usual method. An example of the production method is a method in which the positive electrode active material or the negative electrode active material is mixed with the aforementioned binder, thickening agent, conductive material, solvent, and other components to form a slurry-like positive electrode mixture or a slurry-like negative electrode mixture, and then the resulting mixture is applied to a current collector, dried, and pressed so as to be densified.

In the case of using an alloyed material, a thin film layer (electrode active material layer) may be produced by vapor deposition, sputtering, plating, or the like.

The solvent for forming the slurry may be any solvent that can dissolve or disperse therein the positive electrode active material, the negative electrode active material, the conductive aid, and the binder, as well as a thickening agent used as appropriate. The solvent may be either an aqueous solvent or an organic solvent. Examples of the aqueous medium include water and solvent mixtures of an alcohol and water. Examples of the organic medium include aliphatic hydrocarbons such as hexane; aromatic hydrocarbons such as benzene, toluene, xylene, and methyl naphthalene; heterocyclic compounds such as quinoline and pyridine; ketones such as acetone, methyl ethyl ketone, and cyclohexanone; esters such as methyl acetate and methyl acrylate; amines such as diethylene triamine and N,N-dimethylaminopropylamine; ethers such as diethyl ether, propylene oxide, and tetrahydrofuran (THF); amides such as N-methylpyrrolidone (NMP), dimethyl formamide, and dimethyl acetamide; and aprotic polar solvents such as hexamethyl phospharamide and dimethyl sulfoxide.

The densification may be achieved using a manual press or a roll press, for example. The density of the positive electrode active material layer is preferably 1.5 g/cm³ or higher, more preferably 2 g/cm³ or higher, still more preferably 2.2 g/cm³ or higher, while preferably 5 g/cm³ or lower, more preferably 4.5 g/cm³ or lower, still more preferably 4 g/cm³ or lower. The positive electrode active material layer having a density higher than the above range may cause low permeability of the electrolyte solution toward the vicinity of the interface between the current collector and the active material, and poor charge and discharge characteristics particularly at a high current density, failing to provide high output. The positive electrode active material layer having a density lower than the above range may cause poor conductivity between the active materials and increase the battery resistance, failing to provide high output.

The area of the positive electrode active material layer is preferably large relative to the outer surface area of an external case of the battery. Specifically, the total area of the positive electrode is preferably 15 times or more, more preferably 40 times or more, greater than the surface area of the external case of the secondary battery. For closed, square-shaped cases, the outer surface area of an external case of the battery herein means the total area calculated from the dimensions including the length, width, and thickness of the case portion into which a power-generating element is packed except for a protruding portion of a terminal. For closed, cylinder-like cases, the outer surface area of an external case of the battery herein means the geometric surface area of an approximated cylinder of the case portion into which a power-generating element is packed except for a protruding portion of a terminal. The total area of the positive electrode herein means the geometric surface area of the positive electrode mixture layer opposite to a mixture layer including the negative electrode active material. For structures including a current collector foil and positive electrode mixture layers on both sides of the current collector, the total area of the positive electrode is the sum of the areas calculated on the respective sides.

The positive electrode plate may have any thickness. In order to achieve a high capacity and high output, the lower limit of the thickness of the mixture layer on one side of the current collector excluding the thickness of the base metal foil is preferably 10 µm or greater, more preferably 20 µm or greater, while preferably 500 µm or smaller, more preferably 450 µm or smaller.

To a surface of the positive electrode plate may be attached a substance having a composition different from the positive electrode plate. Examples of the substance attached to the surface include oxides such as aluminum oxide, silicon oxide, titanium oxide, zirconium oxide, magnesium oxide, calcium oxide, boron oxide, antimony oxide, and bismuth oxide; sulfates such as lithium sulfate, sodium sulfate, potassium sulfate, magnesium sulfate, calcium sulfate, and aluminum sulfate; carbonates such as lithium carbonate, calcium carbonate, and magnesium carbonate; and carbon.

The thickness of the negative electrode plate is a design matter in accordance with the positive electrode plate to be used, and may be any value. The thickness of the mixture layer excluding the thickness of the base metal foil is usually 15 µm or greater, preferably 20 µm or greater, more preferably 30 µm or greater, while usually 300 µm or smaller, preferably 280 µm or smaller, more preferably 250 µm or smaller.

To a surface of the negative electrode plate may be attached a substance having a composition different from the negative electrode plate. Examples of the substance attached to the surface include oxides such as aluminum oxide, silicon oxide, titanium oxide, zirconium oxide, magnesium oxide, calcium oxide, boron oxide, antimony oxide, and bismuth oxide; sulfates such as lithium sulfate, sodium sulfate, potassium sulfate, magnesium sulfate, calcium sulfate, and aluminum sulfate; and carbonates such as lithium carbonate, calcium carbonate, and magnesium carbonate.

The electrode of the disclosure is suitable for batteries such as lithium-ion secondary batteries, and is also usable in electrochemical devices other than batteries, such as electric double-layer capacitors.

### <Production method of materials including positive electrode active material>

The positive electrode active material, the current collector foil, the negative electrode active material, the conductive aid, and the electrode of the disclosure described above (hereafter, also collectively referred to as "materials including the positive electrode active material") can be produced by treating the materials including the positive electrode active material in an untreated state with the surface-treating agent for an electrode material.

The untreated state refers to a state where treatment with the surface-treating agent for an electrode material has not been performed and may be a state where another treatment has been performed.

The treatment may be performed by a method including bringing the materials including the positive electrode active material in an untreated state into contact with the surface-treating agent for an electrode material, preferably with a solution (treatment liquid) containing the surface-treating agent for an electrode material.

The contact with the solution may be performed by any method such as a method of applying the solution to the materials including the positive electrode active material in an untreated state or a method of immersing the materials including the positive electrode active material in an untreated state in the solution.

In the case where the materials including the positive electrode active material in an untreated state is immersed in the solution, the immersion time is preferably 10 hours or shorter, more preferably 2 hours or shorter, while preferably 0.01 hours or longer, more preferably 0.05 hours or longer. The temperature of the solution upon immersion is preferably 100°C or lower, more preferably 80°C or lower, while preferably 0°C or higher, more preferably 5°C or higher.

For treatment of the electrode, the electrode may be brought into contact with the surface-treating agent for an electrode material by producing a battery using an electrolyte solution containing the surface-treating agent for an electrode material as an additive.

The amount of the surface-treating agent for an electrode material in the solution is preferably 0.1% by mass or more, more preferably 0.3% by mass or more, while preferably 10% by mass or less, more preferably 5% by mass or less.

As a solvent used for the solution, any types of solvent that can dissolve or disperse the surface-treating agent for an electrode material can be used, and any of those described for the slurry may be used. Examples thereof include:
ester solvents such as methyl formate, ethyl formate, methyl acetate, ethyl acetate, propyl acetate, methyl propionate, γ-butyrolactone, and propylene carbonate;
nitrile solvents such as acetonitrile and propionitrile;
ether solvents such as diethyl ether, dipropyl ether, tetrahydrofuran, cyclopentyl methyl ether (CPME), and methyl tert-butyl ether (MTBE); and
acetone, and preferred among these are acetonitrile, acetone, and tetrahydrofuran.

From the positive electrode active material after the contact with the solution, the solvent in the solution is preferably removed. The removal of the solvent may be performed by any method, such as vaporization of the solvent by heating, vacuum-drying, or other techniques. The conditions for the heating, vacuum-drying, or other techniques may be appropriately adjusted in accordance with the type or amount of the solvent.

### <Battery>

The battery of the disclosure includes the electrode (positive electrode and/or negative electrode).

The configuration other than the electrode is not limited, and the battery may include usual materials such as an electrolyte solution and a separator.

The battery is preferably a non-aqueous electrolyte battery, more preferably a lithium-ion secondary battery.

The battery is useful as a battery for large-size lithium-ion secondary batteries for hybrid vehicles or distributed generation.

### <Electrolyte solution>

The electrolyte solution of the battery contains a solvent and an electrolyte salt.

The solvent of the electrolyte solution preferably includes at least one selected from the group consisting of a carbonate and a carboxylate.

The carbonate may be either a cyclic carbonate or an acyclic carbonate.

The cyclic carbonate may be either a non-fluorinated cyclic carbonate or a fluorinated cyclic carbonate.

An example of the non-fluorinated cyclic carbonate is a non-fluorinated saturated cyclic carbonate. Preferred is a non-fluorinated saturated alkylene carbonate containing a C2-C6 alkylene group, more preferred is a non-fluorinated saturated alkylene carbonate containing a C2-C4 alkylene group.

In order to give high permittivity and suitable viscosity, the non-fluorinated saturated cyclic carbonate preferably includes at least one selected from the group consisting of ethylene carbonate, propylene carbonate, cis-2,3-pentylene carbonate, cis-2,3-butylene carbonate, 2,3-pentylene carbonate, 2,3-butylene carbonate, 1,2-pentylene carbonate, 1,2-butylene carbonate, and butylene carbonate.

One non-fluorinated saturated cyclic carbonate may be used alone, or two or more thereof may be used in any combination at any ratio.

The non-fluorinated saturated cyclic carbonate, when contained, is preferably present in an amount of 5 to 90% by volume, more preferably 10 to 60% by volume, still more preferably 15 to 45% by volume, relative to the solvent.

The fluorinated cyclic carbonate is a cyclic carbonate containing a fluorine atom. A solvent containing a fluorinated cyclic carbonate can suitably be used at high voltage.

The fluorinated cyclic carbonate may be either a fluorinated saturated cyclic carbonate or a fluorinated unsaturated cyclic carbonate.

The fluorinated saturated cyclic carbonate is a saturated cyclic carbonate containing a fluorine atom. Specific examples thereof include a compound represented by the following formula (A): 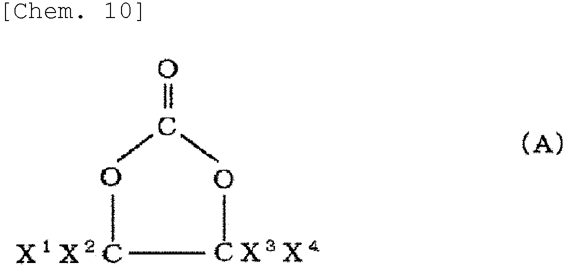 (wherein X¹ to X⁴ are the same as or different from each other, and are each -H, -CH₃, -C₂H₅, -F, a fluorinated alkyl group optionally containing an ether bond, or a fluorinated alkoxy group optionally containing an ether bond; at least one selected from X¹ to X⁴ is -F, a fluorinated alkyl group optionally containing an ether bond, or a fluorinated alkoxy group optionally containing an ether bond). Examples of the fluorinated alkyl group include -CF₃, - CF₂H, and -CH₂F.

The presence of the fluorinated saturated cyclic carbonate in the electrolyte solution can improve the oxidation resistance of the electrolyte solution, resulting in stable and excellent charge and discharge characteristics.

The term "ether bond" herein means a bond represented by -O-.

In order to give a good permittivity and oxidation resistance, one or two of X¹ to X⁴ is/are each preferably - F, a fluorinated alkyl group optionally containing an ether bond, or a fluorinated alkoxy group optionally containing an ether bond.

In anticipation of a decrease in viscosity at low temperature, an increase in flash point, and improvement in solubility of an electrolyte salt, X¹ to X⁴ are each preferably -H, -F, a fluorinated alkyl group (a), a fluorinated alkyl group (b) containing an ether bond, or a fluorinated alkoxy group (c).

The fluorinated alkyl group (a) is a group obtainable by replacing at least one hydrogen atom of an alkyl group with a fluorine atom. The fluorinated alkyl group (a) preferably has a carbon number of 1 to 20, more preferably 1 to 17, still more preferably 1 to 7, particularly preferably 1 to 5.

Too large a carbon number may cause poor low-temperature characteristics and low solubility of an electrolyte salt. Too small a carbon number may cause low solubility of an electrolyte salt, low discharge efficiency, and increased viscosity, for example.

Examples of the fluorinated alkyl group (a) having a carbon number of 1 include CFH₂-, CF₂H-, and CF₃-. In order to give good high-temperature storage characteristics, particularly preferred is CF₂H- or CF₃-. Most preferred is CF₃-.

In order to give good solubility of an electrolyte salt, preferred examples of the fluorinated alkyl group (a) having a carbon number of 2 or greater include fluorinated alkyl groups represented by the following formula (a-1):

R^{a1}-R^{a2}- (a-1)

wherein R^{a1} is an alkyl group having a carbon number of 1 or greater and optionally containing a fluorine atom; R^{a2} is a C1-C3 alkylene group optionally containing a fluorine atom; and at least one of R^{a1} or R^{a2} contains a fluorine atom.
R^{a1} and R^{a2} each may further contain an atom other than carbon, hydrogen, and fluorine atoms.

R^{a1} is an alkyl group having a carbon number of 1 or greater and optionally containing a fluorine atom. R^{a1} is preferably a C1-C16 linear or branched alkyl group. The carbon number of R^{a1} is more preferably 1 to 6, still more preferably 1 to 3.

Specifically, for example, CH₃-, CH₃CH₂-, CH₃CH₂CH₂-, CH₃CH₂CH₂CH₂-, and groups represented by the following formulas may be mentioned as linear or branched alkyl groups for R^{a1}.

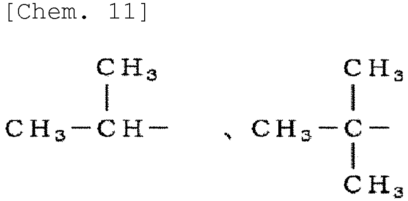

Examples of R^{a1} as a linear alkyl group containing a fluorine atom include CF₃-, CF₃CH₂-, CF₃CF₂-, CF₃CH₂CH₂-, CF₃CF₂CH₂-, CF₃CF₂CF₂-, CF₃CH₂CF₂-, CF₃CH₂CH₂CH₂-, CF₃CF₂CH₂CH₂-, CF₃CH₂CF₂CH₂-, CF₃CF₂CF₂CH₂-, CF₃CF₂CF₂CF₂-, CF₃CF₂CH₂CF₂-, CF₃CH₂CH₂CH₂CH₂-, CF₃CF₂CH₂CH₂CH₂-, CF₃CH₂CF₂CH₂CH₂-, CF₃CF₂CF₂CH₂CH₂-, CF₃CF₂CF₂CF₂CH₂-, CF₃CF₂CH₂CF₂CH₂-, CF₃CF₂CH₂CH₂CH₂CH₂-, CF₃CF₂CF₂CF₂CH₂CH₂-, CF₃CF₂CH₂CF₂CH₂CH₂-, HCF₂-, HCF₂CH₂-, HCF₂CF₂-, HCF₂CH₂CH₂-, HCF₂CF₂CH₂-, HCF₂CH₂CF₂-, HCF₂CF₂CH₂CH₂-, HCF₂CH₂CF₂CH₂-, HCF₂CF₂CF₂CF₂-, HCF₂CF₂CH₂CH₂CH₂-, HCF₂CH₂CF₂CH₂CH₂-, HCF₂CF₂CF₂CF₂CH₂-, HCF₂CF₂CF₂CF₂CH₂CH₂-, FCH₂-, FCH₂CH₂-, FCH₂CF₂-, FCH₂CF₂CH₂-, FCH₂CF₂CF₂-, CH₃CF₂CH₂-, CH₃CF₂CF₂-, CH₃CF₂CH₂CF₂-, CH₃CF₂CF₂CF₂-, CH₃CH₂CF₂CF₂-, CH₃CF₂CH₂CF₂CH₂-, CH₃CF₂CF₂CF₂CH₂-, CH₃CF₂CF₂CH₂CH₂-, CH₃CH₂CF₂CF₂CH₂-, CH₃CF₂CH₂CF₂CH₂CH₂-, CH₃CF₂CH₂CF₂CH₂CH₂-, HCFClCF₂CH₂-, HCF₂CFClCH₂-, HCF₂CFClCF₂CFClCH₂-, and HCFClCF₂CFClCF₂CH₂-.

Examples of R^{a1} as a branched alkyl group containing a fluorine atom include those represented by the following formulas.

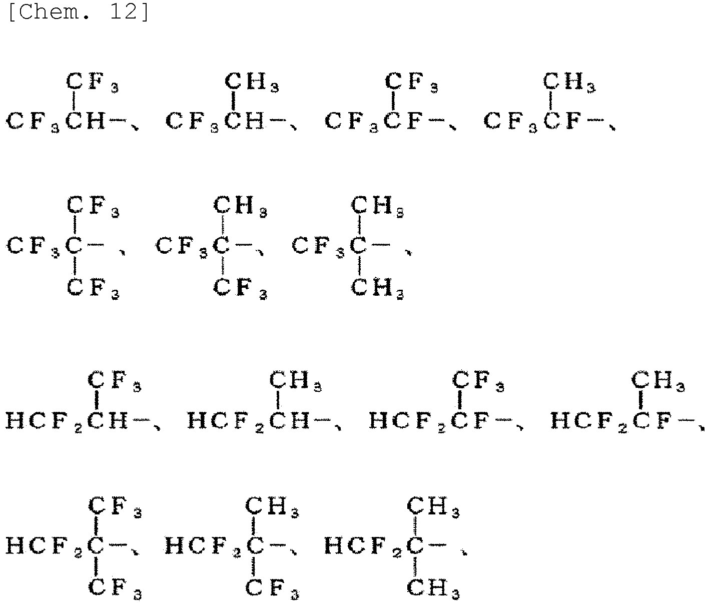

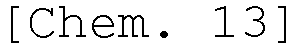

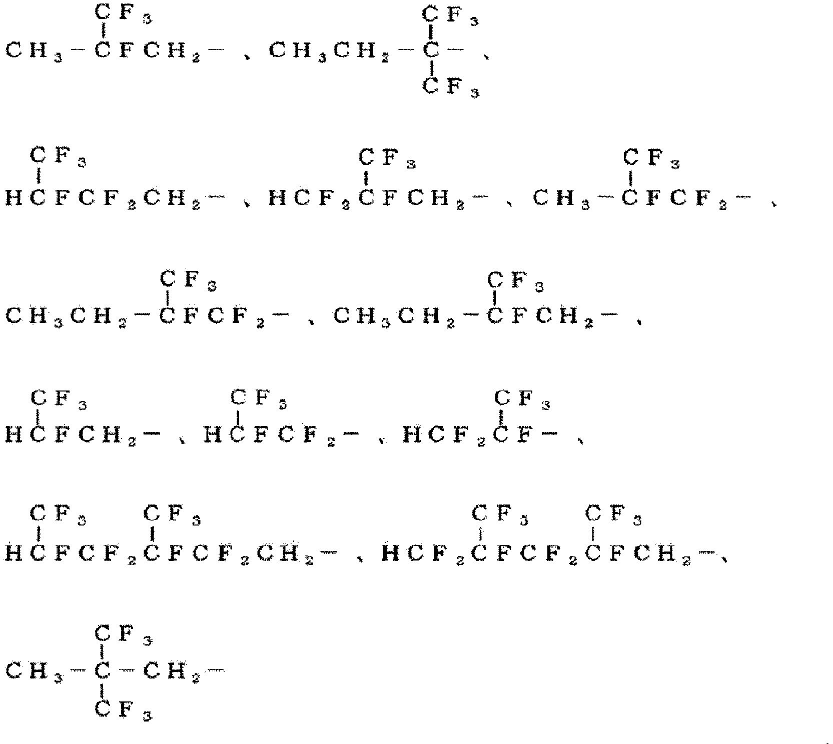

The presence of a branch such as CH₃- or CF₃- may easily cause high viscosity. Thus, the number of such branches is more preferably small (one) or zero.

R^{a2} is a C1-C3 alkylene group optionally containing a fluorine atom. R^{a2} may be either linear or branched. Examples of a minimum structural unit constituting such a linear or branched alkylene group are shown below. R^{a2} is constituted by one or combination of these units.
(i) Linear minimum structural units

   -CH₂-, -CHF-, -CF₂-, -CHCl-, -CFCl-, -CCl₂-
(ii) Branched minimum structural units

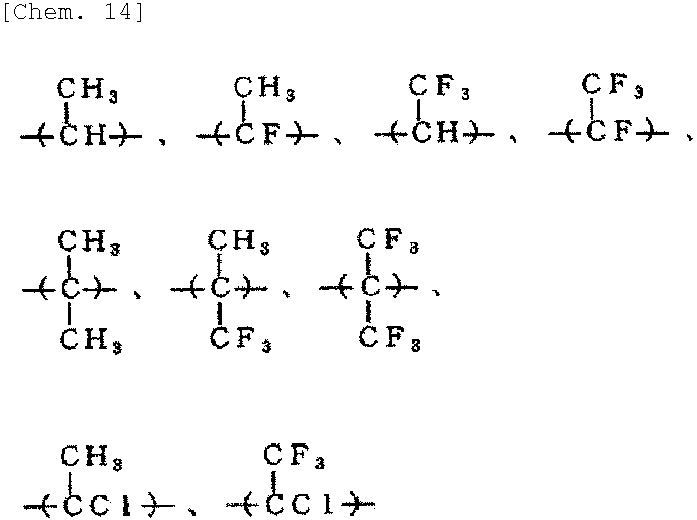

Preferred among these exemplified units are Cl-free structural units because such units may not be dehydrochlorinated by a base, and thus may be more stable.

R^{a2}, when it is a linear group, consists only of any of the above linear minimum structural units, and is preferably -CH₂-, -CH₂CH₂-, or CF₂-. In order to further improve the solubility of an electrolyte salt, -CH₂- or - CH₂CH₂- is more preferred.

R^{a2}, when it is a branched group, includes at least one of the above branched minimum structural units. A preferred example thereof is a group represented by - (CX^{a}X^{b})- (wherein X^{a} is H, F, CH₃, or CF₃; X^{b} is CH₃ or CF₃; when X^{b} is CF₃, X^{a} is H or CH₃). Such a group can much further improve the solubility of an electrolyte salt.

For example, CF₃CF₂-, HCF₂CF₂-, H₂CFCF₂-, CH₃CF₂-, CF₃CHF-, CH₃CF₂-, CF₃CF₂CF₂-, HCF₂CF₂CF₂-, H₂CFCF₂CF₂-, CH₃CF₂CF₂-, and those represented by the following formulas: 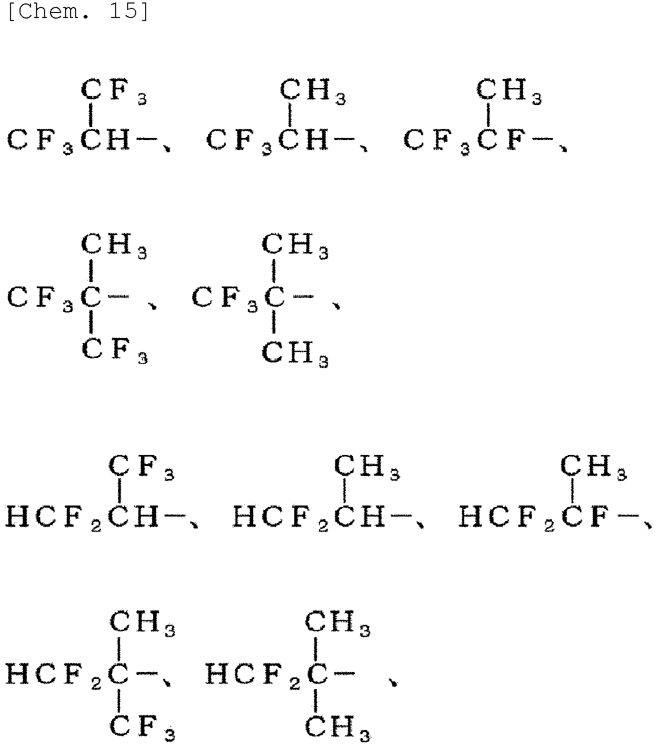 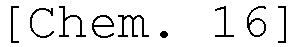 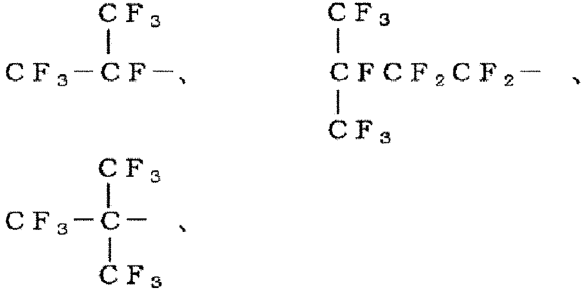 may be mentioned as preferred examples of the fluorinated alkyl group (a).

The fluorinated alkyl group (b) containing an ether bond is a group obtainable by replacing at least one hydrogen atom of an alkyl group containing an ether bond with a fluorine atom. The fluorinated alkyl group (b) containing an ether bond preferably has a carbon number of 2 to 17. Too large a carbon number may cause high viscosity of the fluorinated saturated cyclic carbonate. This may also cause the presence of many fluorine-containing groups, resulting in poor solubility of an electrolyte salt due to reduction in permittivity, and poor miscibility with other solvents. Accordingly, the carbon number of the fluorinated alkyl group (b) containing an ether bond is preferably 2 to 10, more preferably 2 to 7.

The alkylene group constituting the ether moiety of the fluorinated alkyl group (b) containing an ether bond is a linear or branched alkylene group. Examples of a minimum structural unit constituting such a linear or branched alkylene group are shown below.
(i) Linear minimum structural units

   -CH₂-, -CHF-, -CF₂-, -CHCl-, -CFCl-, -CCl₂-
(ii) Branched minimum structural units

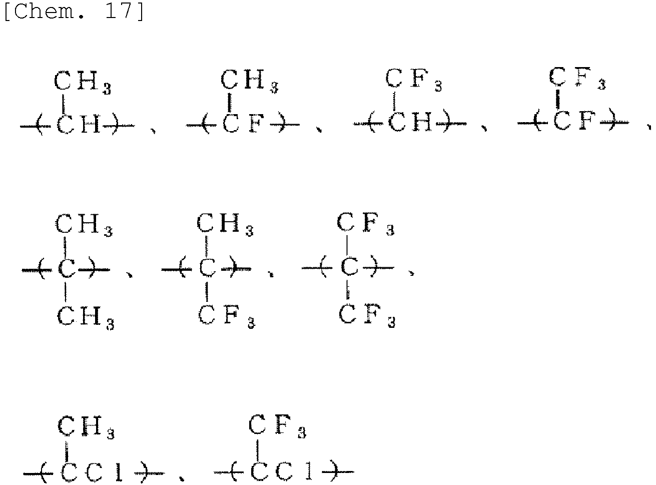

The alkylene group may be constituted by one of these minimum structural units, or may be constituted by multiple linear units (i), by multiple branched units (ii), or by a combination of a linear unit (i) and a branched unit (ii). Preferred examples will be mentioned in detail later.

Preferred among these exemplified units are Cl-free structural units because such units may not be dehydrochlorinated by a base, and thus may be more stable.

A still more preferred example of the fluorinated alkyl group (b) containing an ether bond is a group represented by the following formula (b-1):

R³-(OR⁴)ₙ₁- (b-1)

wherein R³ is preferably a C1-C6 alkyl group optionally containing a fluorine atom; R⁴ is preferably a C1-C4 alkylene group optionally containing a fluorine atom; n1 is an integer of 1 to 3; and at least one of R³ or R⁴ contains a fluorine atom.

Examples of R³ and R⁴ include the following groups, and any appropriate combination of these groups can provide the fluorinated alkyl group (b) containing an ether bond represented by the formula (b-1). Still, the groups are not limited thereto.
(1) R³ is preferably an alkyl group represented by the formula: X^{c}₃C-(R⁵)ₙ₂-, wherein three X^{c}s are the same as or different from each other, and are each H or F; R⁵ is a C1-C5 alkylene group optionally containing a fluorine atom; and n2 is 0 or 1.

When n2 is 0, R³ may be CH₃-, CF₃-, HCF₂-, or H₂CF-, for example.

When n2 is 1, specific examples of R³ as a linear group include CF₃CH₂-, CF₃CF₂-, CF₃CH₂CH₂-, CF₃CF₂CH₂-, CF₃CF₂CF₂-, CF₃CH₂CF₂-, CF₃CH₂CH₂CH₂-, CF₃CF₂CH₂CH₂-, CF₃CH₂CF₂CH₂-, CF₃CF₂CF₂CH₂-, CF₃CF₂CF₂CF₂-, CF₃CF₂CH₂CF₂-, CF₃CH₂CH₂CH₂CH₂-, CF₃CF₂CH₂CH₂CH₂-, CF₃CH₂CF₂CH₂CH₂-, CF₃CF₂CF₂CH₂CH₂-, CF₃CF₂CF₂CF₂CH₂-, CF₃CF₂CH₂CF₂CH₂-, CF₃CF₂CH₂CH₂CH₂CH₂-, CF₃CF₂CF₂CF₂CH₂CH₂-, CF₃CF₂CH₂CF₂CH₂CH₂-, HCF₂CH₂-, HCF₂CF₂-, HCF₂CH₂CH₂-, HCF₂CF₂CH₂-, HCF₂CH₂CF₂-, HCF₂CF₂CH₂CH₂-, HCF₂CH₂CF₂CH₂-, HCF₂CF₂CF₂CF₂-, HCF₂CF₂CH₂CH₂CH₂-, HCF₂CH₂CF₂CH₂CH₂-, HCF₂CF₂CF₂CF₂CH₂-, HCF₂CF₂CF₂CF₂CH₂CH₂-, FCH₂CH₂-, FCH₂CF₂-, FCH₂CF₂CH₂-, CH₃CF₂-, CH₃CH₂-, CH₃CF₂CH₂-, CH₃CF₂CF₂-, CH₃CH₂CH₂-, CH₃CF₂CH₂CF₂-, CH₃CF₂CF₂CF₂-, CH₃CH₂CF₂CF₂-, CH₃CH₂CH₂CH₂-, CH₃CF₂CH₂CF₂CH₂-, CH₃CF₂CF₂CF₂CH₂-, CH₃CF₂CF₂CH₂CH₂-, CH₃CH₂CF₂CF₂CH₂-, CH₃CF₂CH₂CF₂CH₂CH₂-, CH₃CH₂CF₂CF₂CH₂CH₂-, and CH₃CF₂CH₂CF₂CH₂CH₂-.

When n2 is 1, those represented by the following formulas: 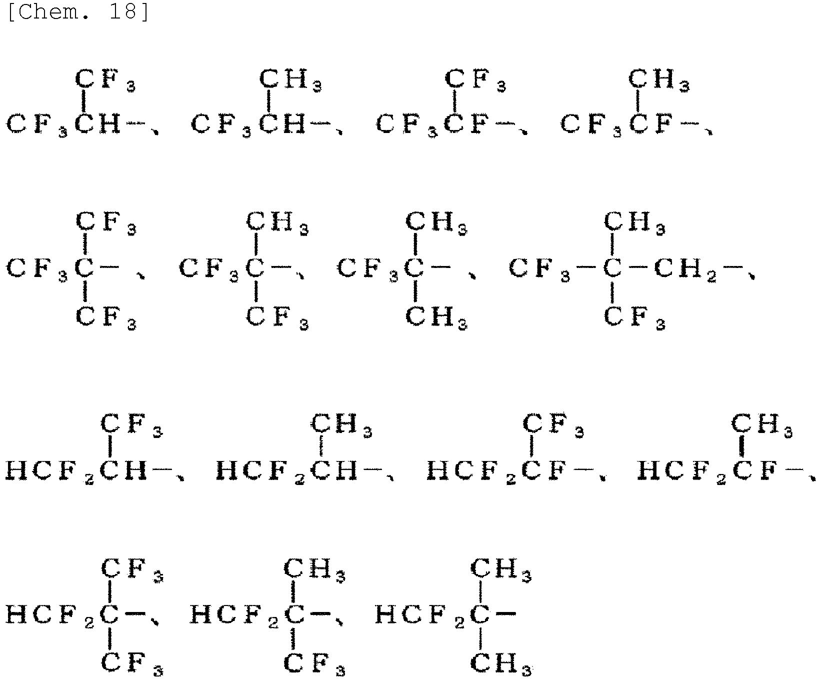 may be mentioned as examples of R³ as a branched group.

The presence of a branch such as CH₃- or CF₃- may easily cause high viscosity. Thus, R³ is more preferably a linear group.

(2) In -(OR⁴)ₙ₁- of the formula (b-1), n1 is an integer of 1 to 3, preferably 1 or 2. When n1 is 2 or 3, R⁴s may be the same as or different from each other.

Preferred specific examples of R⁴ include the following linear or branched groups.

Examples of the linear groups include -CH₂-, -CHF-, - CF₂-, -CH₂CH₂-, -CF₂CH₂-, -CF₂CF₂-, -CH₂CF₂-, -CH₂CH₂CH₂-, - CH₂CH₂CF₂-, -CH₂CF₂CH₂-, -CH₂CF₂CF₂-, -CF₂CH₂CH₂-, -CF₂CF₂CH₂-, -CF₂CH₂CF₂-, and -CF₂CF₂CF₂-.

Those represented by the following formulas: 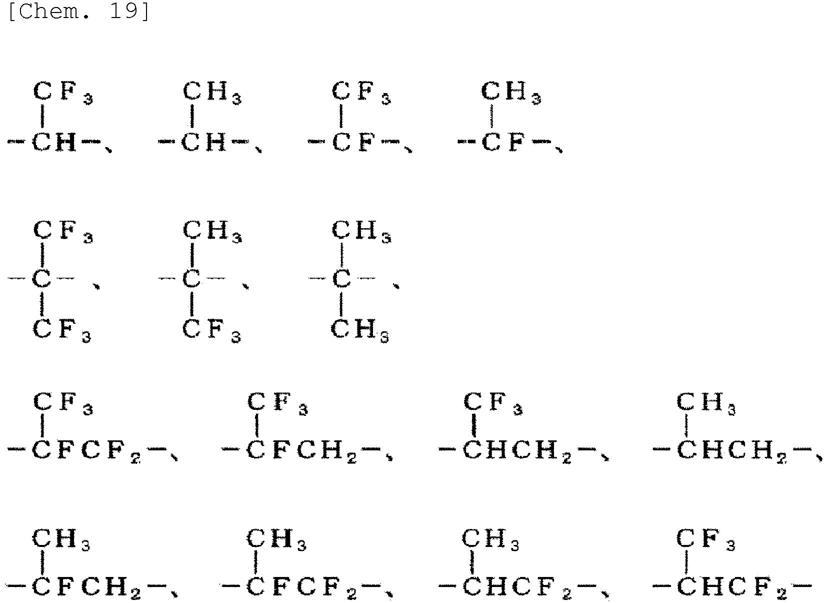 may be mentioned as examples of the branched groups.

The fluorinated alkoxy group (c) is a group obtainable by replacing at least one hydrogen atom of an alkoxy group with a fluorine atom. The fluorinated alkoxy group (c) preferably has a carbon number of 1 to 17, more preferably 1 to 6.

The fluorinated alkoxy group (c) is particularly preferably a fluorinated alkoxy group represented by X^{d}₃C-(R⁶)ₙ₃-O-, wherein three X^{d}s are the same as or different from each other, and are each H or F; R⁶ is preferably a C1-C5 alkylene group optionally containing a fluorine atom; n3 is 0 or 1; and any of the three X^{d}s contain a fluorine atom.

Specific examples of the fluorinated alkoxy group (c) include fluorinated alkoxy groups in which an oxygen atom binds to an end of an alkyl group mentioned as an example for R¹ in the formula (a-1).

The fluorinated alkyl group (a), the fluorinated alkyl group (b) containing an ether bond, and the fluorinated alkoxy group (c) in the fluorinated saturated cyclic carbonate each preferably have a fluorine content of 10% by mass or more. Too less a fluorine content may cause a failure in sufficiently achieving an effect of reducing the viscosity at low temperature and an effect of increasing the flash point. Thus, the fluorine content is more preferably 12% by mass or more, still more preferably 15% by mass or more. The upper limit thereof is usually 76% by mass.

The fluorine content of each of the fluorinated alkyl group (a), the fluorinated alkyl group (b) containing an ether bond, and the fluorinated alkoxy group (c) is a value calculated based on the corresponding structural formula by the following formula: {(Number of fluorine atoms × 19)/(Formula weight of group) } × 100 (%).

In order to give good permittivity and oxidation resistance, the fluorine content in the whole fluorinated saturated cyclic carbonate is preferably 10% by mass or more, more preferably 15% by mass or more. The upper limit thereof is usually 76% by mass.

The fluorine content in the fluorinated saturated cyclic carbonate is a value calculated based on the structural formula of the fluorinated saturated cyclic carbonate by the following formula: {(Number of fluorine atoms × 19)/(Molecular weight of fluorinated saturated cyclic carbonate)} × 100 (%).

Specific examples of the fluorinated saturated cyclic carbonate include the following.

Specific examples of the fluorinated saturated cyclic carbonate in which at least one selected from X¹ to X⁴ is - F include those represented by the following formulas.

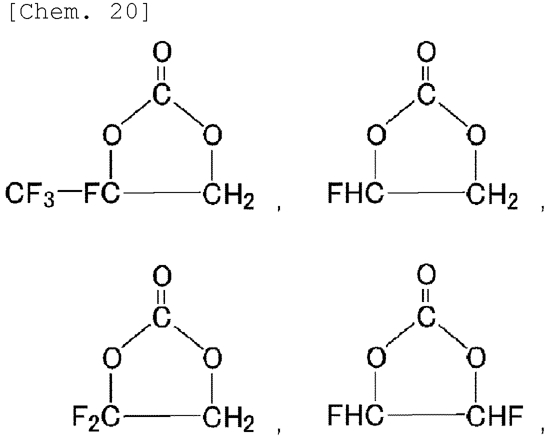

These compounds have a high withstand voltage and give good solubility of an electrolyte salt.

Alternatively, those represented by the following formulas: 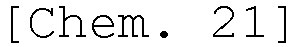 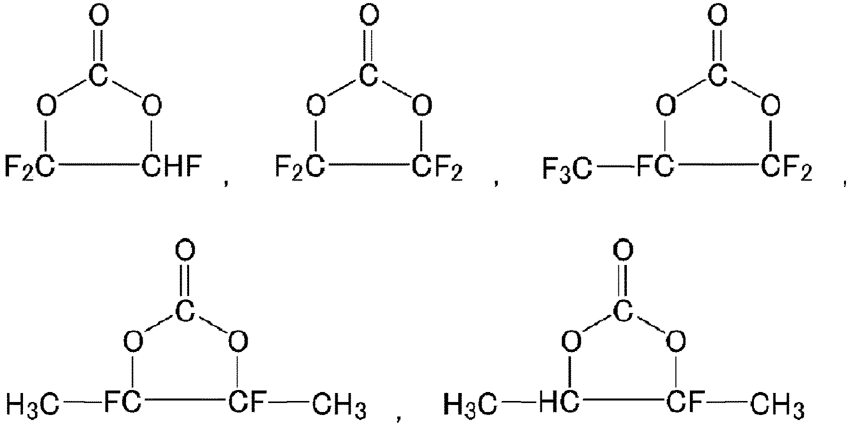 may also be used.

Those represented by the following formulas: 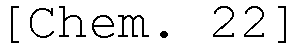 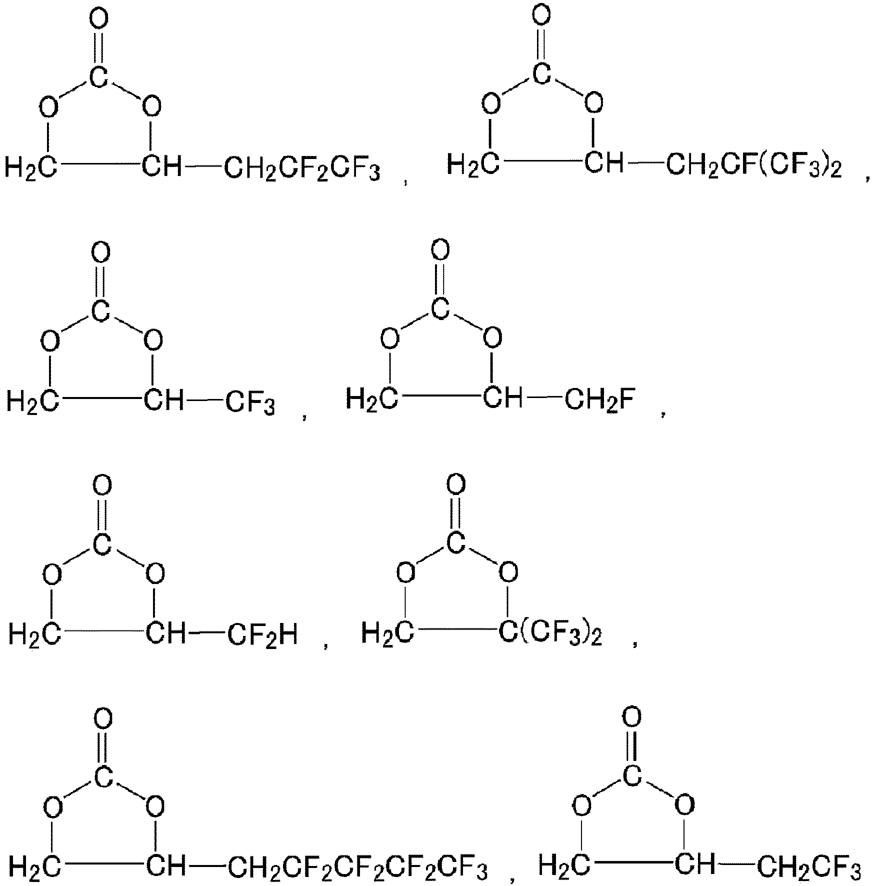 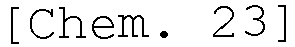 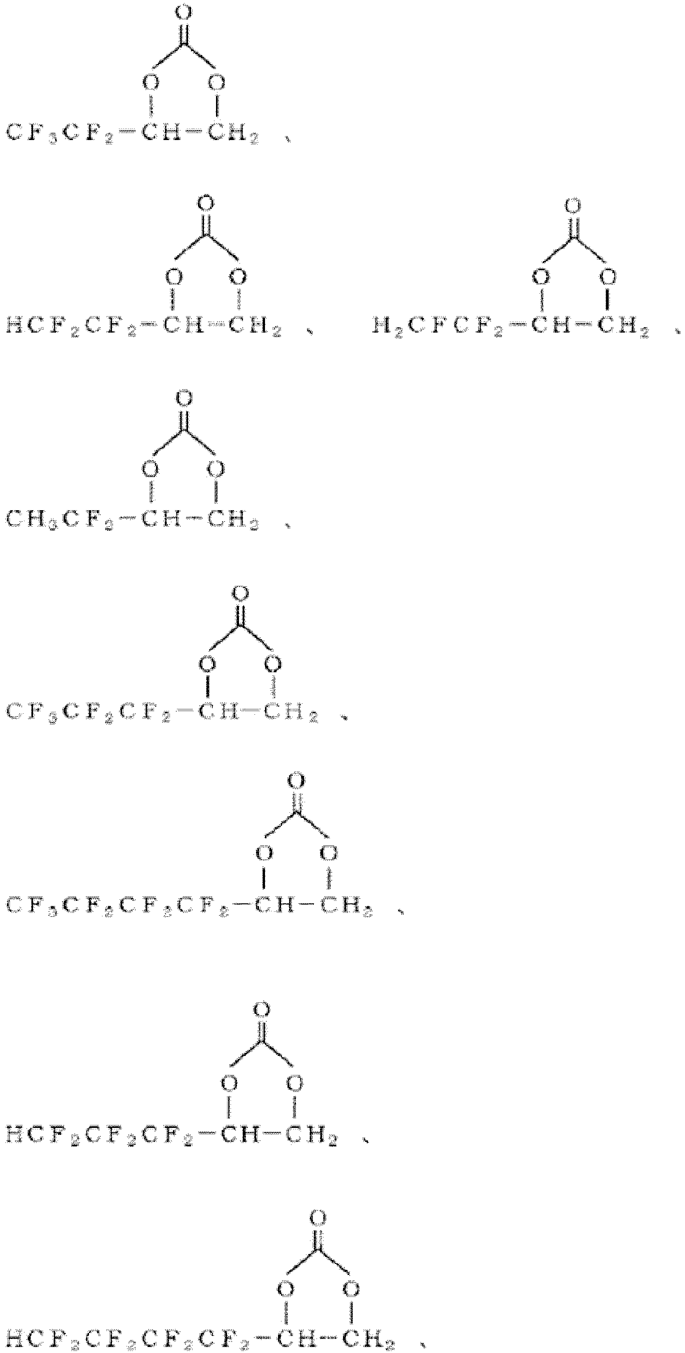 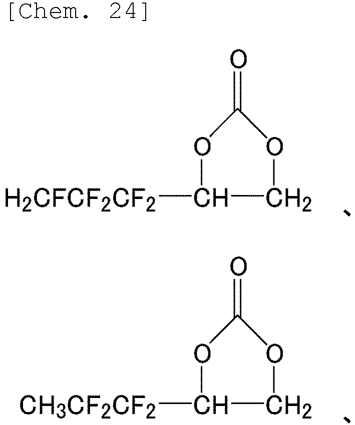 may be mentioned as specific examples of the fluorinated saturated cyclic carbonate in which at least one selected from X¹ to X⁴ is a fluorinated alkyl group (a) and the others are -H.

Those represented by the following formulas: 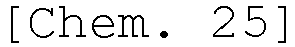 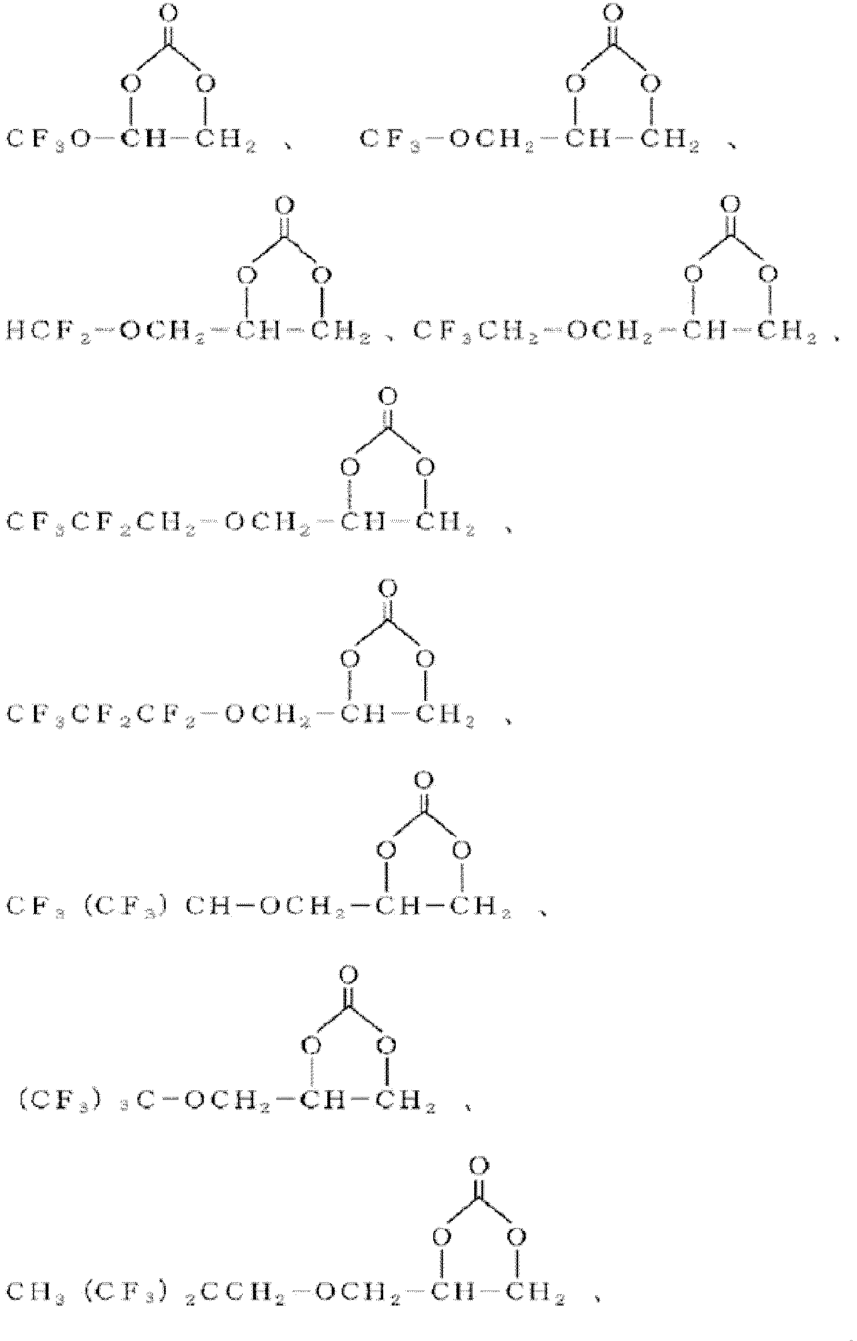 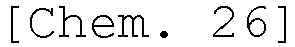 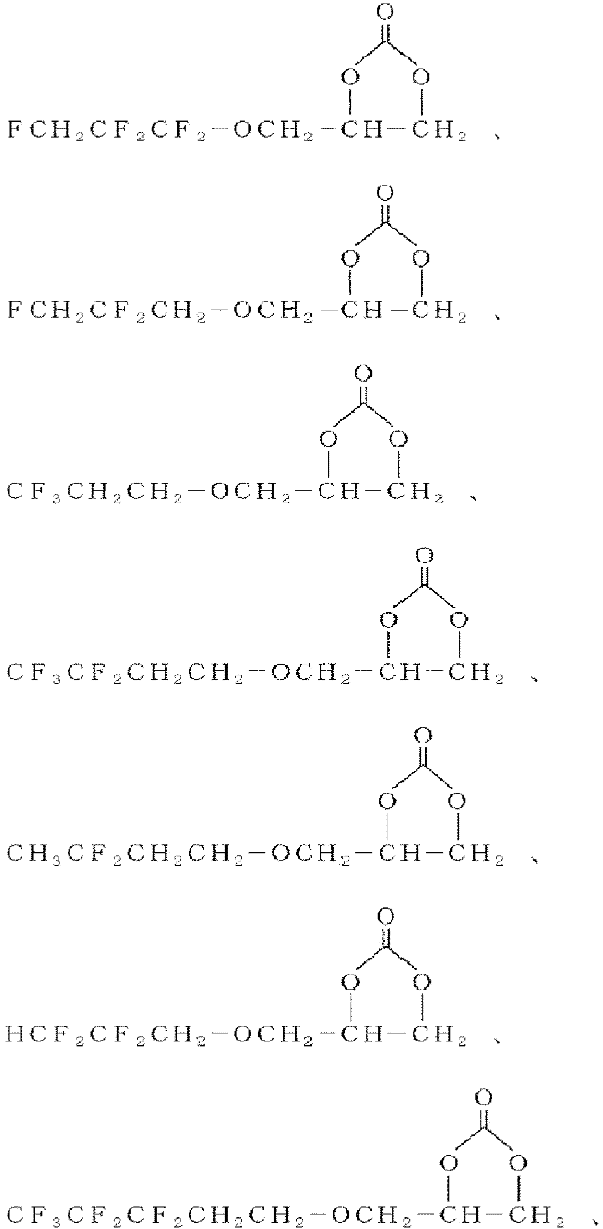 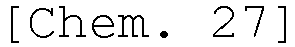 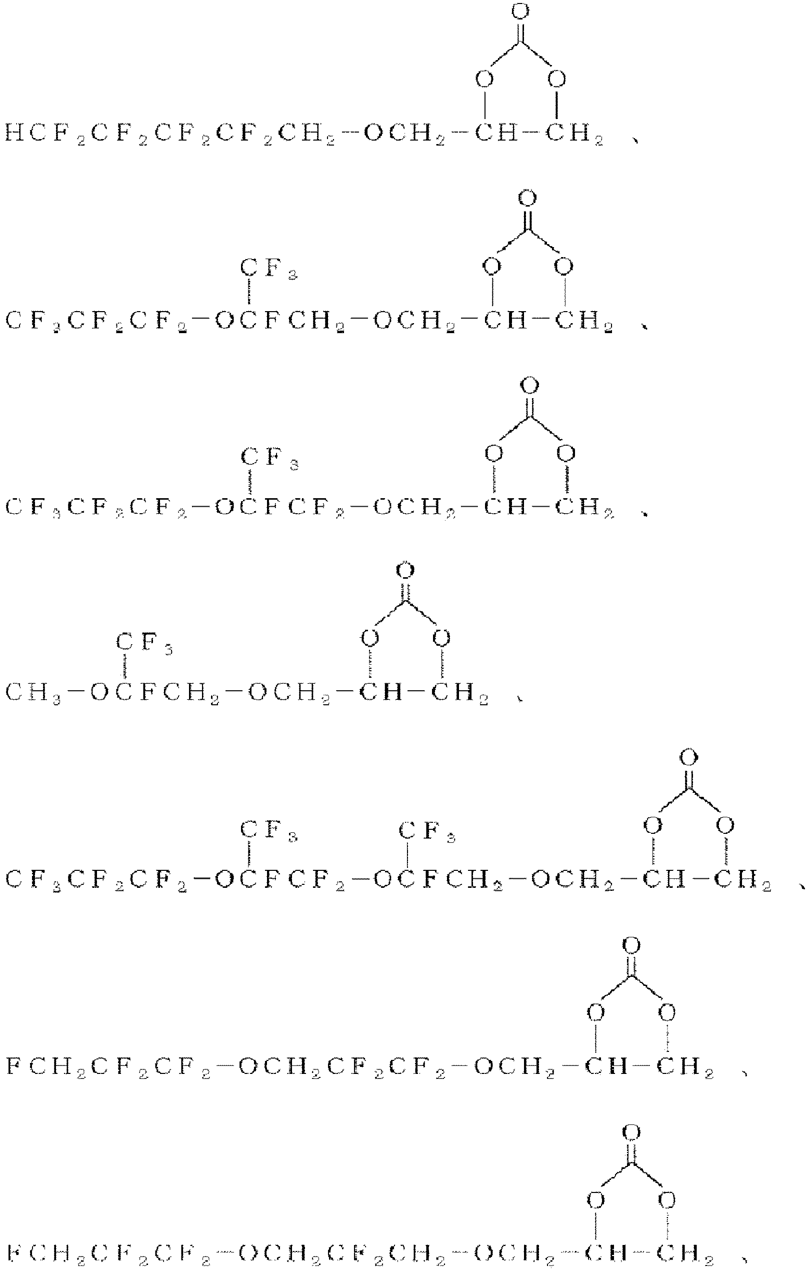 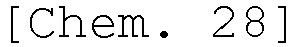 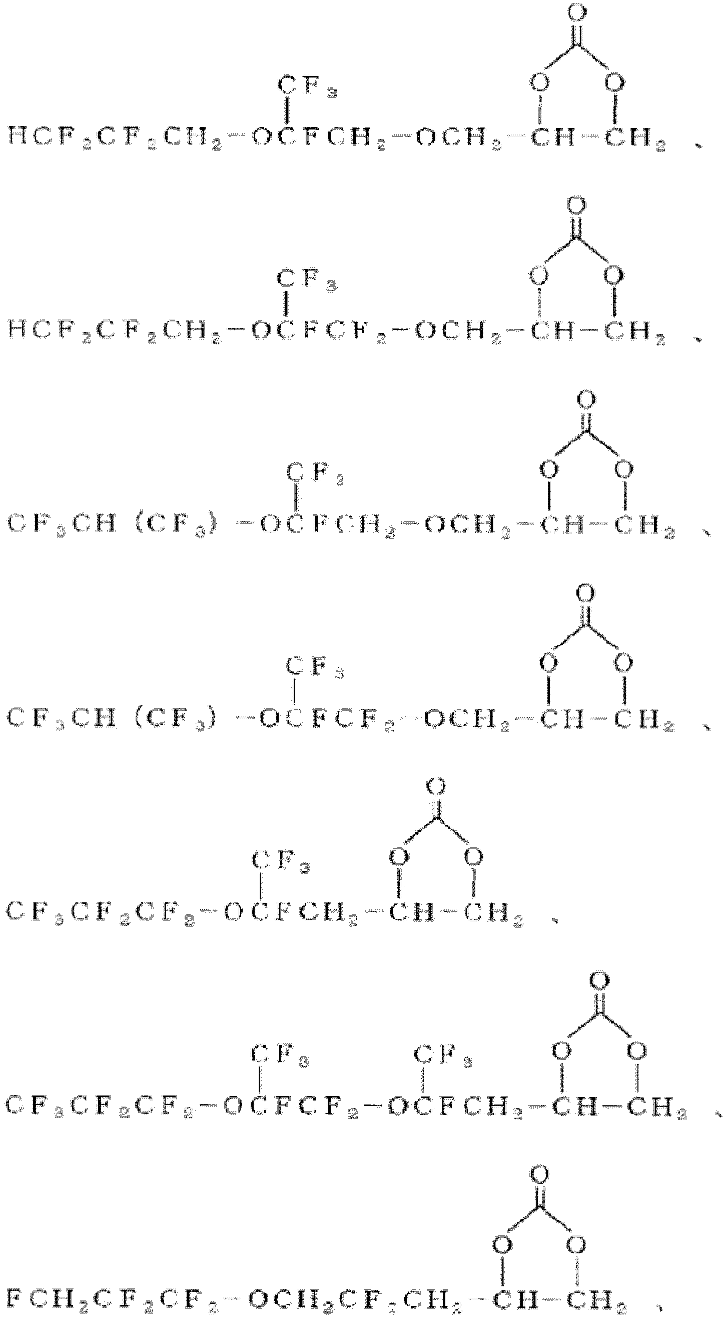 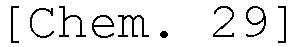 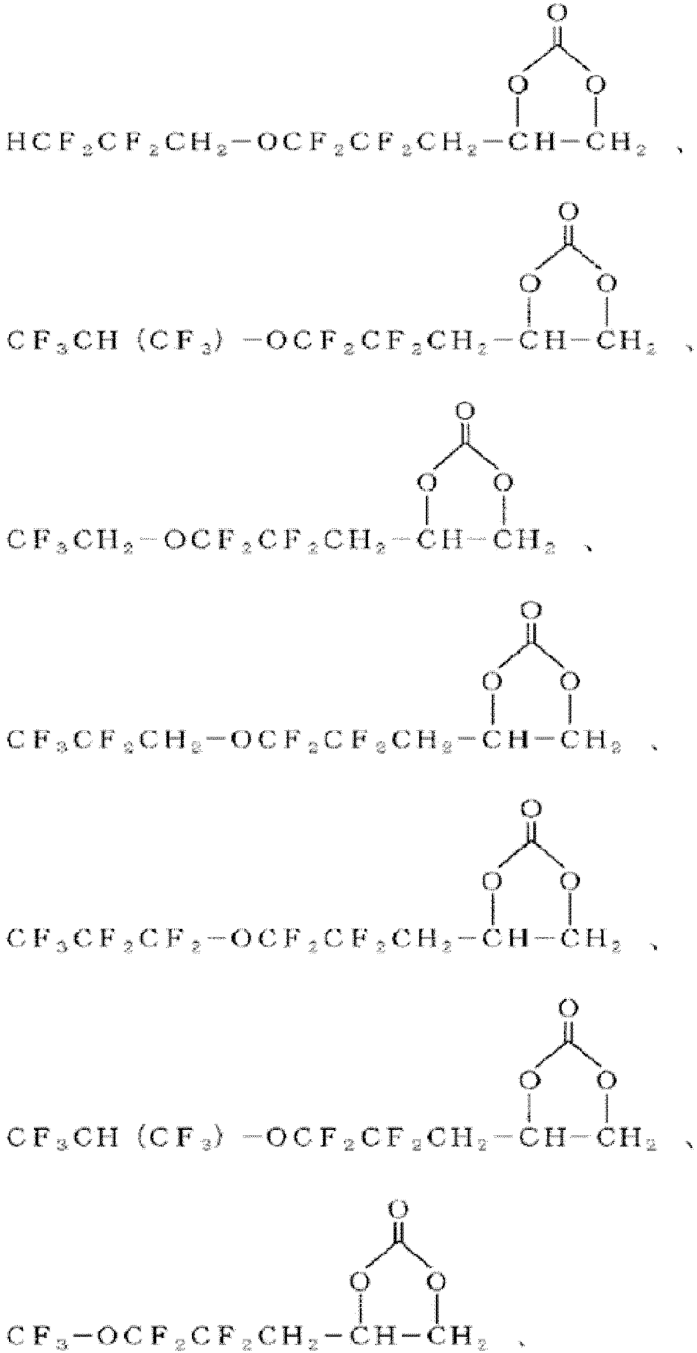 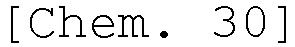 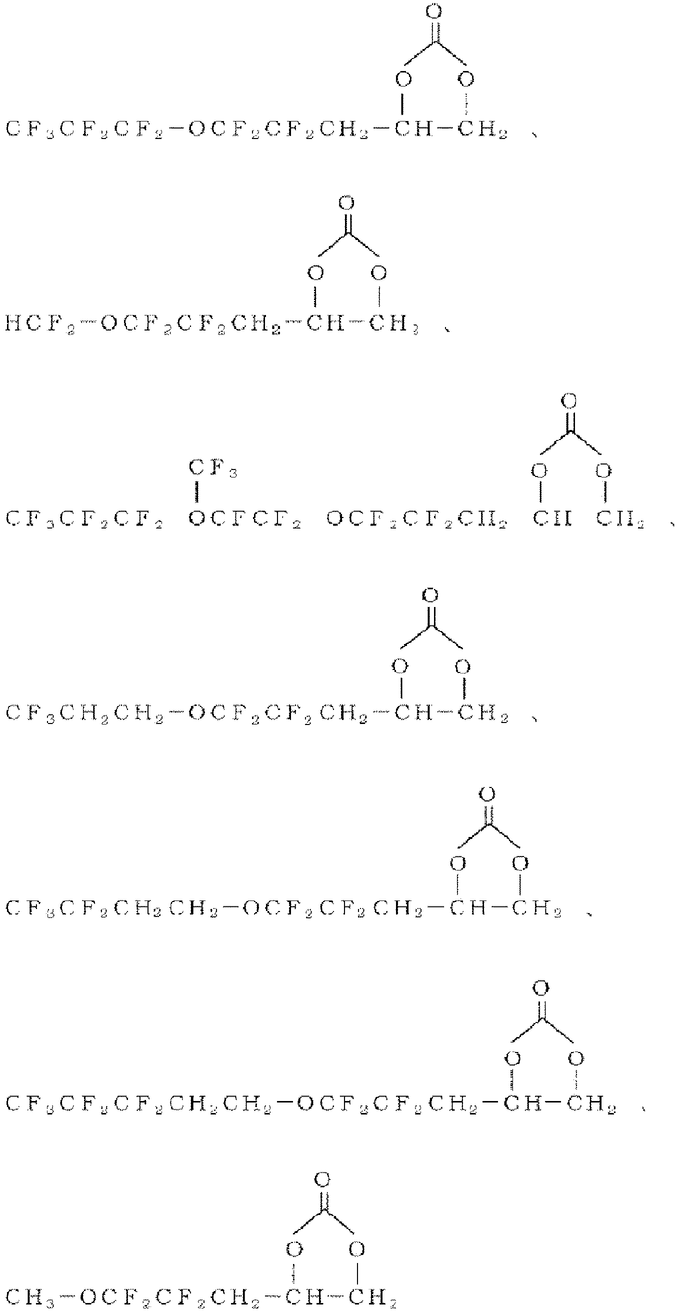 may be mentioned as specific examples of the fluorinated saturated cyclic carbonate in which at least one selected from X¹ to X⁴ is a fluorinated alkyl group (b) containing an ether bond or a fluorinated alkoxy group (c) and the others are -H.

In particular, the fluorinated saturated cyclic carbonate is preferably any of the following compounds.

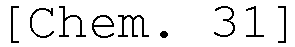

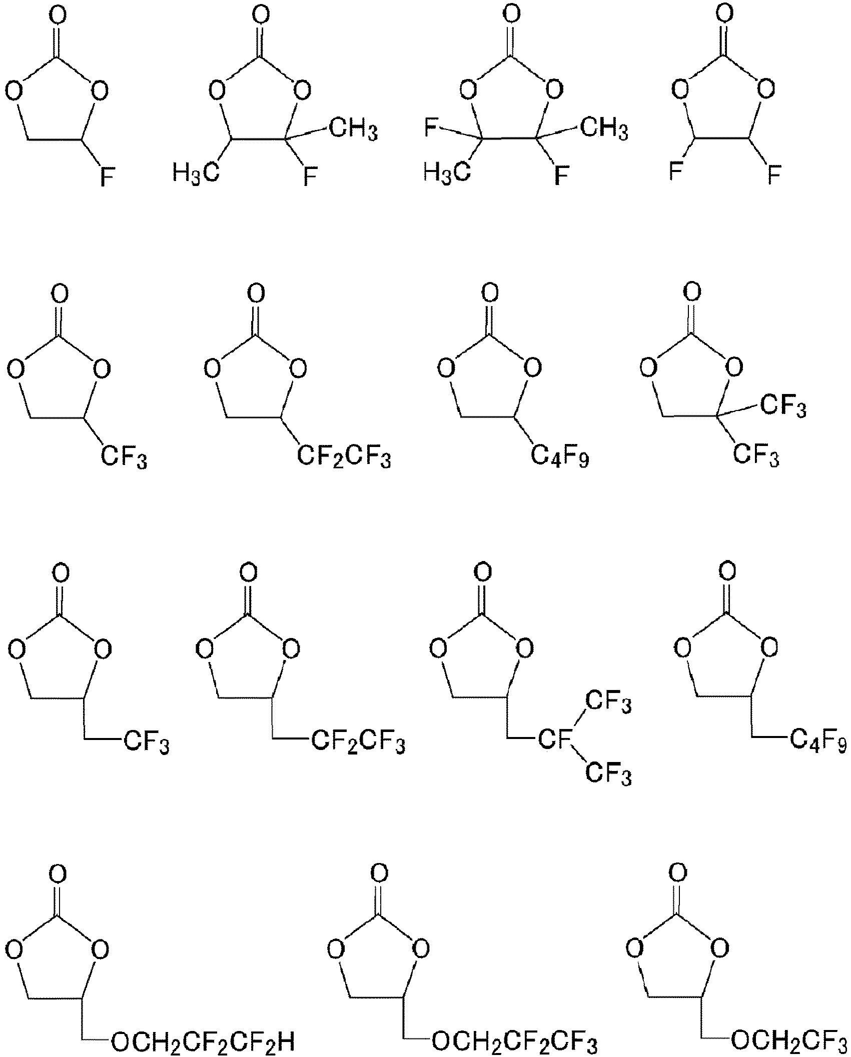

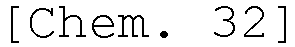

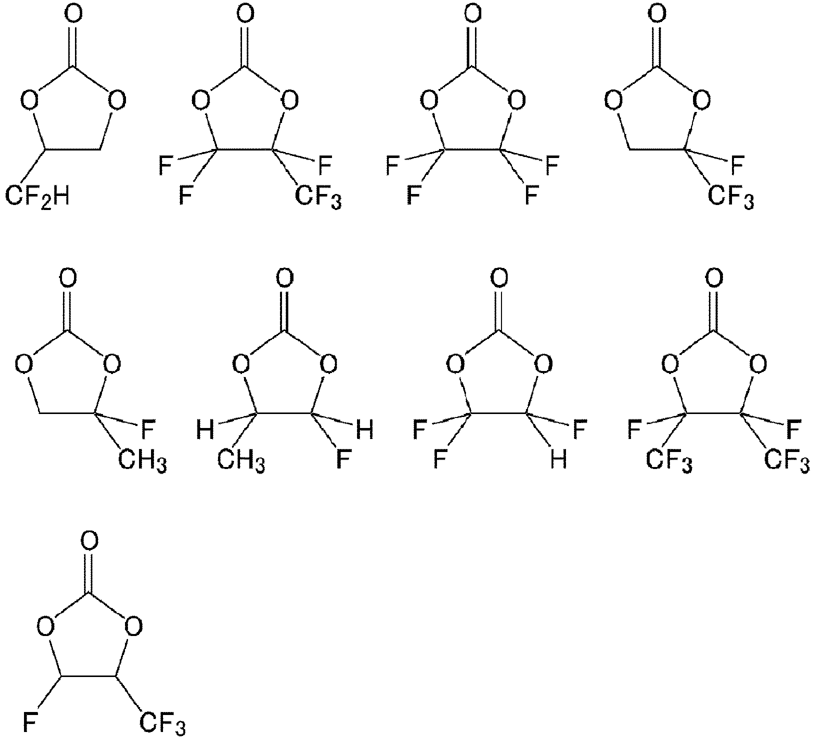

Examples of the fluorinated saturated cyclic carbonate also include trans-4,5-difluoro-1,3-dioxolan-2-one, 5-(1,1-difluoroethyl)-4,4-difluoro-1,3-dioxolan-2-one, 4-methylene-1,3-dioxolan-2-one, 4-methyl-5-trifluoromethyl-1,3-dioxolan-2-one, 4-ethyl-5-fluoro-1,3-dioxolan-2-one, 4-ethyl-5,5-difluoro-1,3-dioxolan-2-one, 4-ethyl-4,5-difluoro-1,3-dioxolan-2-one, 4-ethyl-4,5,5-trifluoro-1,3-dioxolan-2-one, 4,4-difluoro-5-methyl-1,3-dioxolan-2-one, 4-fluoro-5-methyl-1,3-dioxolan-2-one, 4-fluoro-5-trifluoromethyl-1,3-dioxolan-2-one, and 4,4-difluoro-1,3-dioxolan-2-one.

More preferred among these as the fluorinated saturated cyclic carbonate are fluoroethylene carbonate, difluoroethylene carbonate, trifluoromethylethylene carbonate, (3,3,3-trifluoropropylene carbonate), and 2,2,3,3,3-pentafluoropropylethylene carbonate.

The fluorinated unsaturated cyclic carbonate is a cyclic carbonate containing an unsaturated bond and a fluorine atom, and is preferably a fluorinated ethylene carbonate derivative substituted with a substituent containing an aromatic ring or a carbon-carbon double bond. Specific examples thereof include 4,4-difluoro-5-phenyl ethylene carbonate, 4,5-difluoro-4-phenyl ethylene carbonate, 4-fluoro-5-phenyl ethylene carbonate, 4-fluoro-5-vinyl ethylene carbonate, 4-fluoro-4-phenyl ethylene carbonate, 4,4-difluoro-4-vinyl ethylene carbonate, 4,4-difluoro-4-allyl ethylene carbonate, 4-fluoro-4-vinyl ethylene carbonate, 4-fluoro-4,5-diallyl ethylene carbonate, 4,5-difluoro-4-vinyl ethylene carbonate, 4,5-difluoro-4,5-divinyl ethylene carbonate, and 4,5-difluoro-4,5-diallyl ethylene carbonate.

One fluorinated cyclic carbonate may be used alone or two or more thereof may be used in any combination at any ratio.

The fluorinated cyclic carbonate, when contained, is preferably present in an amount of 5 to 90% by volume, more preferably 10 to 60% by volume, still more preferably 15 to 45% by volume, relative to the solvent.

The acyclic carbonate may be either a non-fluorinated acyclic carbonate or a fluorinated acyclic carbonate.

Examples of the non-fluorinated acyclic carbonate include hydrocarbon-based acyclic carbonates such as CH₃OCOOCH₃ (dimethyl carbonate, DMC), CH₃CH₂OCOOCH₂CH₃ (diethyl carbonate, DEC), CH₃CH₂OCOOCH₃ (ethyl methyl carbonate, EMC), CH₃OCOOCH₂CH₂CH₃ (methyl propyl carbonate), methyl butyl carbonate, ethyl propyl carbonate, ethyl butyl carbonate, dipropyl carbonate, dibutyl carbonate, methyl isopropyl carbonate, methyl-2-phenyl phenyl carbonate, phenyl-2-phenyl phenyl carbonate, trans-2,3-pentylene carbonate, trans-2,3-butylene carbonate, and ethyl phenyl carbonate. Preferred among these is at least one selected from the group consisting of ethyl methyl carbonate, diethyl carbonate, and dimethyl carbonate.

One non-fluorinated acyclic carbonate may be used alone or two or more thereof may be used in any combination at any ratio.

The non-fluorinated acyclic carbonate, when contained, is preferably present in an amount of 10 to 90% by volume, more preferably 40 to 85% by volume, still more preferably 50 to 80% by volume, relative to the solvent.

The fluorinated acyclic carbonate is an acyclic carbonate containing a fluorine atom. A solvent containing a fluorinated acyclic carbonate can suitably be used at high voltage.

An example of the fluorinated acyclic carbonate is a compound represented by the following formula (B):

Rf^{a}OCOOR⁷ (B)

wherein Rf^{a} is a C1-C7 fluorinated alkyl group; and R⁷ is a C1-C7 alkyl group optionally containing a fluorine atom.

Rf^{a} is a C1-C7 fluorinated alkyl group and R⁷ is a C1-C7 alkyl group optionally containing a fluorine atom.

The fluorinated alkyl group is a group obtainable by replacing at least one hydrogen atom of an alkyl group with a fluorine atom. When R⁷ is an alkyl group containing a fluorine atom, it is a fluorinated alkyl group.

In order to give low viscosity, Rf^{a} and R⁷ each preferably have a carbon number of 1 to 7, more preferably 1 to 2.

Too large a carbon number may cause poor low-temperature characteristics and low solubility of an electrolyte salt. Too small a carbon number may cause low solubility of an electrolyte salt, low discharge efficiency, and increased viscosity, for example.

Examples of the fluorinated alkyl group having a carbon number of 1 include CFH₂-, CF₂H-, and CF₃-. In order to give high-temperature storage characteristics, particularly preferred is CFH₂- or CF₃-.

In order to give good solubility of an electrolyte salt, preferred examples of the fluorinated alkyl group having a carbon number of 2 or greater include fluorinated alkyl groups represented by the following formula (d-1):

R^{d1}-R^{d2}- (d-1)

wherein R^{d1} is an alkyl group having a carbon number of 1 or greater and optionally containing a fluorine atom; R^{d2} is a C1-C3 alkylene group optionally containing a fluorine atom; and at least one of R^{d1} or R^{d2} contains a fluorine atom.

R^{d1} and R^{d2} each may further contain an atom other than carbon, hydrogen, and fluorine atoms.

R^{d1} is an alkyl group having a carbon number of 1 or greater and optionally containing a fluorine atom. R^{d1} is preferably a C1-C6 linear or branched alkyl group. The carbon number of R^{d1} is more preferably 1 to 3.

Specifically, for example, CH₃-, CF₃-, CH₃CH₂-, CH₃CH₂CH₂-, CH₃CH₂CH₂CH₂-, and groups represented by the following formulas: 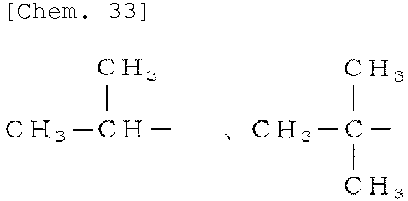 may be mentioned as linear or branched alkyl groups for R^{d1}.

Examples of R^{d1} as a linear alkyl group containing a fluorine atom include CF₃-, CF₃CH₂-, CF₃CF₂-, CF₃CH₂CH₂-, CF₃CF₂CH₂-, CF₃CF₂CF₂-, CF₃CH₂CF₂-, CF₃CH₂CH₂CH₂-, CF₃CF₂CH₂CH₂-, CF₃CH₂CF₂CH₂-, CF₃CF₂CF₂CH₂-, CF₃CF₂CF₂CF₂-, CF₃CF₂CH₂CF₂-, CF₃CH₂CH₂CH₂CH₂-, CF₃CF₂CH₂CH₂CH₂-, CF₃CH₂CF₂CH₂CH₂-, CF₃CF₂CF₂CH₂CH₂-, CF₃CF₂CF₂CF₂CH₂-, CF₃CF₂CH₂CF₂CH₂-, CF₃CF₂CH₂CH₂CH₂CH₂-, CF₃CF₂CF₂CF₂CH₂CH₂-, CF₃CF₂CH₂CF₂CH₂CH₂-, HCF₂-, HCF₂CH₂-, HCF₂CF₂-, HCF₂CH₂CH₂-, HCF₂CF₂CH₂-, HCF₂CH₂CF₂-, HCF₂CF₂CH₂CH₂-, HCF₂CH₂CF₂CH₂-, HCF₂CF₂CF₂CF₂-, HCF₂CF₂CH₂CH₂CH₂-, HCF₂CH₂CF₂CH₂CH₂-, HCF₂CF₂CF₂CF₂CH₂-, HCF₂CF₂CF₂CF₂CH₂CH₂-, FCH₂-, FCH₂CH₂-, FCH₂CF₂-, FCH₂CF₂CH₂-, FCH₂CF₂CF₂-, CH₃CF₂CH₂-, CH₃CF₂CF₂-, CH₃CF₂CH₂CF₂-, CH₃CF₂CF₂CF₂-, CH₃CH₂CF₂CF₂-, CH₃CF₂CH₂CF₂CH₂-, CH₃CF₂CF₂CF₂CH₂-, CH₃CF₂CF₂CH₂CH₂-, CH₃CH₂CF₂CF₂CH₂-, CH₃CF₂CH₂CF₂CH₂CH₂-, CH₃CF₂CH₂CF₂CH₂CH₂-, HCFClCF₂CH₂-, HCF₂CFClCH₂-, HCF₂CFClCF₂CFClCH₂-, and HCFClCF₂CFClCF₂CH₂-.

Examples of R^{d1} as a branched alkyl group containing a fluorine atom include those represented by the following formulas.

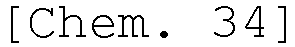

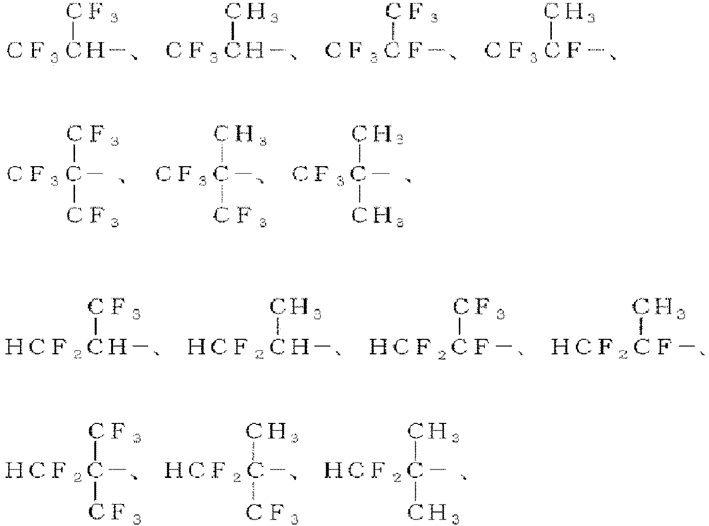

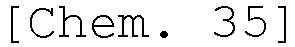

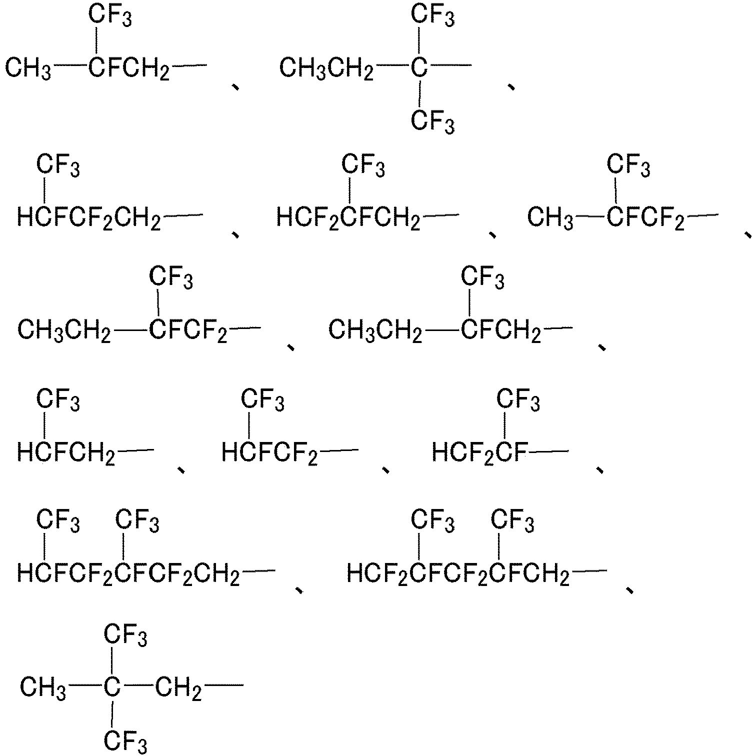

The presence of a branch such as CH₃- or CF₃- may easily cause high viscosity. Thus, the number of such branches is more preferably small (one) or zero.

R^{d2} is a C1-C3 alkylene group optionally containing a fluorine atom. R^{d2} may be either linear or branched. Examples of a minimum structural unit constituting such a linear or branched alkylene group are shown below. R^{d2} is constituted by one or combination of these units.
(i) Linear minimum structural units

   -CH₂-, -CHF-, -CF₂-, -CHCl-, -CFCl-, -CCl₂-
(ii) Branched minimum structural units

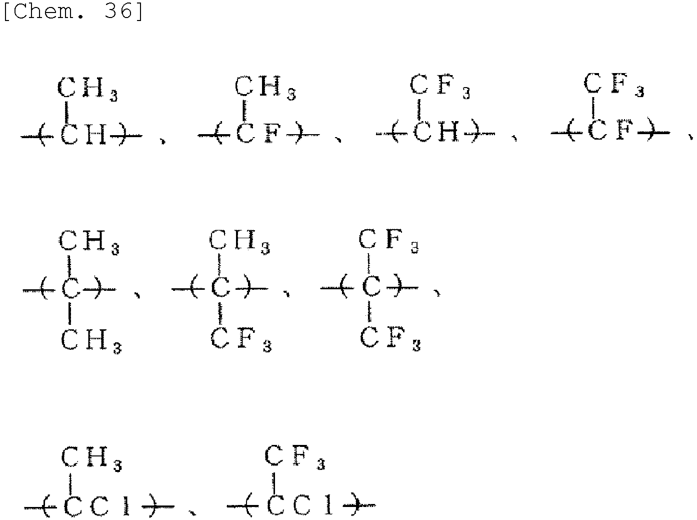

Preferred among these exemplified units are Cl-free structural units because such units may not be dehydrochlorinated by a base, and thus may be more stable.

R^{d2}, when it is a linear group, consists only of any of the above linear minimum structural units, and is preferably -CH₂-, -CH₂CH₂-, or -CF₂-. In order to further improve the solubility of an electrolyte salt, -CH₂- or - CH₂CH₂- is more preferred.

R^{d2}, when it is a branched group, includes at least one of the above branched minimum structural units. A preferred example thereof is a group represented by - (CX^{a}X^{b})- (wherein X^{a} is H, F, CH₃, or CF₃; X^{b} is CH₃ or CF₃; when X^{b} is CF₃, X^{a} is H or CH₃). Such a group can much further improve the solubility of an electrolyte salt.

For example, CF₃CF₂-, HCF₂CF₂-, H₂CFCF₂-, CH₃CF₂-, CF₃CH₂-, CF₃CF₂CF₂-, HCF₂CF₂CF₂-, H₂CFCF₂CF₂-, CH₃CF₂CF₂-, and those represented by the following formulas: 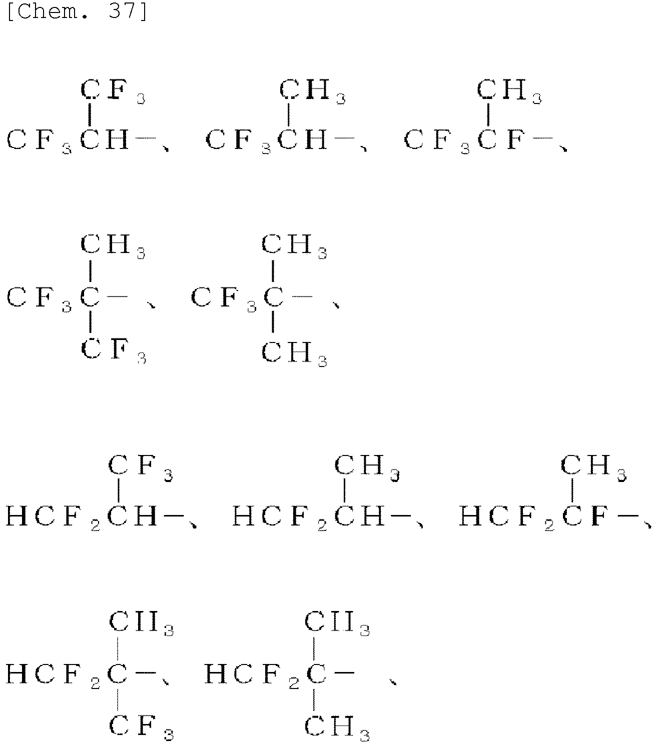 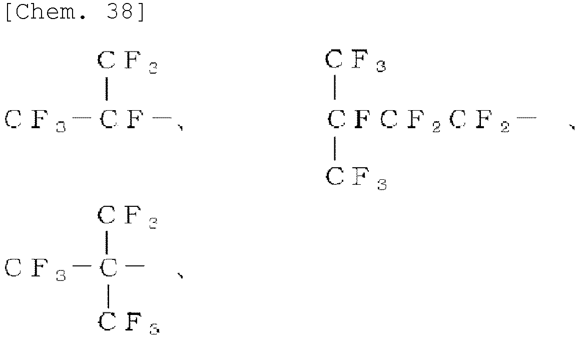 may be specifically mentioned as preferred examples of the fluorinated alkyl group.

The fluorinated alkyl group for Rf² and R⁷ is preferably CF₃-, CF₃CF₂-, (CF₃)₂CH-, CF₃CH₂-, C₂F₅CH₂-, CF₃CF₂CH₂-, HCF₂CF₂CH₂-, CF₃CFHCF₂CH₂-, CFH₂-, and CF₂H-. In order to give high incombustibility and good rate characteristics and oxidation resistance, more preferred are CF₃CH₂-, CF₃CF₂CH₂-, HCF₂CF₂CH₂-, CFH₂-, and CF₂H-.

R⁷, when it is an alkyl group free from a fluorine atom, is a C1-C7 alkyl group. In order to give low viscosity, R⁷ preferably has a carbon number of 1 to 4, more preferably 1 to 3.

Examples of the alkyl group free from a fluorine atom include CH₃-, CH₃CH₂-, (CH₃)₂CH-, and C₃H₇-. In order to give low viscosity and good rate characteristics, preferred are CH₃- and CH₃CH₂-.

The fluorinated acyclic carbonate preferably has a fluorine content of 15 to 70% by mass. The fluorinated acyclic carbonate having a fluorine content within the above range can maintain the miscibility with a solvent and the solubility of a salt. The fluorine content is more preferably 20% by mass or more, still more preferably 30% by mass or more, particularly preferably 35% by mass or more, while more preferably 60% by mass or less, still more preferably 50% by mass or less.

In the disclosure, the fluorine content is a value calculated based on the structural formula of the fluorinated acyclic carbonate by the following formula: {(Number of fluorine atoms × 19)/(Molecular weight of fluorinated acyclic carbonate)} × 100 (%).

In order to give low viscosity, the fluorinated acyclic carbonate is preferably any of the following compounds.

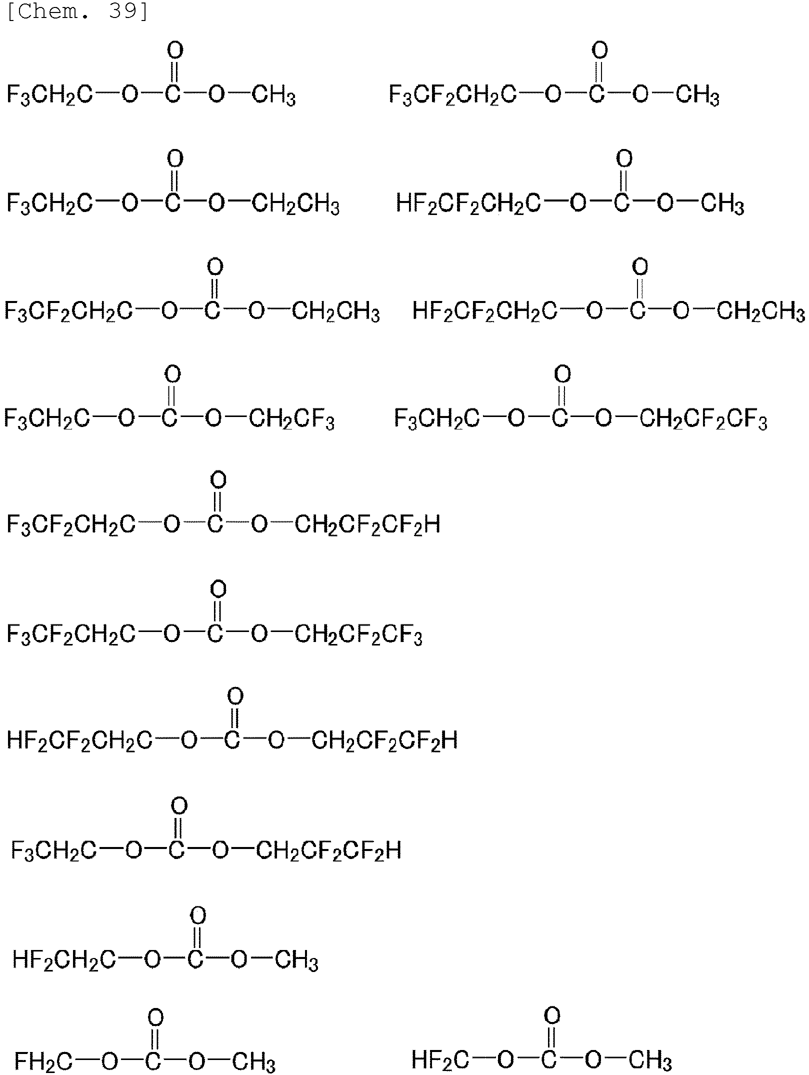

The fluorinated acyclic carbonate is particularly preferably methyl 2,2,2-trifluoroethyl carbonate

(F₃CH₂COC(=O)OCH₃).

One fluorinated acyclic carbonate may be used alone, or two or more thereof may be used in any combination at any ratio.

The fluorinated acyclic carbonate, when contained, is preferably present in an amount of 10 to 90% by volume, more preferably 40 to 85% by volume, still more preferably 50 to 80% by volume, relative to the solvent.

The carboxylate may be either a cyclic carboxylate or an acyclic carboxylate.

The cyclic carboxylate may be either a non-fluorinated cyclic carboxylate or a fluorinated cyclic carboxylate.

Examples of the non-fluorinated cyclic carboxylate include a non-fluorinated saturated cyclic carboxylate, and preferred is a non-fluorinated saturated cyclic carboxylate containing a C2-C4 alkylene group.

Specific examples of the non-fluorinated saturated cyclic carboxylate containing a C2-C4 alkylene group include β-propiolactone, γ-butyrolactone, ε-caprolactone, δ-valerolactone, and α-methyl-γ-butyrolactone. In order to improve the degree of dissociation of lithium ions and to improve the load characteristics, particularly preferred among these are γ-butyrolactone and δ-valerolactone.

One non-fluorinated saturated cyclic carboxylate may be used alone or two or more thereof may be used in any combination at any ratio.

The non-fluorinated saturated cyclic carboxylate, when contained, is preferably present in an amount of 0 to 90% by volume, more preferably 0.001 to 90% by volume, still more preferably 1 to 60% by volume, particularly preferably 5 to 40% by volume, relative to the solvent.

The acyclic carboxylate may be either a non-fluorinated acyclic carboxylate or a fluorinated acyclic carboxylate. The solvent containing the acyclic carboxylate enables further reduction of an increase in resistance after high-temperature storage of the electrolyte solution.

Examples of the non-fluorinated acyclic carboxylate include methyl acetate, ethyl acetate, propyl acetate, butyl acetate, methyl propionate, ethyl propionate, propyl propionate, butyl propionate, tert-butyl propionate, tert-butyl butyrate, sec-butyl propionate, sec-butyl butyrate, n-butyl butyrate, methyl pyrophosphate, ethyl pyrophosphate, tert-butyl formate, tert-butyl acetate, sec-butyl formate, sec-butyl acetate, n-hexyl pivalate, n-propyl formate, n-propyl acetate, n-butyl formate, n-butyl pivalate, n-octyl pivalate, ethyl 2-(dimethoxyphosphoryl)acetate, ethyl 2-(dimethylphosphoryl)acetate, ethyl 2-(diethoxyphosphoryl)acetate, ethyl 2-(diethylphosphoryl)acetate, isopropyl propionate, isopropyl acetate, ethyl formate, ethyl 2-propynyl oxalate, isopropyl formate, isopropyl butyrate, isobutyl formate, isobutyl propionate, isobutyl butyrate, and isobutyl acetate.

Preferred among these are butyl acetate, methyl propionate, ethyl propionate, propyl propionate, and butyl propionate, and particularly preferred are ethyl propionate and propyl propionate.

One non-fluorinated acyclic carboxylate may be used alone or two or more thereof may be used in any combination at any ratio.

The non-fluorinated acyclic carboxylate, when contained, is preferably present in an amount of 0 to 90% by volume, more preferably 0.001 to 90% by volume, still more preferably 1 to 60% by volume, particularly preferably 5 to 40% by volume, relative to the solvent.

The fluorinated acyclic carboxylate is an acyclic carboxylate containing a fluorine atom. A solvent containing a fluorinated acyclic carboxylate can be suitably used at high voltage.

In order to achieve good miscibility with other solvents and to give good oxidation resistance, preferred examples of the fluorinated acyclic carboxylate include a fluorinated acyclic carboxylate represented by the following formula:

R³¹COOR³²

(wherein R³¹ and R³² are each independently a C1-C4 alkyl group optionally containing a fluorine atom, and at least one of R³¹ or R³² contains a fluorine atom).

Examples of R³¹ and R³² include non-fluorinated alkyl groups such as a methyl group (-CH₃), an ethyl group (-CH₂CH₃), a propyl group (-CH₂CH₂CH₃), an isopropyl group (-CH(CH₃)₂), a normal butyl group (-CH₂CH₂CH₂CH₃), and a tertiary butyl group (-C(CH₃)₃); and fluorinated alkyl groups such as -CF₃, -CF₂H, -CFH₂, -CF₂CF₃, -CF₂CF₂H, - CF₂CFH₂, -CH₂CF₃, -CH₂CF₂H, -CH₂CFH₂, -CF₂CF₂CF₃, -CF₂CF₂CF₂H, -CF₂CF₂CFH₂, -CH₂CF₂CF₃, -CH₂CF₂CF₂H, -CH₂CF₂CFH₂, -CH₂CH₂CF₃, - CH₂CH₂CF₂H, -CH₂CH₂CFH₂, -CF(CF₃)₂, -CF(CF₂H)₂, -CF(CFH₂)₂, - CH(CF₃)₂, -CH(CF₂H)₂, -CH(CFH₂)₂, -CF(OCH₃)CF₃, -CF₂CF₂CF₂CF₃, -CF₂CF₂CF₂CF₂H, -CF₂CF₂CF₂CFH₂, -CH₂CF₂CF₂CF₃, -CH₂CF₂CF₂CF₂H, - CH₂CF₂CF₂CFH₂, -CH₂CH₂CF₂CF₃, -CH₂CH₂CF₂CF₂H, -CH₂CH₂CF₂CFH₂, - CH₂CH₂CH₂CF₃, -CH₂CH₂CH₂CF₂H, -CH₂CH₂CH₂CFH₂, -CF(CF₃)CF₂CF₃, - CF(CF₂H)CF₂CF₃, -CF(CFH₂)CF₂CF₃, -CF(CF₃)CF₂CF₂H, - CF(CF₃)CF₂CFH₂, -CF(CF₃)CH₂CF₃, -CF(CF₃)CH₂CF₂H, - CF(CF₃)CH₂CFH₂, -CH(CF₃)CF₂CF₃, -CH(CF₂H)CF₂CF₃, - CH(CFH₂)CF₂CF₃, -CH(CF₃)CF₂CF₂H, -CH(CF₃)CF₂CFH₂, - CH(CF₃)CH₂CF₃, -CH(CF₃)CH₂CF₂H, -CH(CF₃)CH₂CFH₂, - CF₂CF(CF₃)CF₃, -CF₂CF(CF₂H)CF₃, -CF₂CF(CFH₂)CF₃, - CF₂CF(CF₃)CF₂H, -CF₂CF(CF₃)CFH₂, -CH₂CF(CF₃)CF₃, - CH₂CF(CF₂H)CF₃, -CH₂CF(CFH₂)CF₃, -CH₂CF(CF₃)CF₂H, - CH₂CF(CF₃)CFH₂, -CH₂CH(CF₃)CF₃, -CH₂CH(CF₂H)CF₃, - CH₂CH(CFH₂)CF₃, -CH₂CH(CF₃)CF₂H, -CH₂CH(CF₃)CFH₂, - CF₂CH(CF₃)CF₃, -CF₂CH(CF₂H)CF₃, -CF₂CH(CFH₂)CF₃, - CF₂CH(CF₃)CF₂H, -CF₂CH(CF₃)CFH₂, -C(CF₃)₃, -C(CF₂H)₃, and - C(CFH₂)₃. In order to improve the miscibility with other solvents, viscosity, and oxidation resistance, particularly preferred among these are a methyl group, an ethyl group, - CF₃, -CF₂H, -CF₂CF₃, -CH₂CF₃, -CH₂CF₂H, -CH₂CFH₂, -CH₂CH₂CF₃, - CH₂CF₂CF₃, -CH₂CF₂CF₂H, and -CH₂CF₂CFH₂.

Specific examples of the fluorinated acyclic carboxylate include one or two or more of CF₃CH₂C(=O)OCH₃ (methyl 3,3,3-trifluoropropionate), HCF₂C(=O)OCH₃ (methyl difluoroacetate), HCF₂C(=O)OC₂H₅ (ethyl difluoroacetate), CF₃C(=O)OCH₂CH₂CF₃, CF₃C(=O)OCH₂C₂F₅, CF₃C(=O)OCH₂CF₂CF₂H (2,2,3,3-tetrafluoropropyl trifluoroacetate), CF₃C(=O)OCH₂CF₃, CF₃C(=O)OCH(CF₃)₂, ethyl pentafluorobutyrate, methyl pentafluoropropionate, ethyl pentafluoropropionate, methyl heptafluoroisobutyrate, isopropyl trifluorobutyrate, ethyl trifluoroacetate, tert-butyl trifluoroacetate, n-butyl trifluoroacetate, methyl tetrafluoro-2-(methoxy)propionate, 2,2-difluoroethyl acetate, 2,2,3,3-tetrafluoropropyl acetate, CH₃C(=O)OCH₂CF₃ (2,2,2-trifluoroethyl acetate), 1H,1H-heptafluorobutyl acetate, methyl 4,4,4-trifluorobutyrate, ethyl 4,4,4-trifluorobutyrate, ethyl 3,3,3-trifluoropropionate, 3,3,3-trifluoropropyl 3,3,3-trifluoropropionate, ethyl 3-(trifluoromethyl)butyrate, methyl 2,3,3,3-tetrafluoropropionate, butyl 2,2-difluoroacetate, methyl 2,2,3,3-tetrafluoropropionate, methyl 2-(trifluoromethyl)-3,3,3-trifluoropropionate, and methyl heptafluorobutyrate.

In order to achieve good miscibility with other solvents and good rate characteristics, preferred among these are CF₃CH₂C(=O)OCH₃, HCF₂C(=O)OCH₃, HCF₂C(=O)OC₂H₅, CF₃C(=O)OCH₂C₂F₅, CF₃C(=O)OCH₂CF₂CF₂H, CF₃C(=O)OCH₂CF₃, CF₃C(=O)OCH(CF₃)₂, ethyl pentafluorobutyrate, methyl pentafluoropropionate, ethyl pentafluoropropionate, methyl heptafluoroisobutyrate, isopropyl trifluorobutyrate, ethyl trifluoroacetate, tert-butyl trifluoroacetate, n-butyl trifluoroacetate, methyl tetrafluoro-2-(methoxy)propionate, 2,2-difluoroethyl acetate, 2,2,3,3-tetrafluoropropyl acetate, CH₃C(=O)OCH₂CF₃, 1H,1H-heptafluorobutyl acetate, methyl 4,4,4-trifluorobutyrate, ethyl 4,4,4-trifluorobutyrate, ethyl 3,3,3-trifluoropropionate, 3,3,3-trifluoropropyl 3,3,3-trifluoropropionate, ethyl 3-(trifluoromethyl)butyrate, methyl 2,3,3,3-tetrafluoropropionate, butyl 2,2-difluoroacetate, methyl 2,2,3,3-tetrafluoropropionate, methyl 2-(trifluoromethyl)-3,3,3-trifluoropropionate, and methyl heptafluorobutyrate, more preferred are CF₃CH₂C(=O)OCH₃, HCF₂C(=O)OCH₃, HCF₂C(=O)OC₂H₅, and CH₃C(=O)OCH₂CF₃, and particularly preferred are HCF₂C(=O)OCH₃, HCF₂C(=O)OC₂H₅, and CH₃C(=O)OCH₂CF₃.

One fluorinated acyclic carboxylate may be used alone or two or more thereof may be used in any combination at any ratio.

The fluorinated acyclic carboxylate, when contained, is preferably present in an amount of 10 to 90% by volume, more preferably 40 to 85% by volume, still more preferably 50 to 80% by volume, relative to the solvent.

The solvent preferably contains at least one selected from the group consisting of the cyclic carbonate, the acyclic carbonate, and the acyclic carboxylate, and more preferably contains the cyclic carbonate and at least one selected from the group consisting of the acyclic carbonate and the acyclic carboxylate. The cyclic carbonate is preferably a saturated cyclic carbonate.

An electrolyte solution containing a solvent of such a composition allows an electrochemical device to have further improved high-temperature storage characteristics and cycle characteristics.

In the solvent containing the cyclic carbonate and at least one selected from the group consisting of the acyclic carbonate and the acyclic carboxylate, the total amount of the cyclic carbonate and at least one selected from the group consisting of the acyclic carbonate and the acyclic carboxylate is preferably 10 to 90% by volume, more preferably 30 to 80% by volume, still more preferably 50 to 70% by volume.

The solvent containing the cyclic carbonate and at least one selected from the group consisting of the acyclic carbonate and the acyclic carboxylate has a volume ratio of the cyclic carbonate and at least one selected from the group consisting of the acyclic carbonate and the acyclic carboxylate of preferably 5/95 to 95/5, more preferably 10/90 or more, still more preferably 15/85 or more, particularly preferably 20/80 or more, while more preferably 90/10 or less, still more preferably 60/40 or less, particularly preferably 50/50 or less.

The solvent also preferably contains at least one selected from the group consisting of the non-fluorinated saturated cyclic carbonate, the non-fluorinated acyclic carbonate, and the non-fluorinated acyclic carboxylate, more preferably contains the non-fluorinated saturated cyclic carbonate and at least one selected from the group consisting of the non-fluorinated acyclic carbonate and the non-fluorinated acyclic carboxylate. An electrolyte solution containing a solvent of such a composition can suitably be used for electrochemical devices used at relatively low voltage.

In the solvent containing the non-fluorinated saturated cyclic carbonate and at least one selected from the group consisting of the non-fluorinated acyclic carbonate and the non-fluorinated acyclic carboxylate, the total amount of the non-fluorinated saturated cyclic carbonate and at least one selected from the group consisting of the non-fluorinated acyclic carbonate and the non-fluorinated acyclic carboxylate is preferably 5 to 90% by volume, more preferably 20 to 80% by volume, still more preferably 30 to 70% by volume.

The electrolyte solution containing the non-fluorinated saturated cyclic carbonate and at least one selected from the group consisting of the non-fluorinated acyclic carbonate and the non-fluorinated acyclic carboxylate has a volume ratio of the non-fluorinated saturated cyclic carbonate and at least one selected from the group consisting of the non-fluorinated acyclic carbonate and the non-fluorinated acyclic carboxylate of preferably 5/95 to 95/5, more preferably 10/90 or more, still more preferably 15/85 or more, particularly preferably 20/80 or more, while more preferably 90/10 or less, still more preferably 60/40 or less, particularly preferably 50/50 or less.

The solvent preferably contains at least one selected from the group consisting of the fluorinated saturated cyclic carbonate, the fluorinated acyclic carbonate, and the fluorinated acyclic carboxylate, and more preferably contains the fluorinated saturated cyclic carbonate and at least one selected from the group consisting of the fluorinated acyclic carbonate and the fluorinated acyclic carboxylate. An electrolyte solution containing a solvent of such a composition can suitably be used for not only electrochemical devices used at relatively high voltage, but also electrochemical devices used at relatively low voltage.

In the solvent containing the fluorinated saturated cyclic carbonate and at least one selected from the group consisting of the fluorinated acyclic carbonate and the fluorinated acyclic carboxylate, the total amount of the fluorinated saturated cyclic carbonate and at least one selected from the group consisting of the fluorinated acyclic carbonate and the fluorinated acyclic carboxylate is preferably 5 to 100% by volume, more preferably 10 to 100% by volume, still more preferably 30 to 100% by volume.

The solvent containing the fluorinated saturated cyclic carbonate and at least one selected from the group consisting of the fluorinated acyclic carbonate and the fluorinated acyclic carboxylate has a volume ratio of the fluorinated saturated cyclic carbonate and at least one selected from the group consisting of the fluorinated acyclic carbonate and the fluorinated acyclic carboxylate of preferably 5/95 to 95/5, more preferably 10/90 or more, still more preferably 15/85 or more, particularly preferably 20/80 or more, while more preferably 90/10 or less, still more preferably 60/40 or less, particularly preferably 50/50 or less.

The solvent used may be an ionic liquid other than the compound (1). The "ionic liquid" means a liquid containing an ion that is a combination of an organic cation and an anion.

Examples of the organic cation include, but are not limited to, imidazolium ions such as dialkyl imidazolium cations and trialkyl imidazolium cations; tetraalkyl ammonium ions; alkyl pyridinium ions; dialkyl pyrrolidinium ions; and dialkyl piperidinium ions.

Examples of the anion to be used as a counterion of any of these organic cations include, but are not limited to, a PF₆ anion, a PF₃(C₂F₅)₃ anion, a PF₃(CF₃)₃ anion, a BF₄ anion, a BF₂(CF₃)₂ anion, a BF₃(CF₃) anion, a bis(oxalato)boric acid anion, a P(C₂O₄)F₂ anion, a Tf (trifluoromethanesulfonyl) anion, a Nf (nonafluorobutanesulfonyl) anion, a bis(fluorosulfonyl)imide anion, a bis(trifluoromethanesulfonyl)imide anion, a bis(pentafluoroethanesulfonyl)imide anion, a dicyanoamine anion, and halide anions.

The solvent is preferably a non-aqueous solvent, and the composition of the disclosure is preferably a non-aqueous electrolyte solution.

The solvent is preferably present in an amount of 70 to 99.999% by mass, more preferably 80% by mass or more, while more preferably 92% by mass or less, of the electrolyte solution.

Examples of the electrolyte salt used for the electrolyte solution include lithium salts, ammonium salts, and metal salts, as well as any of those to be used for electrolyte solutions such as liquid salts (ionic liquids), inorganic polymer salts, and organic polymer salts (excluding a compound (1) described later).

The electrolyte salt of the electrolyte solution for a lithium-ion secondary battery is preferably a lithium salt.

Any lithium salt may be used. Specific examples thereof include the following: inorganic lithium salts such as LiPF₆, LiBF₄, LiClO₄, LiAlF₄, LiSbF₆, LiTaF₆, LiWF₇, LiAsF₆, LiAlCl₄, LiI, LiBr, LiCl, LiB₁₀Cl₁₀, Li₂SiF₆, Li₂PFO₃, and LiPO₂F₂;
lithium tungstate salts such as LiWOF₅;
lithium carboxylates such as HCO₂Li, CH₃CO₂Li, CH₂FCO₂Li, CHF₂CO₂Li, CF₃CO₂Li, CF₃CH₂CO₂Li, CF₃CF₂CO₂Li, CF₃CF₂CF₂CO₂Li, and CF₃CF₂CF₂CF₂CO₂Li;
lithium salts containing an S=O group such as FSO₃Li, CH₃SO₃Li, CH₂FSO₃Li, CHF₂SO₃Li, CF₃SO₃Li, CF₃CF₂SO₃Li, CF₃CF₂CF₂SO₃Li, CF₃CF₂CF₂CF₂SO₃Li, lithium methylsulfate, lithium ethylsulfate (C₂H₅OSO₃Li), and lithium 2,2,2-trifluoroethylsulfate;
lithium imide salts such as LiN(FCO)₂, LiN(FCO)(FSO₂), LiN(FSO₂)₂, LiN(FSO₂)(CF₃SO₂), LiN(CF₃SO₂)₂, LiN(C₂F₅SO₂)₂, lithium bis-perfluoroethanesulfonyl imide, lithium cyclic 1,2-perfluoroethanedisulfonyl imide, lithium cyclic 1,3-perfluoropropanedisulfonyl imide, lithium cyclic 1,2-ethanedisulfonyl imide, lithium cyclic 1,3-propanedisulfonyl imide, lithium cyclic 1,4-perfluorobutanedisulfonyl imide, LiN(CF₃SO₂)(FSO₂), LiN(CF₃SO₂)(C₃F₇SO₂), LiN(CF₃SO₂)(C₄F₉SO₂), and LiN(POF₂)₂;
lithium methide salts such as LiC(FSO₂)₃, LiC(CF₃SO₂)₃, and LiC(C₂F₅SO₂)₃; and
fluorine-containing organic lithium salts such as salts represented by the formula: LiPFₐ(CₙF₂ₙ₊₁)₆₋ₐ (wherein a is an integer of 0 to 5; and n is an integer of 1 to 6) such as LiPF₃(C₂F₅)₃, LiPF₃(CF₃)₃, LiPF₃(iso-C₃F₇)₃, LiPF₅(iso-C₃F₇), LiPF₄(CF₃)₂, and LiPF₄(C₂F₅)₂, as well as LiPF₄(CF₃SO₂)₂, LiPF₄(C₂F₅SO₂)₂, LiBF₃CF₃, LiBF₃C₂F₅, LiBF₃C₃F₇, LiBF₂(CF₃)₂, LiBF₂(C₂F₅)₂, LiBF₂(CF₃SO₂)₂, and LiBF₂(C₂F₅SO₂)₂, and LiSCN, LiB(CN)₄, LiB(C₆H₅)₄, Li₂(C₂O₄), LiP(C₂O₄)₃, and Li₂B₁₂F_{b}H_{12-b} (wherein b is an integer of 0 to 3).

In order to achieve an effect of improving properties such as output characteristics, high-rate charge and discharge characteristics, high-temperature storage characteristics, and cycle characteristics, particularly preferred among these are LiPF₆, LiBF₄, LiSbF₆, LiTaF₆, LiPO₂F₂, FSO₃Li, CF₃SO₃Li, LiN(FSO₂)₂, LiN(FSO₂)(CF₃SO₂), LiN(CF₃SO₂)₂, LiN(C₂F₅SO₂)₂, lithium cyclic 1,2-perfluoroethanedisulfonyl imide, lithium cyclic 1,3-perfluoropropanedisulfonyl imide, LiC(FSO₂)₃, LiC(CF₃SO₂)₃, LiC(C₂F₅SO₂)₃, LiBF₃CF₃, LiBF₃C₂F₅, LiPF₃(CF₃)₃, LiPF₃(C₂F₅)₃, and the like. Most preferred is at least one lithium salt selected from the group consisting of LiPF₆, LiN(FSO₂)₂, and LiBF₄.

One of these electrolyte salts may be used alone or two or more thereof may be used in any combination. In combination use of two or more thereof, preferred examples thereof include a combination of LiPF₆ and LiBF₄ and a combination of LiPF₆ and LiPO₂F₂, C₂H₅OSO₃Li, or FSO₃Li, each of which have an effect of improving the high-temperature storage characteristics, the load characteristics, and the cycle characteristics.

In this case, LiBF₄, LiPO₂F₂, C₂H₅OSO₃Li, or FSO₃Li may be present in any amount that does not significantly impair the effects of the disclosure in 100% by mass of the whole electrolyte solution. The amount thereof is usually 0.01% by mass or more, preferably 0.1% by mass or more, while the amount thereof is usually 30% by mass or less, preferably 20% by mass or less, more preferably 10% by mass or less, still more preferably 5% by mass or less, relative to the electrolyte solution.

In another example, an inorganic lithium salt and an organic lithium salt are used in combination. Such a combination has an effect of reducing deterioration due to high-temperature storage. The organic lithium salt is preferably CF₃SO₃Li, LiN(FSO₂)₂, LiN(FSO₂)(CF₃SO₂), LiN(CF₃SO₂)₂, LiN(C₂F₅SO₂)₂, lithium cyclic 1,2-perfluoroethanedisulfonyl imide, lithium cyclic 1,3-perfluoropropanedisulfonyl imide, LiC(FSO₂)₃, LiC(CF₃SO₂)₃, LiC(C₂F₅SO₂)₃, LiBF₃CF₃, LiBF₃C₂F₅, LiPF₃(CF₃)₃, LiPF₃(C₂F₅)₃, or the like. In this case, the proportion of the organic lithium salt is preferably 0.1% by mass or more, particularly preferably 0.5% by mass or more, while preferably 30% by mass or less, particularly preferably 20% by mass or less, of 100% by mass of the whole electrolyte solution.

The electrolyte salt in the electrolyte solution may have any concentration that does not impair the effects of the disclosure. In order to make the electric conductivity of the electrolyte solution within a favorable range and to ensure good battery performance, the electrolyte solution has a total mole concentration of lithium of preferably 0.3 mol/L or higher, more preferably 0.4 mol/L or higher, still more preferably 0.5 mol/L or higher, while preferably 3.0 mol/L or lower, more preferably 2.5 mol/L or lower, still more preferably 2.0 mol/L or lower.

Too low a total mole concentration of lithium may cause insufficient electric conductivity of the electrolyte solution, while too high a concentration may cause an increase in viscosity and then reduction in electric conductivity, impairing the battery performance.

The electrolyte solution may further contain a compound (1) represented by the following formula (1).

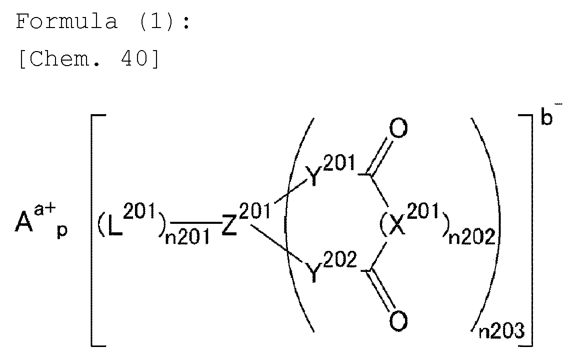

(In the formula, A^{a+} is a metal ion, a hydrogen ion, or an onium ion; a is an integer of 1 to 3; b is an integer of 1 to 3; p is b/a; n203 is an integer of 1 to 4; n201 is an integer of 0 to 8; n202 is 0 or 1; and Z²⁰¹ is a transition metal or an element of group III, IV, or V of the periodic table.

X²⁰¹ is O, S, a C1-C10 alkylene group, a C1-C10 halogenated alkylene group, a C6-C20 arylene group, or a C6-C20 halogenated arylene group (the alkylene, halogenated alkylene, arylene, and halogenated arylene groups may have a substituent or a hetero atom in the structure, and when n202 is 1 and n203 is 2 to 4, n203 X²⁰¹s may be bonded to each other).

L²⁰¹ is a halogen atom, a cyano group, a C1-C10 alkyl group, a C1-C10 halogenated alkyl group, a C6-C20 aryl group, a C6-C20 halogenated aryl group (the alkylene, halogenated alkylene, arylene, and halogenated arylene groups may have a substituent or a hetero atom in the structure, and when n201 is 2 to 8, n201 L²⁰¹s may be bonded to form a ring), or -Z²⁰³Y²⁰³.

Y²⁰¹, Y²⁰², and Z²⁰³ are each independently O, S, NY²⁰⁴, a hydrocarbon group, or a fluorinated hydrocarbon group. Y²⁰³ and Y²⁰⁴ are each independently H, F, a C1-C10 alkyl group, a C1-C10 halogenated alkyl group, a C6-C20 aryl group, or a C6-C20 halogenated aryl group (the alkyl, halogenated alkyl, aryl, and halogenated aryl groups may have a substituent or a hetero atom in the structure, and when multiple Y²⁰³s or Y²⁰⁴s are present, they may be bonded to form a ring).

Examples of A^{a+} include a lithium ion, a sodium ion, a potassium ion, a magnesium ion, a calcium ion, a barium ion, a cesium ion, a silver ion, a zinc ion, a copper ion, a cobalt ion, an iron ion, a nickel ion, a manganese ion, a titanium ion, a lead ion, a chromium ion, a vanadium ion, a ruthenium ion, a yttrium ion, a lanthanoid ion, an actinoid ion, a tetrabutylammonium ion, a tetraethylammonium ion, a tetramethylammonium ion, a triethylmethylammonium ion, a triethylammonium ion, a pyridinium ion, an imidazolium ion, a hydrogen ion, a tetraethylphosphonium ion, a tetramethylphosphonium ion, a tetraphenylphosphonium ion, a triphenylsulfonium ion, and a triethylsulfonium ion.

For applications such as electrochemical devices, A^{a+} is preferably a lithium ion, a sodium ion, a magnesium ion, a tetraalkylammonium ion, or a hydrogen ion, particularly preferably a lithium ion. The valence a of an A^{a+} cation is an integer of 1 to 3. When the valence a is larger than 3, the crystal lattice energy becomes larger, which problematically makes it difficult to dissolve the electrolyte solution of the disclosure in a solvent. The valence is therefore more preferably 1 when the solubility is required. The valence b of an anion is similarly an integer of 1 to 3, particularly preferably 1. The constant p which represents a ratio between the cation and the anion is necessarily determined by the ratio b/a of their valence values.

Next, a ligand portion of the formula (1) is described. Herein, the organic or inorganic moiety bonded to Z²⁰¹ in the formula (1) is referred to as a ligand.

Z²⁰¹ is preferably Al, B, V, Ti, Si, Zr, Ge, Sn, Cu, Y, Zn, Ga, Nb, Ta, Bi, P, As, Sc, Hf, or Sb, more preferably Al, B, or P.

X²⁰¹ represents O, S, a C1-C10 alkylene group, a C1-C10 halogenated alkylene group, a C6-C20 arylene group, or a C6-C20 halogenated arylene group. These alkylene and arylene groups may have a substituent or a hetero atom in the structure. Specifically, they may have a halogen atom or a linear or cyclic alkyl group, an aryl group, an alkenyl group, an alkoxy group, an aryloxy group, a sulfonyl group, an amino group, a cyano group, a carbonyl group, an acyl group, an amide group, or a hydroxy group as a substituent in place of hydrogen on the alkylene or arylene group. They also may have a structure where nitrogen, sulfur, or oxygen is introduced in place of carbon on the alkylene or arylene group. When n202 is 1 and n203 is 2 to 4, n203 X²⁰¹s may be bonded to each other. Examples of such a structure include ligands such as ethylenediaminetetraacetic acid.

L²⁰¹ represents a halogen atom, a cyano group, a C1-C10 alkyl group, a C1-C10 halogenated alkyl group, a C6-C20 aryl group, a C6-C20 halogenated aryl group, or -Z²⁰³Y²⁰³ (Z²⁰³ and Y²⁰³ are described later). As in the case of X²⁰¹, the alkyl and aryl groups here may have a substituent or a hetero atom in the structure, and when n201 is 2 to 8, n201 L²⁰¹s may be bonded to form a ring. L²⁰¹ is preferably a fluorine atom or a cyano group. When L²⁰¹ is a fluorine atom, solubility and dissociation of anionic compound salts are increased, which in turn favorably increases ionic conductivity. In addition, oxidation resistance is improved to reduce or inhibit occurrence of side reactions.

Y²⁰¹, Y²⁰², and Z²⁰³ each independently represent O, S, NY²⁰⁴, a hydrocarbon group, or a fluorinated hydrocarbon group. Y²⁰¹ and Y²⁰² each are preferably O, S, or NY²⁰⁴, more preferably O. The compound (1) is characterized in that a bond of Y²⁰¹ with Z²⁰¹ and a bond of Y²⁰² with Z²⁰¹ are present within the same ligand and such ligands form a chelate structure with Z²⁰¹. Owing to this chelate, the compound has higher heat resistance, higher chemical stability, and higher hydrolysis resistance. The constant n202 in this ligand is 0 or 1. When the constant n202 is 0, in particular, the chelate ring is a 5-membered ring, which favorably allows strongest exertion of the chelate effect to increase the stability.

The fluorinated hydrocarbon group herein is a group obtainable by replacing at least one hydrogen atom of a hydrocarbon group with a fluorine atom.

Y²⁰³ and Y²⁰⁴ are each independently H, F, a C1-C10 alkyl group, a C1-C10 halogenated alkyl group, a C6-C20 aryl group, or a C6-C20 halogenated aryl group. These alkyl and aryl groups may have a substituent or a hetero atom in the structure. When multiple Y²⁰³s or Y²⁰⁴s are present, they may be bonded to form a ring.

The constant n203 related to the number of ligands described above is an integer of 1 to 4, preferably 1 or 2, more preferably 2. The constant n201 related to the number of ligands described above is an integer of 0 to 8, preferably an integer of 0 to 4, more preferably 0, 2, or 4. Preferably, n201 is 2 when n203 is 1 and n201 is 0 when n203 is 2.

In the formula (1), the alkyl, halogenated alkyl, aryl, and halogenated aryl groups may be those with a different functional group such as a branch, a hydroxy group, or an ether bond.

The compound (1) is preferably a compound represented by the following formula: 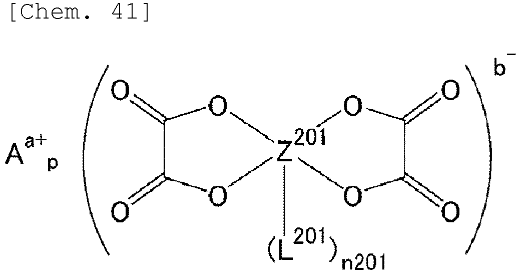 (wherein A^{a+}, a, b, p, n201, Z²⁰¹, and L²⁰¹ are as described above), or a compound represented by the following formula: 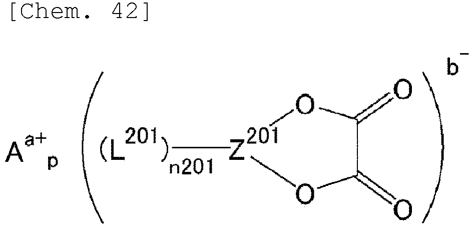 (wherein A^{a+}, a, b, p, n201, Z²⁰¹, and L²⁰¹ are as described above) .

Examples of the compound (1) include lithium (oxalate)borate salts including lithium bis(oxalate)borate (LIBOB) represented by the following formula: 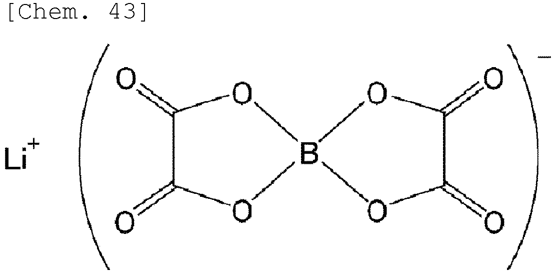 ; lithium difluorooxalatoborate (LIDFOB) represented by the following formula: 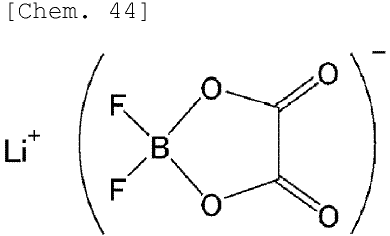 ; lithium difluorooxaratophosphanite (LIDFOP) represented by the following formula: 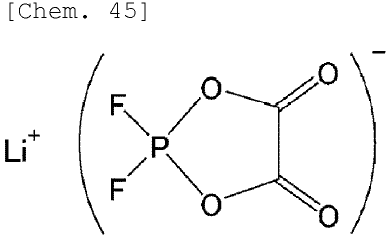 ; lithium tetrafluorooxalatophosphanite (LITFOP) represented by the following formula: 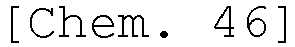 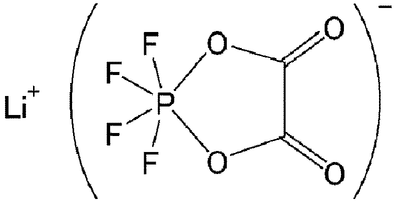 ; and lithium bis(oxalato)difluorophosphanite represented by the following formula: 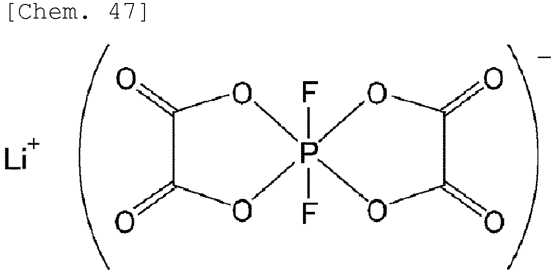 .

Examples of the compound (1) also include dicarboxylic acid complex salts in which the complex center element is boron, such as lithium bis(malonato)borate, lithium difluoro(malonato)borate, lithium bis(methylmalonato)borate, lithium difluoro(methylmalonato)borate, lithium bis(dimethylmalonato)borate, and lithium difluoro(dimethylmalonato)borate.

Examples of the compound (1) also include dicarboxylic acid complex salts in which the complex center element is phosphorus, such as lithium tris(oxalato)phosphate, lithium tris(malonato)phosphate, lithium difluorobis(malonato)phosphate, lithium tetrafluoro(malonato)phosphate, lithium tris(methylmalonato)phosphate, lithium difluorobis(methylmalonato)phosphate, lithium tetrafluoro(methylmalonato)phosphate, lithium tris(dimethylmalonato)phosphate, lithium difluorobis(dimethylmalonato)phosphate, and lithium tetrafluoro(dimethylmalonato)phosphate.

Examples of the compound (1) also include dicarboxylic acid complex salts in which the complex center element is aluminum, such as LiAl(C₂O₄)₂ and LiAlF₂(C₂O₄).

In order to enable easy availability and contribute to formation of a stable film-shaped structure, more preferred among these are lithium bis(oxalato)borate, lithium difluoro(oxalato)borate, lithium tris(oxalato)phosphate, lithium difluorobis(oxalato)phosphate, and lithium tetrafluoro(oxalato)phosphate.

The compound (1) is particularly preferably lithium bis(oxalato)borate.

In order to give much better cycle characteristics, the compound (1) is preferably in an amount of 0.001% by mass or more, more preferably 0.01% by mass or more, while preferably 10% by mass or less, more preferably 3% by mass or less, relative to the solvent.

The electrolyte solution preferably further contains a compound (2) represented by the following formula (2): 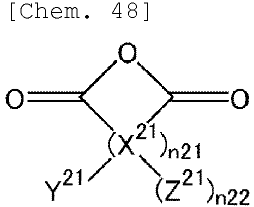 (wherein X²¹ is a group containing at least H or C; n21 is an integer of 1 to 3; Y²¹ and Z²¹ are the same as or different from each other, and are each a group containing at least H, C, O, or F; n22 is 0 or 1; and Y²¹ and Z²¹ optionally bind to each other to form a ring). The electrolyte solution containing the compound (2) can cause much less reduction in capacity retention and can cause a much less increase in amount of gas generated even when stored at high temperature.

When n21 is 2 or 3, the two or three X²¹s may be the same as or different from each other.

When multiple Y²¹s and multiple Z²¹s are present, the multiple Y²¹s may be the same as or different from each other and the multiple Z²¹s may be the same as or different from each other.

X²¹ is preferably a group represented by -CY²¹Z²¹-(wherein Y²¹ and Z²¹ are defined as described above) or a group represented by -CY²¹=CZ²¹- (wherein Y²¹ and Z²¹ are defined as described above).

Y²¹ preferably includes at least one selected from the group consisting of H-, F-, CH₃-, CH₃CH₂-, CH₃CH₂CH₂-, CF₃-, CF₃CF₂-, CH₂FCH₂-, and CF₃CF₂CF₂-.

Z²¹ preferably includes at least one selected from the group consisting of H-, F-, CH₃-, CH₃CH₂-, CH₃CH₂CH₂-, CF₃-, CF₃CF₂-, CH₂FCH₂-, and CF₃CF₂CF₂-.

Alternatively, Y²¹ and Z²¹ may bind to each other to form a carbon ring or a heterocyclic ring that may contain an unsaturated bond and may have aromaticity. The ring preferably has a carbon number of 3 to 20.

Next, specific examples of the compound (2) are described. In the following examples, the term "analog" means an acid anhydride obtainable by replacing part of the structure of an acid anhydride mentioned as an example with another structure within the scope of the disclosure. Examples thereof include dimers, trimers, and tetramers each composed of a plurality of acid anhydrides, those having respective substituents which are structural isomers having the same carbon number but having different branch structures, and those having the same substituent but at different sites in an acid anhydride.

Specific examples of an acid anhydride having a 5-membered cyclic structure include succinic anhydride, methylsuccinic anhydride (4-methylsuccinic anhydride), dimethylsuccinic anhydride (e.g., 4,4-dimethylsuccinic anhydride, 4,5-dimethylsuccinic anhydride), 4,4,5-trimethylsuccinic anhydride, 4,4,5,5-tetramethylsuccinic anhydride, 4-vinylsuccinic anhydride, 4,5-divinylsuccinic anhydride, phenylsuccinic anhydride (4-phenylsuccinic anhydride), 4,5-diphenylsuccinic anhydride, 4,4-diphenylsuccinic anhydride, citraconic anhydride, maleic anhydride, methylmaleic anhydride (4-methylmaleic anhydride), 4,5-dimethylmaleic anhydride, phenylmaleic anhydride (4-phenylmaleic anhydride), 4,5-diphenylmaleic anhydride, itaconic anhydride, 5-methylitaconic anhydride, 5,5-dimethylitaconic anhydride, phthalic anhydride, and 3,4,5,6-tetrahydrophthalic anhydride, and analogs thereof.

Specific examples of an acid anhydride having a 6-membered cyclic structure include cyclohexanedicarboxylic anhydride (e.g., cyclohexane-1,2-dicarboxylic anhydride), 4-cyclohexene-1,2-dicarboxylic anhydride, glutaric anhydride, glutaconic anhydride, and 2-phenylglutaric anhydride, and analogs thereof.

Specific examples of an acid anhydride having a different cyclic structure include 5-norbornene-2,3-dicarboxylic anhydride, cyclopentanetetracarboxylic dianhydride, pyromellitic anhydride, and diglycolic anhydride, and analogs thereof.

Specific examples of an acid anhydride having a cyclic structure and substituted with a halogen atom include monofluorosuccinic anhydride (e.g., 4-fluorosuccinic anhydride), 4,4-difluorosuccinic anhydride, 4,5-difluorosuccinic anhydride, 4,4,5-trifluorosuccinic anhydride, trifluoromethylsuccinic anhydride, tetrafluorosuccinic anhydride (4,4,5,5-tetrafluorosuccinic anhydride), 4-fluoromaleic anhydride, 4,5-difluoromaleic anhydride, trifluoromethylmaleic anhydride, 5-fluoroitaconic anhydride, and 5,5-difluoroitaconic anhydride, and analogs thereof.

Preferred among these as the compound (2) are glutaric anhydride, citraconic anhydride, glutaconic anhydride, itaconic anhydride, diglycolic anhydride, cyclohexanedicarboxylic anhydride, cyclopentanetetracarboxylic dianhydride, 4-cyclohexene-1,2-dicarboxylic anhydride, 3,4,5,6-tetrahydrophthalic anhydride, 5-norbornene-2,3-dicarboxylic anhydride, phenylsuccinic anhydride, 2-phenylglutaric anhydride, maleic anhydride, methylmaleic anhydride, trifluoromethylmaleic anhydride, phenylmaleic anhydride, succinic anhydride, methylsuccinic anhydride, dimethylsuccinic anhydride, trifluoromethylsuccinic anhydride, monofluorosuccinic anhydride, and tetrafluorosuccinic anhydride. More preferred are maleic anhydride, methylmaleic anhydride, trifluoromethylmaleic anhydride, succinic anhydride, methylsuccinic anhydride, trifluoromethylsuccinic anhydride, and tetrafluorosuccinic anhydride, and still more preferred are maleic anhydride and succinic anhydride.

The compound (2) preferably includes at least one selected from the group consisting of: a compound (3) represented by the following formula (3): 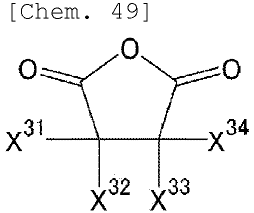 (wherein X³¹ to X³⁴ are the same as or different from each other, and are each a group containing at least H, C, O, or F); and a compound (4) represented by the following formula (4): 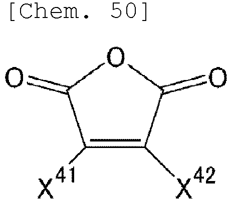 (wherein X⁴¹ and X⁴² are the same as or different from each other, and are each a group containing at least H, C, O, or F).

X³¹ to X³⁴ are the same as or different from each other, and preferably include at least one selected from the group consisting of an alkyl group, a fluorinated alkyl group, an alkenyl group, and a fluorinated alkenyl group. X³¹ to X³⁴ each preferably have a carbon number of 1 to 10, more preferably 1 to 3.

X³¹ to X³⁴ are the same as or different from each other, and more preferably include at least one selected from the group consisting of H-, F-, CH₃-, CH₃CH₂-, CH₃CH₂CH₂-, CF₃-, CF₃CF₂-, CH₂FCH₂-, and CF₃CF₂CF₂-.

X⁴¹ and X⁴² are the same as or different from each other, and preferably include at least one selected from the group consisting of an alkyl group, a fluorinated alkyl group, an alkenyl group, and a fluorinated alkenyl group. X⁴¹ and X⁴² each preferably have a carbon number of 1 to 10, more preferably 1 to 3.

X⁴¹ and X⁴² are the same as or different from each other, and more preferably include at least one selected from the group consisting of H-, F-, CH₃-, CH₃CH₂-, CH₃CH₂CH₂-, CF₃-, CF₃CF₂-, CH₂FCH₂-, and CF₃CF₂CF₂-.

The compound (3) is preferably any of the following compounds.

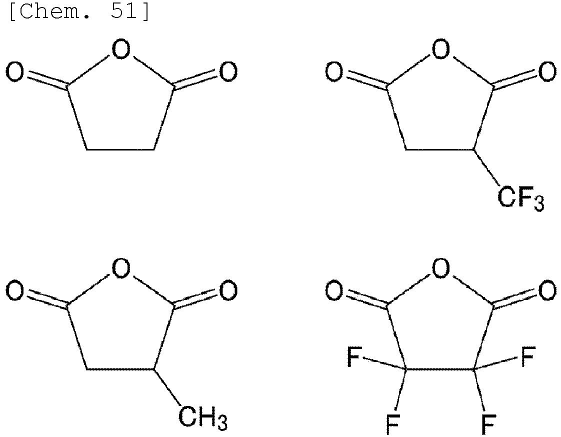

The compound (4) is preferably any of the following compounds.

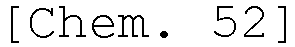

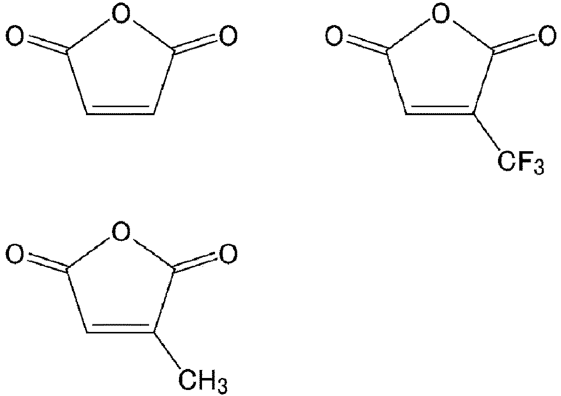

In order to cause much less reduction in capacity retention and a much less increase in amount of gas generated even when stored at high temperature, the electrolyte solution preferably contains 0.0001 to 15% by mass of the compound (4) relative to the electrolyte solution. The amount of the compound (4) is more preferably 0.01 to 10% by mass, still more preferably 0.1 to 3% by mass, particularly preferably 0.1 to 1.0% by mass.

In order to cause much less reduction in capacity retention and a much less increase in amount of gas generated even when stored at high temperature, the electrolyte solution, when containing both the compounds (3) and (4), preferably contains 0.08 to 2.50% by mass of the compound (3) and 0.02 to 1.50% by mass of the compound (4), more preferably 0.80 to 2.50% by mass of the compound (3) and 0.08 to 1.50% by mass of the compound (4), relative to the electrolyte solution.

The electrolyte solution may contain at least one selected from the group consisting of nitrile compounds represented by the following formulas (1a), (1b), and (1c): 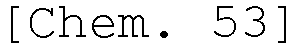 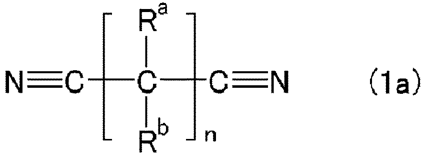 (wherein R^{a} and R^{b} are each independently a hydrogen atom, a cyano group (CN), a halogen atom, an alkyl group, or a group obtainable by replacing at least one hydrogen atom of an alkyl group with a halogen atom; and n is an integer of 1 to 10); 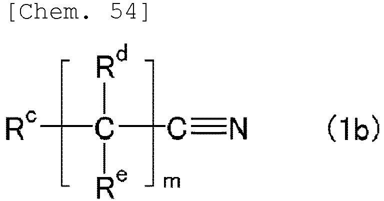 (wherein R^{c} is a hydrogen atom, a halogen atom, an alkyl group, a group obtainable by replacing at least one hydrogen atom of an alkyl group with a halogen atom, or a group represented by NC-R^{c1}-X^{c1}- (wherein R^{c1} is an alkylene group, x^{c1} is an oxygen atom or a sulfur atom); R^{d} and R^{e} are each independently a hydrogen atom, a halogen atom, an alkyl group, or a group obtainable by replacing at least one hydrogen atom of an alkyl group with a halogen atom; and m is an integer of 1 to 10); 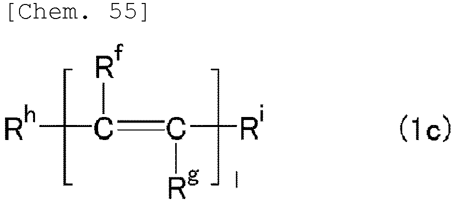 (wherein R^{f}, R^{g}, R^{h}, and Rⁱ are each independently a group containing a cyano group (CN), a hydrogen atom (H), a halogen atom, an alkyl group, or a group obtainable by replacing at least one hydrogen atom of an alkyl group with a halogen atom; at least one selected from the group consisting of R^{f}, R^{g}, R^{h}, and Rⁱ is a group containing a cyano group; and l is an integer of 1 to 3).

This can improve the high-temperature storage characteristics of an electrochemical device. One nitrile compound may be used alone, or two or more thereof may be used in any combination at any ratio.

In the formula (1a), R^{a} and R^{b} are each independently a hydrogen atom, a cyano group (CN), a halogen atom, an alkyl group, or a group obtainable by replacing at least one hydrogen atom of an alkyl group with a halogen atom.

Examples of the halogen atom include a fluorine atom, a chlorine atom, a bromine atom, and an iodine atom. Preferred among these is a fluorine atom.

The alkyl group is preferably a C1-C5 alkyl group. Specific examples of the alkyl group include a methyl group, an ethyl group, a propyl group, an isopropyl group, a butyl group, an isobutyl group, and a tert-butyl group.

An example of the group obtainable by replacing at least one hydrogen atom of an alkyl group with a halogen atom is a group obtainable by replacing at least one hydrogen atom of the aforementioned alkyl group with the aforementioned halogen atom.

When R^{a} and R^{b} are alkyl groups or groups each obtainable by replacing at least one hydrogen atom of an alkyl group with a halogen atom, R^{a} and R^{b} may bind to each other to form a cyclic structure (e.g., a cyclohexane ring).

R^{a} and R^{b} are each preferably a hydrogen atom or an alkyl group.

In the formula (1a), n is an integer of 1 to 10. When n is 2 or greater, all of n R^{a}s may be the same as each other, or at least part of them may be different from the others. The same applies to R^{b}. In the formula, n is preferably an integer of 1 to 7, more preferably an integer of 2 to 5.

Preferred as the nitrile compound represented by the formula (1a) are dinitriles and tricarbonitriles.

Specific examples of the dinitriles include malononitrile, succinonitrile, glutaronitrile, adiponitrile, pimelonitrile, suberonitrile, azelanitrile, sebaconitrile, undecanedinitrile, dodecanedinitrile, methylmalononitrile, ethylmalononitrile, isopropylmalononitrile, tert-butylmalononitrile, methylsuccinonitrile, 2,2-dimethylsuccinonitrile, 2,3-dimethylsuccinonitrile, 2,3,3-trimethylsuccinonitrile, 2,2,3,3-tetramethylsuccinonitrile, 2,3-diethyl-2,3-dimethylsuccinonitrile, 2,2-diethyl-3,3-dimethylsuccinonitrile, bicyclohexyl-1,1-dicarbonitrile, bicyclohexyl-2,2-dicarbonitrile, bicyclohexyl-3,3-dicarbonitrile, 2,5-dimethyl-2,5-hexanedicarbonitrile, 2,3-diisobutyl-2,3-dimethylsuccinonitrile, 2,2-diisobutyl-3,3-dimethylsuccinonitrile, 2-methylglutaronitrile, 2,3-dimethylglutaronitrile, 2,4-dimethylglutaronitrile, 2,2,3,3-tetramethylglutaronitrile, 2,2,4,4-tetramethylglutaronitrile, 2,2,3,4-tetramethylglutaronitrile, 2,3,3,4-tetramethylglutaronitrile, 1,4-dicyanopentane, 2,6-dicyanoheptane, 2,7-dicyanooctane, 2,8-dicyanononane, 1,6-dicyanodecane, 1,2-dicyanobenzene, 1,3-dicyanobenzene, 1,4-dicyanobenzene, 3,3'-(ethylenedioxy)dipropionitrile, 3,3'-(ethylenedithio)dipropionitrile, 3,9-bis(2-cyanoethyl)-2,4,8,10-tetraoxaspiro[5,5]undecane, butanenitrile, and phthalonitrile. Particularly preferred among these are succinonitrile, glutaronitrile, and adiponitrile.

Specific examples of the tricarbonitriles include pentanetricarbonitrile, propanetricarbonitrile, 1,3,5-hexanetricarbonitrile, 1,3,6-hexanetricarbonitrile, heptanetricarbonitrile, 1,2,3-propanetricarbonitrile, 1,3,5-pentanetricarbonitrile, cyclohexanetricarbonitrile, triscyanoethylamine, triscyanoethoxypropane, tricyanoethylene, and tris(2-cyanoethyl)amine. Particularly preferred are 1,3,6-hexanetricarbonitrile and cyclohexanetricarbonitrile, and most preferred is cyclohexanetricarbonitrile.

In the formula (1b), R^{c} is a hydrogen atom, a halogen atom, an alkyl group, a group obtainable by replacing at least one hydrogen atom of an alkyl group with a halogen atom, or a group represented by NC-R^{c1}-X^{c1}- (wherein R^{c1} is an alkylene group; and x^{c1} is an oxygen atom or a sulfur atom); R^{d} and R^{e} are each independently a hydrogen atom, a halogen atom, an alkyl group, or a group obtainable by replacing at least one hydrogen atom of an alkyl group with a halogen atom.

Examples of the halogen atom, the alkyl group, and the group obtainable by replacing at least one hydrogen atom of an alkyl group with a halogen atom include those mentioned as examples thereof for the formula (1a).

R^{c1} in NC-R^{c1}-X^{c1}- is an alkylene group. The alkylene group is preferably a C1-C3 alkylene group.

R^{c}, R^{d}, and R^{e} are each preferably independently a hydrogen atom, a halogen atom, an alkyl group, or a group obtainable by replacing at least one hydrogen atom of an alkyl group with a halogen atom.

At least one selected from R^{c}, R^{d}, and R^{e} is preferably a halogen atom or a group obtainable by replacing at least one hydrogen atom of an alkyl group with a halogen atom, more preferably a fluorine atom or a group obtainable by replacing at least one hydrogen atom of an alkyl group with a fluorine atom.

When R^{d} and R^{e} are each an alkyl group or a group obtainable by replacing at least one hydrogen atom of an alkyl group with a halogen atom, R^{d} and R^{e} may bind to each other to form a cyclic structure (e.g., a cyclohexane ring).

In the formula (1b), m is an integer of 1 to 10. When m is 2 or greater, all of m R^{d}s may be the same as each other, or at least part of them may be different from the others. The same applies to R^{e}. In the formula, m is preferably an integer of 2 to 7, more preferably an integer of 2 to 5.

Examples of the nitrile compound represented by the formula (1b) include acetonitrile, propionitrile, butyronitrile, isobutyronitrile, valeronitrile, isovaleronitrile, lauronitrile, 3-methoxypropionitrile, 2-methylbutyronitrile, trimethylacetonitrile, hexanenitrile, cyclopentanecarbonitrile, cyclohexanecarbonitrile, fluoroacetonitrile, difluoroacetonitrile, trifluoroacetonitrile, 2-fluoropropionitrile, 3-fluoropropionitrile, 2,2-difluoropropionitrile, 2,3-difluoropropionitrile, 3,3-difluoropropionitrile, 2,2,3-trifluoropropionitrile, 3,3,3-trifluoropropionitrile, 3,3'-oxydipropionitrile, 3,3'-thiodipropionitrile, pentafluoropropionitrile, methoxyacetonitrile, and benzonitrile. Particularly preferred among these is 3,3,3-trifluoropropionitrile.

In the formula (1c), R^{f}, R^{g}, R^{h}, and Rⁱ are each independently a group containing a cyano group (CN), a hydrogen atom, a halogen atom, an alkyl group, or a group obtainable by replacing at least one hydrogen atom of an alkyl group with a halogen atom.

Examples of the halogen atom, the alkyl group, and the group obtainable by replacing at least one hydrogen atom of an alkyl group with a halogen atom include those mentioned as examples thereof for the formula (1a).

Examples of the group containing a cyano group include a cyano group and a group obtainable by replacing at least one hydrogen atom of an alkyl group with a cyano group. Examples of the alkyl group in this case include those mentioned as examples for the formula (1a).

At least one selected from R^{f}, R^{g}, R^{h}, and Rⁱ is a group containing a cyano group. Preferably, at least two selected from R^{f}, R^{g}, R^{h}, and Rⁱ are each a group containing a cyano group. More preferably, R^{h} and Rⁱ are each a group containing a cyano group. When R^{h} and Rⁱ are each a group containing a cyano group, R^{f} and R^{g} are preferably hydrogen atoms.

In the formula (1c), l is an integer of 1 to 3. When l is 2 or greater, all of l R^{f}s may be the same as each other, or at least part of them may be different from the others. The same applies to R^{g}. In the formula, l is preferably an integer of 1 or 2.

Examples of the nitrile compound represented by the formula (1c) include 3-hexenedinitrile, mucononitrile, maleonitrile, fumaronitrile, acrylonitrile, methacrylonitrile, crotononitrile, 3-methylcrotononitrile, 2-methyl-2-butenenitrile, 2-pentenenitrile, 2-methyl-2-pentenenitrile, 3-methyl-2-pentenenitrile, and 2-hexenenitrile. Preferred are 3-hexenedinitrile and mucononitrile, and particularly preferred is 3-hexenedinitrile.

The nitrile compounds are preferably present in an amount of 0.2 to 7% by mass relative to the electrolyte solution. This can further improve the high-temperature storage characteristics and safety of an electrochemical device at high voltage. The lower limit of the total amount of the nitrile compounds is more preferably 0.3% by mass, still more preferably 0.5% by mass. The upper limit thereof is more preferably 5% by mass, still more preferably 2% by mass, particularly preferably 0.5% by mass.

The electrolyte solution may contain a compound containing an isocyanate group (hereinafter, also abbreviated as "isocyanate"). The isocyanate used may be any isocyanate. Examples of the isocyanate include monoisocyanates, diisocyanates, and triisocyanates.

Specific examples of the monoisocyanates include isocyanatomethane, isocyanatoethane, 1-isocyanatopropane, 1-isocyanatobutane, 1-isocyanatopentane, 1-isocyanatohexane, 1-isocyanatoheptane, 1-isocyanatooctane, 1-isocyanatononane, 1-isocyanatodecane, isocyanatocyclohexane, methoxycarbonyl isocyanate, ethoxycarbonyl isocyanate, propoxycarbonyl isocyanate, butoxycarbonyl isocyanate, methoxysulfonyl isocyanate, ethoxysulfonyl isocyanate, propoxysulfonyl isocyanate, butoxysulfonyl isocyanate, fluorosulfonyl isocyanate, methyl isocyanate, butyl isocyanate, phenyl isocyanate, 2-isocyanatoethyl acrylate, 2-isocyanatoethyl methacrylate, and ethyl isocyanate.

Specific examples of the diisocyanates include 1,4-diisocyanatobutane, 1,5-diisocyanatopentane, 1,6-diisocyanatohexane, 1,7-diisocyanatoheptane, 1,8-diisocyanatooctane, 1,9-diisocyanatononane, 1,10-diisocyanatodecane, 1,3-diisocyanatopropene, 1,4-diisocyanato-2-butene, 1,4-diisocyanato-2-fluorobutane, 1,4-diisocyanato-2,3-difluorobutane, 1,5-diisocyanato-2-pentene, 1,5-diisocyanato-2-methylpentane, 1,6-diisocyanato-2-hexene, 1,6-diisocyanato-3-hexene, 1,6-diisocyanato-3-fluorohexane, 1,6-diisocyanato-3,4-difluorohexane, toluene diisocyanate, xylene diisocyanate, tolylene diisocyanate, 1,2-bis(isocyanatomethyl)cyclohexane, 1,3-bis(isocyanatomethyl)cyclohexane, 1,4-bis(isocyanatomethyl)cyclohexane, 1,2-diisocyanatocyclohexane, 1,3-diisocyanatocyclohexane, 1,4-diisocyanatocyclohexane, dicyclohexylmethane-1,1'-diisocyanate, dicyclohexylmethane-2,2'-diisocyanate, dicyclohexylmethane-3,3'-diisocyanate, dicyclohexylmethane-4,4'-diisocyanate, isophorone diisocyanate, bicyclo[2.2.1]heptane-2,5-diylbis(methyl=isocyanate), bicyclo[2.2.1]heptane-2,6-diylbis(methyl=isocyanate), 2,4,4-trimethylhexamethylene diisocyanate, 2,2,4-trimethylhexamethylene diisocyanate, hexamethylene diisocyanate, 1,4-phenylene diisocyanate, octamethylene diisocyanate, and tetramethylene diisocyanate.

Specific examples of the triisocyanates include 1,6,11-triisocyanatoundecane, 4-isocyanatomethyl-1,8-octamethylene diisocyanate, 1,3,5-triisocyanatomethylbenzene, 1,3,5-tris(6-isocyanatohex-1-yl)-1,3,5-triazine-2,4,6(1H,3H,5H)-trione, and 4-(isocyanatomethyl)octamethylene=diisocyanate.

In order to enable industrially easy availability and cause low cost in production of an electrolyte solution, preferred among these are 1,6-diisocyanatohexane, 1,3-bis(isocyanatomethyl)cyclohexane, 1,3,5-tris(6-isocyanatohex-1-yl)-1,3,5-triazine-2,4,6(1H,3H,5H)-trione, 2,4,4-trimethylhexamethylene diisocyanate, and 2,2,4-trimethylhexamethylene diisocyanate. From the technical viewpoint, they can contribute to formation of a stable film-shaped structure and can therefore more suitably be used.

The isocyanate may be present in any amount that does not significantly impair the effects of the disclosure. The amount is preferably, but not limited to, 0.001% by mass or more and 1.0% by mass or less relative to the electrolyte solution. The isocyanate in an amount of not smaller than this lower limit can give a sufficient effect of improving the cycle characteristics to a non-aqueous electrolyte secondary battery. The isocyanate in an amount of not larger than this upper limit can eliminate an initial increase in resistance of a non-aqueous electrolyte secondary battery. The amount of the isocyanate is more preferably 0.01% by mass or more, still more preferably 0.1% by mass or more, particularly preferably 0.2% by mass or more, while more preferably 0.8% by mass or less, still more preferably 0.7% by mass or less, particularly preferably 0.6% by mass or less.

The electrolyte solution may contain a cyclic sulfonate. The cyclic sulfonate may be any cyclic sulfonate. Examples of the cyclic sulfonate include a saturated cyclic sulfonate, an unsaturated cyclic sulfonate, a saturated cyclic disulfonate, and an unsaturated cyclic disulfonate.

Specific examples of the saturated cyclic sulfonate include 1,3-propanesultone, 1-fluoro-1,3-propanesultone, 2-fluoro-1,3-propanesultone, 3-fluoro-1,3-propanesultone, 1-methyl-1,3-propanesultone, 2-methyl-1,3-propanesultone, 3-methyl-1,3-propanesultone, 1,3-butanesultone, 1,4-butanesultone, 1-fluoro-1,4-butanesultone, 2-fluoro-1,4-butanesultone, 3-fluoro-1,4-butanesultone, 4-fluoro-1,4-butanesultone, 1-methyl-1,4-butanesultone, 2-methyl-1,4-butanesultone, 3-methyl-1,4-butanesultone, 4-methyl-1,4-butanesultone, and 2,4-butanesultone.

Specific examples of the unsaturated cyclic sulfonate include 1-propene-1,3-sultone, 2-propene-1,3-sultone, 1-fluoro-1-propene-1,3-sultone, 2-fluoro-1-propene-1,3-sultone, 3-fluoro-1-propene-1,3-sultone, 1-fluoro-2-propene-1,3-sultone, 2-fluoro-2-propene-1,3-sultone, 3-fluoro-2-propene-1,3-sultone, 1-methyl-1-propene-1,3-sultone, 2-methyl-1-propene-1,3-sultone, 3-methyl-1-propene-1,3-sultone, 1-methyl-2-propene-1,3-sultone, 2-methyl-2-propene-1,3-sultone, 3-methyl-2-propene-1,3-sultone, 1-butene-1,4-sultone, 2-butene-1,4-sultone, 3-butene-1,4-sultone, 1-fluoro-1-butene-1,4-sultone, 2-fluoro-1-butene-1,4-sultone, 3-fluoro-1-butene-1,4-sultone, 4-fluoro-1-butene-1,4-sultone, 1-fluoro-2-butene-1,4-sultone, 2-fluoro-2-butene-1,4-sultone, 3-fluoro-2-butene-1,4-sultone, 4-fluoro-2-butene-1,4-sultone, 1,3-propenesultone, 1-fluoro-3-butene-1,4-sultone, 2-fluoro-3-butene-1,4-sultone, 3-fluoro-3-butene-1,4-sultone, 4-fluoro-3-butene-1,4-sultone, 1-methyl-1-butene-1,4-sultone, 2-methyl-1-butene-1,4-sultone, 3-methyl-1-butene-1,4-sultone, 4-methyl-1-butene-1,4-sultone, 1-methyl-2-butene-1,4-sultone, 2-methyl-2-butene-1,4-sultone, 3-methyl-2-butene-1,4-sultone, 4-methyl-2-butene-1,4-sultone, 1-methyl-3-butene-1,4-sultone, 2-methyl-3-butene-1,4-sultone, 3-methyl-3-butene-1,4-sultone, and 4-methyl-3-butene-1,4-sultone.

In order to enable easy availability and contribute to formation of a stable film-shaped structure, more preferred among these are 1,3-propanesultone, 1-fluoro-1,3-propanesultone, 2-fluoro-1,3-propanesultone, 3-fluoro-1,3-propanesultone, and 1-propene-1,3-sultone. The cyclic sulfonate may be in any amount that does not significantly impair the effects of the disclosure. The amount is preferably, but not limited to, 0.001% by mass or more and 3.0% by mass or less relative to the electrolyte solution.

The cyclic sulfonate in an amount of not smaller than this lower limit can give a sufficient effect of improving the cycle characteristics to a non-aqueous electrolyte secondary battery. The cyclic sulfonate in an amount of not larger than this upper limit can eliminate an increase in the cost of producing a non-aqueous electrolyte secondary battery. The amount of the cyclic sulfonate is more preferably 0.01% by mass or more, still more preferably 0.1% by mass or more, particularly preferably 0.2% by mass or more, while more preferably 2.5% by mass or less, still more preferably 2.0% by mass or less, particularly preferably 1.8% by mass or less.

The electrolyte solution may further contain a polyethylene oxide that has a weight average molecular weight of 2000 to 4000 and has -OH, -OCOOH, or -COOH at an end.

The presence of such a compound can improve the stability at the interfaces with the respective electrodes, improving the characteristics of an electrochemical device.

Examples of the polyethylene oxide include polyethylene oxide monool, polyethylene oxide carboxylic acid, polyethylene oxide diol, polyethylene oxide dicarboxylic acid, polyethylene oxide triol, and polyethylene oxide tricarboxylate. One of these may be used alone or two or more thereof may be used in any combination.

In order to give better battery characteristics, preferred are a mixture of polyethylene oxide monool and polyethylene oxide diol and a mixture of polyethylene carboxylic acid and polyethylene dicarboxylic acid.

The polyethylene oxide having too small a weight average molecular weight may be easily oxidatively decomposed. The weight average molecular weight is more preferably 3000 to 4000.

The weight average molecular weight can be determined by gel permeation chromatography (GPC) in polystyrene equivalent.

The polyethylene oxide is preferably present in an amount of 1 × 10⁻⁶ to 1 × 10⁻² mol/kg in the electrolyte solution. Too large an amount of the polyethylene oxide may cause poor characteristics of an electrochemical device.

The amount of the polyethylene oxide is more preferably 5 × 10⁻⁶ mol/kg or more.

The electrolyte solution may further contain as an additive any of other components such as a fluorinated saturated cyclic carbonate, an unsaturated cyclic carbonate, an overcharge inhibitor, and a known different aid. This can reduce impairment of the characteristics of an electrochemical device.

Examples of the fluorinated saturated cyclic carbonate include compounds represented by the aforementioned formula (A). Preferred among these are fluoroethylene carbonate, difluoroethylene carbonate, monofluoromethyl ethylene carbonate, trifluoromethyl ethylene carbonate, and 2,2,3,3,3-pentafluoropropylethylene carbonate (4-(2,2,3,3,3-pentafluoro-propyl)-[1,3]dioxolan-2-one). One fluorinated saturated cyclic carbonate may be used alone, or two or more thereof may be used in any combination at any ratio.

The fluorinated saturated cyclic carbonate is preferably present in an amount of 0.001 to 10% by mass, more preferably 0.01 to 5% by mass, still more preferably 0.1 to 3% by mass, relative to the electrolyte solution.

Examples of the unsaturated cyclic carbonate include vinylene carbonate compounds, ethylene carbonate compounds substituted with a substituent that contains an aromatic ring, a carbon-carbon double bond, or a carbon-carbon triple bond, phenyl carbonate compounds, vinyl carbonate compounds, allyl carbonate compounds, and catechol carbonate compounds.

Examples of the vinylene carbonate compounds include vinylene carbonate, methylvinylene carbonate, 4,5-dimethylvinylene carbonate, phenylvinylene carbonate, 4,5-diphenylvinylene carbonate, vinylvinylene carbonate, 4,5-divinylvinylene carbonate, allylvinylene carbonate, 4,5-diallylvinylene carbonate, 4-fluorovinylene carbonate, 4-fluoro-5-methylvinylene carbonate, 4-fluoro-5-phenylvinylene carbonate, 4-fluoro-5-vinylvinylene carbonate, 4-allyl-5-fluorovinylene carbonate, ethynylethylene carbonate, propargylethylene carbonate, methylvinylene carbonate, and dimethylvinylene carbonate.

Specific examples of the ethylene carbonate compounds substituted with a substituent that contains an aromatic ring, a carbon-carbon double bond, or a carbon-carbon triple bond include vinylethylene carbonate, 4,5-divinylethylene carbonate, 4-methyl-5-vinylethylene carbonate, 4-allyl-5-vinylethylene carbonate, ethynylethylene carbonate, 4,5-diethynylethylene carbonate, 4-methyl-5-ethynylethylene carbonate, 4-vinyl-5-ethynylethylene carbonate, 4-allyl-5-ethynylethylene carbonate, phenylethylene carbonate, 4,5-diphenylethylene carbonate, 4-phenyl-5-vinylethylene carbonate, 4-allyl-5-phenylethylene carbonate, allylethylene carbonate, 4,5-diallylethylene carbonate, 4-methyl-5-allylethylene carbonate, 4-methylene-1,3-dioxolan-2-one, 4,5-dimethylene-1,3-dioxolan-2-one, and 4-methyl-5-allylethylene carbonate.

The unsaturated cyclic carbonate is preferably vinylene carbonate, methylvinylene carbonate, 4,5-dimethylvinylene carbonate, vinylvinylene carbonate, 4,5-vinylvinylene carbonate, allylvinylene carbonate, 4,5-diallylvinylene carbonate, vinylethylene carbonate, 4,5-divinylethylene carbonate, 4-methyl-5-vinylethylene carbonate, allylethylene carbonate, 4,5-diallylethylene carbonate, 4-methyl-5-allylethylene carbonate, 4-allyl-5-vinylethylene carbonate, ethynylethylene carbonate, 4,5-diethynylethylene carbonate, 4-methyl-5-ethynylethylene carbonate, and 4-vinyl-5-ethynylethylene carbonate. In order to form a more stable interface protecting film, particularly preferred are vinylene carbonate, vinylethylene carbonate, and ethynylethylene carbonate, and most preferred is vinylene carbonate.

The unsaturated cyclic carbonate may have any molecular weight that does not significantly impair the effects of the disclosure. The molecular weight is preferably 50 or higher and 250 or lower. The unsaturated cyclic carbonate having a molecular weight within this range can easily ensure its solubility in the electrolyte solution and can easily lead to sufficient achievement of the effects of the disclosure. The molecular weight of the unsaturated cyclic carbonate is more preferably 80 or higher and 150 or lower.

The unsaturated cyclic carbonate may be produced by any production method, and may be produced by a known method selected as appropriate.

One unsaturated cyclic carbonate may be used alone or two or more thereof may be used in any combination at any ratio.

The unsaturated cyclic carbonate may be present in any amount that does not significantly impair the effects of the disclosure. The amount of the unsaturated cyclic carbonate is preferably 0.001% by mass or more, more preferably 0.01% by mass or more, still more preferably 0.1% by mass or more, of 100% by mass of the electrolyte solution. The amount is preferably 5% by mass or less, more preferably 4% by mass or less, still more preferably 3% by mass or less. The unsaturated cyclic carbonate in an amount within the above range allows an electrochemical device containing the electrolyte solution to easily exhibit a sufficient effect of improving the cycle characteristics, and can easily avoid a situation with impaired high-temperature storage characteristics, generation of a large amount of gas, and a reduced discharge capacity retention.

In addition to the aforementioned non-fluorinated unsaturated cyclic carbonates, a fluorinated unsaturated cyclic carbonate may also suitably be used as an unsaturated cyclic carbonate.

The fluorinated unsaturated cyclic carbonate is a cyclic carbonate containing an unsaturated bond and a fluorine atom. The number of fluorine atoms in the fluorinated unsaturated cyclic carbonate may be any number that is 1 or greater. The number of fluorine atoms is usually 6 or smaller, preferably 4 or smaller, most preferably 1 or 2.

Examples of the fluorinated unsaturated cyclic carbonate include fluorinated vinylene carbonate derivatives and fluorinated ethylene carbonate derivatives substituted with a substituent that contains an aromatic ring or a carbon-carbon double bond.

Examples of the fluorinated vinylene carbonate derivatives include 4-fluorovinylene carbonate, 4-fluoro-5-methylvinylene carbonate, 4-fluoro-5-phenylvinylene carbonate, 4-allyl-5-fluorovinylene carbonate, and 4-fluoro-5-vinylvinylene carbonate.

Examples of the fluorinated ethylene carbonate derivatives substituted with a substituent that contains an aromatic ring or a carbon-carbon double bond include 4-fluoro-4-vinylethylene carbonate, 4-fluoro-4-allylethylene carbonate, 4-fluoro-5-vinylethylene carbonate, 4-fluoro-5-allylethylene carbonate, 4,4-difluoro-4-vinylethylene carbonate, 4,4-difluoro-4-allylethylene carbonate, 4,5-difluoro-4-vinylethylene carbonate, 4,5-difluoro-4-allylethylene carbonate, 4-fluoro-4,5-divinylethylene carbonate, 4-fluoro-4,5-diallylethylene carbonate, 4,5-difluoro-4,5-divinylethylene carbonate, 4,5-difluoro-4,5-diallylethylene carbonate, 4-fluoro-4-phenylethylene carbonate, 4-fluoro-5-phenylethylene carbonate, 4,4-difluoro-5-phenylethylene carbonate, and 4,5-difluoro-4-phenylethylene carbonate.

In order to form a stable interface protecting film, more preferably used as the fluorinated unsaturated cyclic carbonate are 4-fluorovinylene carbonate, 4-fluoro-5-methylvinylene carbonate, 4-fluoro-5-vinylvinylene carbonate, 4-allyl-5-fluorovinylene carbonate, 4-fluoro-4-vinylethylene carbonate, 4-fluoro-4-allylethylene carbonate, 4-fluoro-5-vinylethylene carbonate, 4-fluoro-5-allylethylene carbonate, 4,4-difluoro-4-vinylethylene carbonate, 4,4-difluoro-4-allylethylene carbonate, 4,5-difluoro-4-vinylethylene carbonate, 4,5-difluoro-4-allylethylene carbonate, 4-fluoro-4,5-divinylethylene carbonate, 4-fluoro-4,5-diallylethylene carbonate, 4,5-difluoro-4,5-divinylethylene carbonate, and 4,5-difluoro-4,5-diallylethylene carbonate.

The fluorinated unsaturated cyclic carbonate may have any molecular weight that does not significantly impair the effects of the disclosure. The molecular weight is preferably 50 or higher and 500 or lower. The fluorinated unsaturated cyclic carbonate having a molecular weight within this range can easily ensure the solubility of the fluorinated unsaturated cyclic carbonate in the electrolyte solution.

The fluorinated unsaturated cyclic carbonate may be produced by any method, and may be produced by any known method selected as appropriate. The molecular weight is more preferably 100 or higher and 200 or lower.

One fluorinated unsaturated cyclic carbonate may be used alone or two or more thereof may be used in any combination at any ratio. The fluorinated unsaturated cyclic carbonate may be contained in any amount that does not significantly impair the effects of the disclosure. The amount of the fluorinated unsaturated cyclic carbonate is usually preferably 0.001% by mass or more, more preferably 0.01% by mass or more, still more preferably 0.1% by mass or more, while preferably 5% by mass or less, more preferably 4% by mass or less, still more preferably 3% by mass or less, of 100% by mass of the electrolyte solution. The fluorinated unsaturated cyclic carbonate in an amount within this range allows an electrochemical device containing the electrolyte solution to exhibit an effect of sufficiently improving the cycle characteristics and can easily avoid a situation with reduced high-temperature storage characteristics, generation of a large amount of gas, and a reduced discharge capacity retention.

The electrolyte solution may contain a compound containing a triple bond. This compound may be of any type as long as it contains one or more triple bonds in the molecule.

Specific examples of the compound containing a triple bond include the following compounds:
hydrocarbon compounds such as 1-penthyne, 2-penthyne, 1-hexyne, 2-hexyne, 3-hexyne, 1-heptyne, 2-heptyne, 3-heptyne, 1-octyne, 2-octyne, 3-octyne, 4-octyne, 1-nonyne, 2-nonyne, 3-nonyne, 4-nonyne, 1-dodecyne, 2-dodecyne, 3-dodecyne, 4-dodecyne, 5-dodecyne, phenyl acetylene, 1-phenyl-1-propyne, 1-phenyl-2-propyne, 1-phenyl-1-butyne, 4-phenyl-1-butyne, 4-phenyl-1-butyne, 1-phenyl-1-penthyne, 5-phenyl-1-penthyne, 1-phenyl-1-hexyne, 6-phenyl-1-hexyne, diphenyl acetylene, 4-ethynyl toluene, and dicyclohexyl acetylene;
monocarbonates such as 2-propynylmethyl carbonate, 2-propynylethyl carbonate, 2-propynylpropyl carbonate, 2-propynylbutyl carbonate, 2-propynylphenyl carbonate, 2-propynylcyclohexyl carbonate, di-2-propynylcarbonate, 1-methyl-2-propynylmethyl carbonate, 1,1-dimethyl-2-propynylmethyl carbonate, 2-butynylmethyl carbonate, 3-butynylmethyl carbonate, 2-pentynylmethyl carbonate, 3-pentynylmethyl carbonate, and 4-pentynylmethyl carbonate; dicarbonates such as 2-butyne-1,4-diol dimethyl dicarbonate, 2-butyne-1,4-diol diethyl dicarbonate, 2-butyne-1,4-diol dipropyl dicarbonate, 2-butyne-1,4-diol dibutyl dicarbonate, 2-butyne-1,4-diol diphenyl dicarbonate, and 2-butyne-1,4-diol dicyclohexyl dicarbonate;
monocarboxylates such as 2-propynyl acetate, 2-propynyl propionate, 2-propynyl butyrate, 2-propynyl benzoate, 2-propynyl cyclohexylcarboxylate, 1,1-dimethyl-2-propynyl acetate, 1,1-dimethyl-2-propynyl propionate, 1,1-dimethyl-2-propynyl butyrate, 1,1-dimethyl-2-propynyl benzoate, 1,1-dimethyl-2-propynyl cyclohexylcarboxylate, 2-butynyl acetate, 3-butynyl acetate, 2-pentynyl acetate, 3-pentynyl acetate, 4-pentynyl acetate, methyl acrylate, ethyl acrylate, propyl acrylate, vinyl acrylate, 2-propenyl acrylate, 2-butenyl acrylate, 3-butenyl acrylate, methyl methacrylate, ethyl methacrylate, propyl methacrylate, vinyl methacrylate, 2-propenyl methacrylate, 2-butenyl methacrylate, 3-butenyl methacrylate, methyl 2-propynoate, ethyl 2-propynoate, propyl 2-propynoate, vinyl 2-propynoate, 2-propenyl 2-propynoate, 2-butenyl 2-propynoate, 3-butenyl 2-propynoate, methyl 2-butynoate, ethyl 2-butynoate, propyl 2-butynoate, vinyl 2-butynoate, 2-propenyl 2-butynoate, 2-butenyl 2-butynoate, 3-butenyl 2-butynoate, methyl 3-butynoate, ethyl 3-butynoate, propyl 3-butynoate, vinyl 3-butynoate, 2-propenyl 3-butynoate, 2-butenyl 3-butynoate, 3-butenyl 3-butynoate, methyl 2-penthynoate, ethyl 2-penthynoate, propyl 2-penthynoate, vinyl 2-penthynoate, 2-propenyl 2-penthynoate, 2-butenyl 2-penthynoate, 3-butenyl 2-penthynoate, methyl 3-penthynoate, ethyl 3-penthynoate, propyl 3-penthynoate, vinyl 3-penthynoate, 2-propenyl 3-penthynoate, 2-butenyl 3-penthynoate, 3-butenyl 3-penthynoate, methyl 4-penthynoate, ethyl 4-penthynoate, propyl 4-penthynoate, vinyl 4-penthynoate, 2-propenyl 4-penthynoate, 2-butenyl 4-penthynoate, and 3-butenyl 4-penthynoate, fumarates, methyl trimethylacetate, and ethyl trimethylacetate;
dicarboxylates such as 2-butyne-1,4-diol diacetate, 2-butyne-1,4-diol dipropionate, 2-butyne-1,4-diol dibutyrate, 2-butyne-1,4-diol dibenzoate, 2-butyne-1,4-diol dicyclohexanecarboxylate, hexahydrobenzo[1,3,2]dioxathiolane-2-oxide (1,2-cyclohexane diol), 2,2-dioxide-1,2-oxathiolan-4-yl acetate, and 2,2-dioxide-1,2-oxathiolan-4-yl acetate;
oxalic acid diesters such as methyl 2-propynyl oxalate, ethyl 2-propynyl oxalate, propyl 2-propynyl oxalate, 2-propynyl vinyl oxalate, allyl 2-propynyl oxalate, di-2-propynyl oxalate, 2-butynyl methyl oxalate, 2-butynyl ethyl oxalate, 2-butynyl propyl oxalate, 2-butynyl vinyl oxalate, allyl 2-butynyl oxalate, di-2-butynyl oxalate, 3-butynyl methyl oxalate, 3-butynyl ethyl oxalate, 3-butynyl propyl oxalate, 3-butynyl vinyl oxalate, allyl 3-butynyl oxalate, and di-3-butynyl oxalate;
phosphine oxides such as methyl(2-propynyl) (vinyl)phosphine oxide, divinyl(2-propynyl)phosphine oxide, di(2-propynyl) (vinyl)phosphine oxide, di(2-propenyl)2(-propynyl)phosphine oxide, di(2-propynyl) (2-propenyl)phosphine oxide, di(3-butenyl) (2-propynyl)phosphine oxide, and di(2-propynyl) (3-butenyl)phosphine oxide;
phosphinates such as 2-propynyl methyl(2-propenyl)phosphinate, 2-propynyl 2-butenyl(methyl)phosphinate, 2-propynyl di(2-propenyl)phosphinate, 2-propynyl di(3-butenyl)phosphinate, 1,1-dimethyl-2-propynyl methyl(2-propenyl)phosphinate, 1,1-dimethyl-2-propynyl 2-butenyl(methyl)phosphinate, 1,1-dimethyl-2-propynyl di(2-propenyl)phosphinate, 1,1-dimethyl-2-propynyl di(3-butenyl)phosphinate, 2-propenyl methyl(2-propynyl)phosphinate, 3-butenyl methyl(2-propynyl)phosphinate, 2-propenyl di(2-propynyl)phosphinate, 3-butenyl di(2-propynyl)phosphinate, 2-propenyl 2-propynyl(2-propenyl)phosphinate, and 3-butenyl 2-propynyl(2-propenyl)phosphinate;
phosphonates such as methyl 2-propynyl 2-propenylphosphonate, methyl(2-propynyl) 2-butenylphosphonate, (2-propynyl) (2-propenyl) 2-propenylphosphonate, (3-butenyl) (2-propynyl) 3-butenylphosphonate, (1,1-dimethyl-2-propynyl)(methyl) 2-propenylphosphonate, (1,1-dimethyl-2-propynyl)(methyl) 2-butenylphosphonate, (1,1-dimethyl-2-propynyl) (2-propenyl) 2-propenylphosphonate, (3-butenyl) (1,1-dimethyl-2-propynyl) 3-butenylphosphonate, (2-propynyl) (2-propenyl) methylphosphonate, (3-butenyl) (2-propynyl) methylphosphonate, (1,1-dimethyl-2-propynyl) (2-propenyl) methylphosphonate, (3-butenyl) (1,1-dimethyl-2-propynyl) methylphosphonate, (2-propynyl) (2-propenyl) ethylphosphonate, (3-butenyl) (2-propynyl) ethylphosphonate, (1,1-dimethyl-2-propynyl) (2-propenyl) ethylphosphonate, and (3-butenyl)(1,1-dimethyl-2-propynyl) ethylphosphonate; and
phosphates such as (methyl) (2-propenyl) (2-propynyl) phosphate, (ethyl) (2-propenyl) (2-propynyl) phosphate, (2-butenyl) (methyl) (2-propynyl) phosphate, (2-butenyl) (ethyl) (2-propynyl) phosphate, (1,1-dimethyl-2-propynyl) (methyl) (2-propenyl) phosphate, (1,1-dimethyl-2-propynyl) (ethyl) (2-propenyl) phosphate, (2-butenyl) (1,1-dimethyl-2-propynyl) (methyl) phosphate, and (2-butenyl) (ethyl) (1,1-dimethyl-2-propynyl) phosphate.

In order to more stably form a negative electrode film in the electrolyte solution, preferred among these are compounds containing an alkynyloxy group.

In order to improve the storage characteristics, particularly preferred are compounds such as 2-propynylmethyl carbonate, di-2-propynyl carbonate, 2-butyne-1,4-diol dimethyl dicarbonate, 2-propynyl acetate, 2-butyne-1,4-diol diacetate, methyl 2-propynyl oxalate, and di-2-propynyl oxalate.

One compound containing a triple bond may be used alone or two or more thereof may be used in any combination at any ratio. The compound containing a triple bond relative to the whole electrolyte solution may be present in any amount that does not significantly impair the effects of the disclosure. The compound is usually contained at a concentration of 0.01% by mass or more, preferably 0.05% by mass or more, more preferably 0.1% by mass or more, while usually 5% by mass or less, preferably 3% by mass or less, more preferably 1% by mass or less, relative to the electrolyte solution of the disclosure. The compound satisfying the above range can further improve the effects such as output characteristics, load characteristics, cycle characteristics, and high-temperature storage characteristics.

In order to effectively reduce burst or combustion of a battery in case of overcharge, for example, of an electrochemical device containing the electrolyte solution, the electrolyte solution may contain an overcharge inhibitor.

Examples of the overcharge inhibitor include aromatic compounds, including unsubstituted or alkyl-substituted terphenyl derivatives such as biphenyl, o-terphenyl, m-terphenyl, and p-terphenyl, partially hydrogenated products of unsubstituted or alkyl-substituted terphenyl derivatives, cyclohexylbenzene, t-butylbenzene, t-amylbenzene, diphenyl ether, dibenzofuran, diphenyl cyclohexane, 1,1,3-trimethyl-3-phenylindan, cyclopentylbenzene, cyclohexylbenzene, cumene, 1,3-diisopropylbenzene, 1,4-diisopropylbenzene, t-butylbenzene, t-amylbenzene, t-hexylbenzene, and anisole; partially fluorinated products of the aromatic compounds such as 2-fluorobiphenyl, 4-fluorobiphenyl, o-cyclohexylfluorobenzene, p-cyclohexylfluorobenzene, o-cyclohexylfluorobenzene, p-cyclohexylfluorobenzene, fluorotoluene, and benzotrifluoride; fluorine-containing anisole compounds such as 2,4-difluoroanisole, 2,5-difluoroanisole, 1,6-difluoroanisole, 2,6-difluoroanisole, and 3,5-difluoroanisole; aromatic acetates such as 3-propylphenyl acetate, 2-ethylphenyl acetate, benzylphenyl acetate, methylphenyl acetate, benzyl acetate, and phenethylphenyl acetate; aromatic carbonates such as diphenyl carbonate and methylphenyl carbonate, toluene derivatives such as toluene and xylene, and unsubstituted or alkyl-substituted biphenyl derivatives such as 2-methylbiphenyl, 3-methylbiphenyl, 4-methylbiphenyl, and o-cyclohexylbiphenyl. Preferred among these are aromatic compounds such as biphenyl, alkylbiphenyl, terphenyl, partially hydrogenated terphenyl, cyclohexylbenzene, t-butylbenzene, t-amylbenzene, diphenyl ether, and dibenzofuran, diphenyl cyclohexane, 1,1,3-trimethyl-3-phenylindan, 3-propylphenyl acetate, 2-ethylphenyl acetate, benzylphenyl acetate, methylphenyl acetate, benzyl acetate, diphenyl carbonate, and methylphenyl carbonate. One of these compounds may be used alone or two or more thereof may be used in any combination. In order to achieve good balance between the overcharge inhibiting characteristics and the high-temperature storage characteristics with a combination use of two or more thereof, preferred is a combination of cyclohexylbenzene and t-butylbenzene or t-amylbenzene, or a combination of at least one oxygen-free aromatic compound selected from the group consisting of biphenyl, alkylbiphenyl, terphenyl, partially hydrogenated terphenyl, cyclohexylbenzene, t-butylbenzene, t-amylbenzene, and the like and at least one oxygencontaining aromatic compound selected from the group consisting of diphenyl ether, dibenzofuran, and the like.

The electrolyte solution used in the disclosure may contain a carboxylic anhydride other than the compound (2). The carboxylic anhydride is preferably a compound represented by the following formula (6). The carboxylic anhydride may be produced by any method which may be selected from known methods as appropriate.

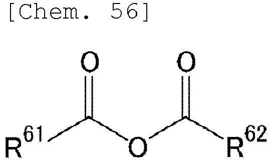

In the formula (6), R⁶¹ and R⁶² are each independently a hydrocarbon group having a carbon number of 1 or greater and 15 or smaller and optionally containing a substituent.

R⁶¹ and R⁶² each may be any monovalent hydrocarbon group. For example, each of them may be either an aliphatic hydrocarbon group or an aromatic hydrocarbon group, or may be a bond of an aliphatic hydrocarbon group and an aromatic hydrocarbon group. The aliphatic hydrocarbon group may be a saturated hydrocarbon group and may contain an unsaturated bond (carbon-carbon double bond or carbon-carbon triple bond). The aliphatic hydrocarbon group may be either acyclic or cyclic. In the case of an acyclic group, it may be either linear or branched. The group may be a bond of an acyclic group and a cyclic group. R⁶¹ and R⁶² may be the same as or different from each other.

When the hydrocarbon group for R⁶¹ and R⁶² contains a substituent, the substituent may be of any type as long as it is not beyond the scope of the disclosure. Examples thereof include halogen atoms such as a fluorine atom, a chlorine atom, a bromine atom, and an iodine atom. Preferred is a fluorine atom. Examples of the substituent other than the halogen atoms include substituents containing a functional group such as an ester group, a cyano group, a carbonyl group, or an ether group. Preferred are a cyano group and a carbonyl group. The hydrocarbon group for R⁶¹ and R⁶² may contain only one of these substituents or may contain two or more thereof. When two or more substituents are contained, these substituents may be the same as or different from each other.

The hydrocarbon group for R⁶¹ and R⁶² has a carbon number of usually 1 or greater, while usually 15 or smaller, preferably 12 or smaller, more preferably 10 or smaller, still more preferably 9 or smaller. When R⁶¹ and R⁶² bind to each other to form a divalent hydrocarbon group, the divalent hydrocarbon group has a carbon number of usually 1 or greater, while usually 15 or smaller, preferably 13 or smaller, more preferably 10 or smaller, still more preferably 8 or smaller. When the hydrocarbon group for R⁶¹ and R⁶² contains a substituent that contains a carbon atom, the carbon number of the whole R⁶¹ or R⁶² including the substituent preferably satisfies the above range.

Next, specific examples of the acid anhydride represented by the formula (6) are described. In the following examples, the term "analog" means an acid anhydride obtainable by replacing part of the structure of an acid anhydride mentioned as an example with another structure within the scope of the disclosure. Examples thereof include dimers, trimers, and tetramers each composed of a plurality of acid anhydrides, those having respective substituents which are structural isomers having the same carbon number but having different branch structures, and those having the same substituent but at different sites in an acid anhydride.

First, specific examples of an acid anhydride in which R⁶¹ and R⁶² are the same as each other are described.

Specific examples of an acid anhydride in which R⁶¹ and R⁶² are acyclic alkyl groups include acetic anhydride, propionic anhydride, butanoic anhydride, 2-methylpropionic anhydride, 2,2-dimethylpropionic anhydride, 2-methylbutanoic anhydride, 3-methylbutanoic anhydride, 2,2-dimethylbutanoic anhydride, 2,3-dimethylbutanoic anhydride, 3,3-dimethylbutanoic anhydride, 2,2,3-trimethylbutanoic anhydride, 2,3,3-trimethylbutanoic anhydride, 2,2,3,3-tetramethylbutanoic anhydride, and 2-ethylbutanoic anhydride, and analogs thereof.

Specific examples of an acid anhydride in which R⁶¹ and R⁶² are cyclic alkyl groups include cyclopropanecarboxylic anhydride, cyclopentanecarboxylic anhydride, and cyclohexanecarboxylic anhydride, and analogs thereof.

Specific examples of an acid anhydride in which R⁶¹ and R⁶² are alkenyl groups include acrylic anhydride, 2-methylacrylic anhydride, 3-methylacrylic anhydride, 2,3-dimethylacrylic anhydride, 3,3-dimethylacrylic anhydride, 2,3,3-trimethylacrylic anhydride, 2-phenylacrylic anhydride, 3-phenylacrylic anhydride, 2,3-diphenylacrylic anhydride, 3,3-diphenylacrylic anhydride, 3-butenoic anhydride, 2-methyl-3-butenoic anhydride, 2,2-dimethyl-3-butenoic anhydride, 3-methyl-3-butenoic anhydride, 2-methyl-3-methyl-3-butenoic anhydride, 2,2-dimethyl-3-methyl-3-butenoic anhydride, 3-pentenoic anhydride, 4-pentenoic anhydride, 2-cyclopentenecarboxylic anhydride, 3-cyclopentenecarboxylic anhydride, and 4-cyclopentenecarboxylic anhydride, and analogs thereof.

Specific examples of an acid anhydride in which R⁶¹ and R⁶² are alkynyl groups include propynoic anhydride, 3-phenylpropynoic anhydride, 2-butynoic anhydride, 2-penthynoic anhydride, 3-butynoic anhydride, 3-penthynoic anhydride, and 4-penthynoic anhydride, and analogs thereof.

Specific examples of an acid anhydride in which R⁶¹ and R⁶² are aryl groups include benzoic anhydride, 4-methylbenzoic anhydride, 4-ethylbenzoic anhydride, 4-tert-butylbenzoic anhydride, 2-methylbenzoic anhydride, 2,4,6-trimethylbenzoic anhydride, 1-naphthalenecarboxylic anhydride, and 2-naphthalenecarboxylic anhydride, and analogs thereof.

Examples of an acid anhydride substituted with a fluorine atom are mainly listed below as examples of the acid anhydride in which R⁶¹ and R⁶² are substituted with a halogen atom. Acid anhydrides obtainable by replacing any or all the fluorine atoms thereof with a chlorine atom, a bromine atom, or an iodine atom are also included in the exemplary compounds.

Examples of an acid anhydride in which R⁶¹ and R⁶² are halogen-substituted acyclic alkyl groups include fluoroacetic anhydride, difluoroacetic anhydride, trifluoroacetic anhydride, 2-fluoropropionic anhydride, 2,2-difluoropropionic anhydride, 2,3-difluoropropionic anhydride, 2,2,3-trifluoropropionic anhydride, 2,3,3-trifluoropropionic anhydride, 2,2,3,3-tetrapropionic anhydride, 2,3,3,3-tetrapropionic anhydride, 3-fluoropropionic anhydride, 3,3-difluoropropionic anhydride, 3,3,3-trifluoropropionic anhydride, and perfluoropropionic anhydride, and analogs thereof.

Examples of an acid anhydride in which R⁶¹ and R⁶² are halogen-substituted cyclic alkyl groups include 2-fluorocyclopentanecarboxylic anhydride, 3-fluorocyclopentanecarboxylic anhydride, and 4-fluorocyclopentanecarboxylic anhydride, and analogs thereof.

Examples of an acid anhydride in which R⁶¹ and R⁶² are halogen-substituted alkenyl groups include 2-fluoroacrylic anhydride, 3-fluoroacrylic anhydride, 2,3-difluoroacrylic anhydride, 3,3-difluoroacrylic anhydride, 2,3,3-trifluoroacrylic anhydride, 2-(trifluoromethyl)acrylic anhydride, 3-(trifluoromethyl)acrylic anhydride, 2,3-bis(trifluoromethyl)acrylic anhydride, 2,3,3-tris(trifluoromethyl)acrylic anhydride, 2-(4-fluorophenyl)acrylic anhydride, 3-(4-fluorophenyl)acrylic anhydride, 2,3-bis(4-fluorophenyl)acrylic anhydride, 3,3-bis(4-fluorophenyl)acrylic anhydride, 2-fluoro-3-butenoic anhydride, 2,2-difluoro-3-butenoic anhydride, 3-fluoro-2-butenoic anhydride, 4-fluoro-3-butenoic anhydride, 3,4-difluoro-3-butenoic anhydride, and 3,3,4-trifluoro-3-butenoic anhydride, and analogs thereof.

Examples of an acid anhydride in which R⁶¹ and R⁶² are halogen-substituted alkynyl groups include 3-fluoro-2-propynoic anhydride, 3-(4-fluorophenyl)-2-propynoic anhydride, 3-(2,3,4,5,6-pentafluorophenyl)-2-propynoic anhydride, 4-fluoro-2-butynoic anhydride, 4,4-difluoro-2-butynoic anhydride, and 4,4,4-trifluoro-2-butynoic anhydride, and analogs thereof.

Examples of an acid anhydride in which R⁶¹ and R⁶² are halogen-substituted aryl groups include 4-fluorobenzoic anhydride, 2,3,4,5,6-pentafluorobenzoic anhydride, and 4-trifluoromethylbenzoic anhydride, and analogs thereof.

Examples of an acid anhydride in which R⁶¹ and R⁶² each contain a substituent containing a functional group such as an ester, a nitrile, a ketone, an ether, or the like include methoxyformic anhydride, ethoxyformic anhydride, methyloxalic anhydride, ethyloxalic anhydride, 2-cyanoacetic anhydride, 2-oxopropionic anhydride, 3-oxobutanoic anhydride, 4-acetylbenzoic anhydride, methoxyacetic anhydride, and 4-methoxybenzoic anhydride, and analogs thereof.

Then, specific examples of an acid anhydride in which R⁶¹ and R⁶² are different from each other are described below.

R⁶¹ and R⁶² may be in any combination of those mentioned as examples above and analogs thereof. The following gives representative examples.

Examples of a combination of acyclic alkyl groups include acetic propionic anhydride, acetic butanoic anhydride, butanoic propionic anhydride, and acetic 2-methylpropionic anhydride.

Examples of a combination of an acyclic alkyl group and a cyclic alkyl group include acetic cyclopentanoic anhydride, acetic cyclohexanoic anhydride, and cyclopentanoic propionic anhydride.

Examples of a combination of an acyclic alkyl group and an alkenyl group include acetic acrylic anhydride, acetic 3-methylacrylic anhydride, acetic 3-butenoic anhydride, and acrylic propionic anhydride.

Examples of a combination of an acyclic alkyl group and an alkynyl group include acetic propynoic anhydride, acetic 2-butynoic anhydride, acetic 3-butynoic anhydride, acetic 3-phenyl propynoic anhydride, and propionic propynoic anhydride.

Examples of a combination of an acyclic alkyl group and an aryl group include acetic benzoic anhydride, acetic 4-methylbenzoic anhydride, acetic 1-naphthalenecarboxylic anhydride, and benzoic propionic anhydride.

Examples of a combination of an acyclic alkyl group and a hydrocarbon group containing a functional group include acetic fluoroacetic anhydride, acetic trifluoroacetic anhydride, acetic 4-fluorobenzoic anhydride, fluoroacetic propionic anhydride, acetic alkyloxalic anhydride, acetic 2-cyanoacetic anhydride, acetic 2-oxopropionic anhydride, acetic methoxyacetic anhydride, and methoxyacetic propionic anhydride.

Examples of a combination of cyclic alkyl groups include cyclopentanoic cyclohexanoic anhydride.

Examples of a combination of a cyclic alkyl group and an alkenyl group include acrylic cyclopentanoic anhydride, 3-methylacrylic cyclopentanoic anhydride, 3-butenoic cyclopentanoic anhydride, and acrylic cyclohexanoic anhydride.

Examples of a combination of a cyclic alkyl group and an alkynyl group include propynoic cyclopentanoic anhydride, 2-butynoic cyclopentanoic anhydride, and propynoic cyclohexanoic anhydride.

Examples of a combination of a cyclic alkyl group and an aryl group include benzoic cyclopentanoic anhydride, 4-methylbenzoic cyclopentanoic anhydride, and benzoic cyclohexanoic anhydride.

Examples of a combination of a cyclic alkyl group and a hydrocarbon group containing a functional group include fluoroacetic cyclopentanoic anhydride, cyclopentanoic trifluoroacetic anhydride, cyclopentanoic 2-cyanoacetic anhydride, cyclopentanoic methoxyacetic anhydride, and cyclohexanoic fluoroacetic anhydride.

Examples of a combination of alkenyl groups include acrylic 2-methylacrylic anhydride, acrylic 3-methylacrylic anhydride, acrylic 3-butenoic anhydride, and 2-methylacrylic 3-methylacrylic anhydride.

Examples of a combination of an alkenyl group and an alkynyl group include acrylic propynoic anhydride, acrylic 2-butynoic anhydride, and 2-methylacrylic propynoic anhydride.

Examples of a combination of an alkenyl group and an aryl group include acrylic benzoic anhydride, acrylic 4-methylbenzoic anhydride, and 2-methylacrylic benzoic anhydride.

Examples of a combination of an alkenyl group and a hydrocarbon group containing a functional group include acrylic fluoroacetic anhydride, acrylic trifluoroacetic anhydride, acrylic 2-cyanoacetic anhydride, acrylic methoxyacetic anhydride, and 2-methylacrylic fluoroacetic anhydride.

Examples of a combination of alkynyl groups include propynoic 2-butynoic anhydride, propynoic 3-butynoic anhydride, and 2-butynoic 3-butynoic anhydride.

Examples of a combination of an alkynyl group and an aryl group include benzoic propynoic anhydride, 4-methylbenzoic propynoic anhydride, and benzoic 2-butynoic anhydride.

Examples of a combination of an alkynyl group and a hydrocarbon group containing a functional group include propynoic fluoroacetic anhydride, propynoic trifluoroacetic anhydride, propynoic 2-cyanoacetic anhydride, propynoic methoxyacetic anhydride, and 2-butynoic fluoroacetic anhydride.

Examples of a combination of aryl groups include benzoic 4-methylbenzoic anhydride, benzoic 1-naphthalenecarboxylic anhydride, and 4-methylbenzoic 1-naphthalenecarboxylic anhydride.

Examples of a combination of an aryl group and a hydrocarbon group containing a functional group include benzoic fluoroacetic anhydride, benzoic trifluoroacetic anhydride, benzoic 2-cyanoacetic anhydride, benzoic methoxyacetic anhydride, and 4-methylbenzoic fluoroacetic anhydride.

Examples of a combination of hydrocarbon groups each containing a functional group include fluoroacetic trifluoroacetic anhydride, fluoroacetic 2-cyanoacetic anhydride, fluoroacetic methoxyacetic anhydride, and trifluoroacetic 2-cyanoacetic anhydride.

Preferred among the acid anhydrides having an acyclic structure are acetic anhydride, propionic anhydride, 2-methylpropionic anhydride, cyclopentanecarboxylic anhydride, cyclohexanecarboxylic anhydride, acrylic anhydride, 2-methylacrylic anhydride, 3-methylacrylic anhydride, 2,3-dimethylacrylic anhydride, 3,3-dimethylacrylic anhydride, 3-butenoic anhydride, 2-methyl-3-butenoic anhydride, propynoic anhydride, 2-butynoic anhydride, benzoic anhydride, 2-methylbenzoic anhydride, 4-methylbenzoic anhydride, 4-tert-butylbenzoic anhydride, trifluoroacetic anhydride, 3,3,3-trifluoropropionic anhydride, 2-(trifluoromethyl)acrylic anhydride, 2-(4-fluorophenyl)acrylic anhydride, 4-fluorobenzoic anhydride, 2,3,4,5,6-pentafluorobenzoic anhydride, methoxyformic anhydride, and ethoxyformic anhydride. More preferred are acrylic anhydride, 2-methylacrylic anhydride, 3-methylacrylic anhydride, benzoic anhydride, 2-methylbenzoic anhydride, 4-methylbenzoic anhydride, 4-tert-butylbenzoic anhydride, 4-fluorobenzoic anhydride, 2,3,4,5,6-pentafluorobenzoic anhydride, methoxyformic anhydride, and ethoxyformic anhydride.

These compounds are preferred because they can appropriately form a bond with lithium oxalate to provide a film having excellent durability, thereby improving especially the charge and discharge rate characteristics after a durability test, input and output characteristics, and impedance characteristics.

The carboxylic anhydride may have any molecular weight that does not significantly impair the effects of the disclosure. The molecular weight is usually 90 or higher, preferably 95 or higher, while usually 300 or lower, preferably 200 or lower. The carboxylic anhydride having a molecular weight within the above range can reduce an increase in viscosity of an electrolyte solution and can give a reasonable film density, appropriately improving durability.

The carboxylic anhydride may be formed by any production method which may be selected from known methods. One of the carboxylic anhydrides described above alone may be contained in the non-aqueous electrolyte solution of the disclosure, or two or more thereof may be contained in any combination at any ratio.

The carboxylic anhydride may be contained in any amount that does not significantly impair the effects of the disclosure relative to the electrolyte solution. The carboxylic anhydride is usually contained at a concentration of 0.01% by mass or more, preferably 0.1% by mass or more, while usually 5% by mass or less, preferably 3% by mass or less, relative to the electrolyte solution. The carboxylic anhydride in an amount within the above range can easily achieve an effect of improving the cycle characteristics and have good reactivity, easily improving the battery characteristics.

The electrolyte solution may further contain a known different aid. Examples of the different aid include hydrocarbon compounds such as pentane, heptane, octane, nonane, decane, cycloheptane, benzene, furan, naphthalene, 2-phenyl bicyclohexyl, cyclohexane, 2,4,8,10-tetraoxaspiro[5.5]undecane, and 3,9-divinyl-2,4,8,10-tetraoxaspiro[5.5]undecane;
fluorine-containing aromatic compounds such as fluorobenzene, difluorobenzene, hexafluorobenzene, benzotrifluoride, monofluorobenzene, 1-fluoro-2-cyclohexylbenzene, 1-fluoro-4-tert-butylbenzene, 1-fluoro-3-cyclohexylbenzene, 1-fluoro-2-cyclohexylbenzene, and biphenyl fluoride;
carbonate compounds such as erythritan carbonate, spiro-bis-dimethylene carbonate, and methoxyethyl-methyl carbonate;
ether compounds such as dioxolane, dioxane, 2,5,8,11-tetraoxadodecane, 2,5,8,11,14-pentaoxapentadecane, ethoxymethoxyethane, trimethoxymethane, glyme, and ethyl monoglyme;
ketone compounds such as dimethyl ketone, diethyl ketone, and 3-pentanone;
acid anhydrides such as 2-allylsuccinic anhydride;
ester compounds such as dimethyl oxalate, diethyl oxalate, ethylmethyl oxalate, di(2-propynyl) oxalate, methyl 2-propynyl oxalate, dimethyl succinate, di(2-propynyl) glutarate, methyl formate, ethyl formate, 2-propynyl formate, 2-butyne-1,4-diyl diformate, 2-propynyl methacrylate, and dimethyl malonate;
amide compounds such as acetamide, N-methyl formamide, N,N-dimethyl formamide, and N,N-dimethyl acetamide;
sulfur-containing compounds such as ethylene sulfate, vinylene sulfate, ethylene sulfite, methyl fluorosulfonate, ethyl fluorosulfonate, methyl methanesulfonate, ethyl methanesulfonate, busulfan, sulfolene, diphenyl sulfone, N,N-dimethylmethanesulfonamide, N,N-diethylmethanesulfonamide, methyl vinyl sulfonate, ethyl vinyl sulfonate, allyl vinyl sulfonate, propargyl vinyl sulfonate, methyl allyl sulfonate, ethyl allyl sulfonate, allyl allyl sulfonate, propargyl allyl sulfonate, 1,2-bis(vinylsulfonyloxy)ethane, propanedisulfonic anhydride, sulfobutyric anhydride, sulfobenzoic anhydride, sulfopropionic anhydride, ethanedisulfonic anhydride, methylene methanedisulfonate, 2-propynyl methanesulfonate, pentene sulfite, pentafluorophenyl methanesulfonate, propylene sulfate, propylene sulfite, propane sultone, butylene sulfite, butane-2,3-diyl dimethanesulfonate, 2-butyne-1,4-diyl dimethanesulfonate, 2-propynyl vinyl sulfonate, bis(2-vinylsulfonylethyl)ether, 5-vinyl-hexahydro-1,3,2-benzodioxathiol-2-oxide, 2-propynyl 2-(methanesulfonyloxy)propionate, 5,5-dimethyl-1,2-oxathiolan-4-one 2,2-dioxide, 3-sulfo-propionic anhydride, trimethylene methanedisulfonate, 2-methyl tetrahydrofuran, trimethylene methanedisulfonate, tetramethylene sulfoxide, dimethylene methanedisulfonate, difluoroethyl methyl sulfone, divinyl sulfone, 1,2-bis(vinylsulfonyl)ethane, methyl ethylenebissulfonate, ethyl ethylenebissulfonate, ethylene sulfate, and thiophene 1-oxide;
nitrogen-containing compounds such as 1-methyl-2-pyrrolidinone, 1-methyl-2-piperidone, 3-methyl-2-oxazolidinone, 1,3-dimethyl-2-imidazolidinone, N-methylsuccinimide, nitromethane, nitroethane, and ethylene diamine;
phosphorus-containing compounds such as trimethyl phosphite, triethyl phosphite, triphenyl phosphite, trimethyl phosphate, triethyl phosphate, triphenyl phosphate, dimethyl methyl phosphonate, diethyl ethyl phosphonate, dimethyl vinyl phosphonate, diethyl vinyl phosphonate, ethyl diethyl phosphonoacetate, methyl dimethyl phosphinate, ethyl diethyl phosphinate, trimethylphosphine oxide, triethylphosphine oxide, bis(2,2-difluoroethyl)2,2,2-trifluoroethyl phosphate, bis(2,2,3,3-tetrafluoropropyl)2,2,2-trifluoroethyl phosphate, bis(2,2,2-trifluoroethyl)methyl phosphate, bis(2,2,2-trifluoroethyl)ethyl phosphate, bis(2,2,2-trifluoroethyl)2,2-difluoroethyl phosphate, bis(2,2,2-trifluoroethyl)2,2,3,3-tetrafluoropropyl phosphate, tributyl phosphate, tris(2,2,2-trifluoroethyl) phosphate, tris(1,1,1,3,3,3-hexafluoropropan-2-yl) phosphate, trioctyl phosphate, 2-phenylphenyldimethyl phosphate, 2-phenylphenyldiethyl phosphate, (2,2,2-trifluoroethyl) (2,2,3,3-tetrafluoropropyl)methyl phosphate, methyl 2-(dimethoxyphosphoryl) acetate, methyl 2-(dimethylphosphoryl) acetate, methyl 2-(diethoxyphosphoryl) acetate, methyl 2-(diethylphosphoryl) acetate, methyl methylenebisphosphonate, ethyl methylenebisphosphonte, methyl ethylenebisphosphonte, ethyl ethylenebisphosphonte, methyl butylenebisphosphonate, ethyl butylenebisphosphonate, 2-propynyl 2-(dimethoxyphosphoryl) acetate, 2-propynyl 2-(dimethylphosphoryl) acetate, 2-propynyl 2-(diethoxyphosphoryl) acetate, 2-propynyl 2-(diethylphosphoryl) acetate, tris(trimethylsilyl) phosphate, tris(triethylsilyl) phosphate, tris(trimethoxysilyl) phosphate, tris(trimethylsilyl) phosphite, tris(triethylsilyl) phosphite, tris(trimethoxysilyl) phosphite, and trimethylsilyl polyphosphate;
boron-containing compounds such as tris(trimethylsilyl) borate and tris(trimethoxysilyl) borate; and
silane compounds such as dimethoxyaluminoxytrimethoxysilane, diethoxyaluminoxytriethoxysilane, dipropoxyaluminoxytriethoxysilane, dibutoxyaluminoxytrimethoxysilane, dibutoxyaluminoxytriethoxysilane, titanium tetrakis(trimethylsiloxide), titanium tetrakis(triethylsiloxide), and tetramethylsilane.

One of these compounds may be used alone or two or more thereof may be used in any combination. These aids can improve the capacity retention characteristics and the cycle characteristics after high-temperature storage.

Preferred among these as the different aid are phosphorus-containing compounds, and preferred are tris(trimethylsilyl) phosphate and tris(trimethylsilyl) phosphite.

The different aid may be present in any amount that does not significantly impair the effects of the disclosure. The amount of the different aid is preferably 0.01% by mass or more and 5% by mass or less of 100% by mass of the electrolyte solution. The different aid in an amount within this range can easily sufficiently exhibit the effects thereof and can easily avoid a situation with impairment of battery characteristics such as high-load discharge characteristics. The amount of the different aid is more preferably 0.1% by mass or more, still more preferably 0.2% by mass or more, while more preferably 3% by mass or less, still more preferably 1% by mass or less.

The electrolyte solution may further contain as an additive any of a cyclic carboxylate, an acyclic carboxylate, an ether compound other than the compounds (1) and (2), a nitrogen-containing compound, a boron-containing compound, an organosilicon-containing compound, a fireproof agent (flame retardant), a surfactant, an additive for increasing the permittivity, an improver for cycle characteristics and rate characteristics, and a sulfonebased compound to the extent that the effects of the disclosure are not impaired.

Examples of the cyclic carboxylate include those having a carbon number of 3 to 12 in total in the structural formula. Specific examples thereof include gamma-butyrolactone, gamma-valerolactone, gammacaprolactone, epsilon-caprolactone, and 3-methyl-γ-butyrolactone. In order to improve the characteristics of an electrochemical device owing to improvement in the degree of dissociation of lithium ions, particularly preferred is gamma-butyrolactone.

In general, the cyclic carboxylate as an additive is preferably present in an amount of 0.1% by mass or more, more preferably 1% by mass or more, in 100% by mass of the solvent. The cyclic carboxylate in an amount within this range can easily improve the electric conductivity of the electrolyte solution, improving the large-current discharge characteristics of an electrochemical device. The amount of the cyclic carboxylate is also preferably 10% by mass or less, more preferably 5% by mass or less. Such an upper limit may allow the electrolyte solution to have a viscosity within an appropriate range, may make it possible to avoid a reduction in the electric conductivity, may reduce an increase in the resistance of the negative electrode, and may allow an electrochemical device to have large-current discharge characteristics within a favorable range.

The cyclic carboxylate to be suitably used may also be a fluorinated cyclic carboxylate (fluorine-containing lactone). Examples of the fluorine-containing lactone include fluorine-containing lactones represented by the following formula (C): 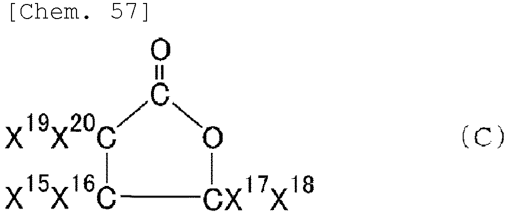 wherein X¹⁵ to X²⁰ are the same as or different from each other, and are each -H, -F, -Cl, -CH₃, or a fluorinated alkyl group; and at least one selected from X¹⁵ to X²⁰ is a fluorinated alkyl group.

Examples of the fluorinated alkyl group for X¹⁵ to X²⁰ include -CFH₂, -CF₂H, -CF₃, -CH₂CF₃, -CF₂CF₃, -CH₂CF₂CF₃, and -CF(CF₃)₂. In order to achieve high oxidation resistance and an effect of improving safety, -CH₂CF₃ and -CH₂CF₂CF₃ are preferred.

Only one of X¹⁵ to X²⁰ or a plurality thereof may be replaced by -H, -F, -Cl, -CH₃, or a fluorinated alkyl group as long as at least one selected from X¹⁵ to X²⁰ is a fluorinated alkyl group. In order to give good solubility of an electrolyte salt, the number of substituents is preferably 1 to 3, more preferably 1 or 2.

The substitution of the fluorinated alkyl group may be at any of the above sites. In order to give a good synthesizing yield, the substitution site is preferably X¹⁷ and/or X¹⁸. In particular, X¹⁷ or X¹⁸ is preferably a fluorinated alkyl group, especially -CH₂CF₃ or -CH₂CF₂CF₃. The substituent for X¹⁵ to X²⁰ other than the fluorinated alkyl group is -H, -F, -Cl, or CH₃. In order to give good solubility of an electrolyte salt, -H is preferred.

In addition to those represented by the above formula, the fluorine-containing lactone may also be a fluorine-containing lactone represented by the following formula (D): 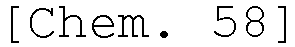 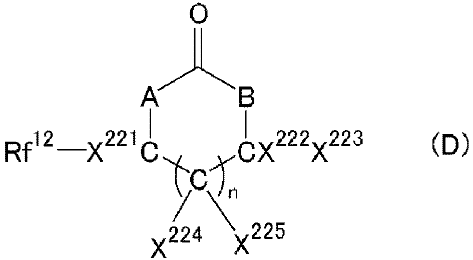 wherein one of A or B is CX²²⁶X²²⁷ (where X²²⁶ and X²²⁷ are the same as or different from each other, and are each -H, -F, -Cl, -CF₃, -CH₃, or an alkylene group in which a hydrogen atom is optionally replaced by a halogen atom and which optionally contains a hetero atom in the chain) and the other is an oxygen atom; Rf¹² is a fluorinated alkyl group optionally containing an ether bond or a fluorinated alkoxy group; X²²¹ and X²²² are the same as or different from each other, and are each -H, -F, -Cl, -CF₃, or CH₃; X²²³ to X²²⁵ are the same as or different from each other, and are each -H, -F, -Cl, or an alkyl group in which a hydrogen atom is optionally replaced by a halogen atom and which optionally contains a hetero atom in the chain; and n = 0 or 1.

A preferred example of the fluorine-containing lactone represented by the formula (D) is a 5-membered ring structure represented by the following formula (E): 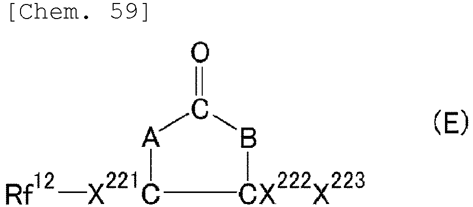 (wherein A, B, Rf¹², X²²¹, X²²², and X²²³ are defined as in the formula (D)) because it can be easily synthesized and can have good chemical stability. Further, in relation to the combination of A and B, fluorine-containing lactones represented by the following formula (F): 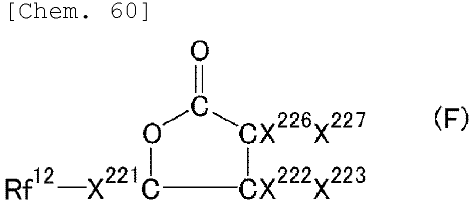 (wherein Rf¹², X²²¹, X²²², X²²³, X²²⁶, and X²²⁷ are defined as in the formula (D)) and fluorine-containing lactones represented by the following formula (G): 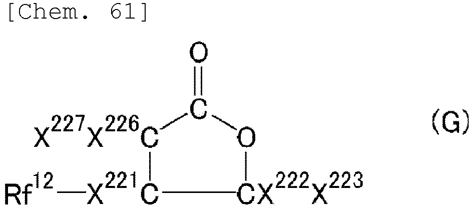 (wherein Rf¹², X²²¹, X²²², X²²³, X²²⁶, and X²²⁷ are defined as in the formula (D)) may be mentioned.

In order to particularly give excellent characteristics such as high permittivity and high withstand voltage, and to improve the characteristics of the electrolyte solution in the disclosure, for example, to give good solubility of an electrolyte salt and to reduce the internal resistance well, those represented by the following formulas: 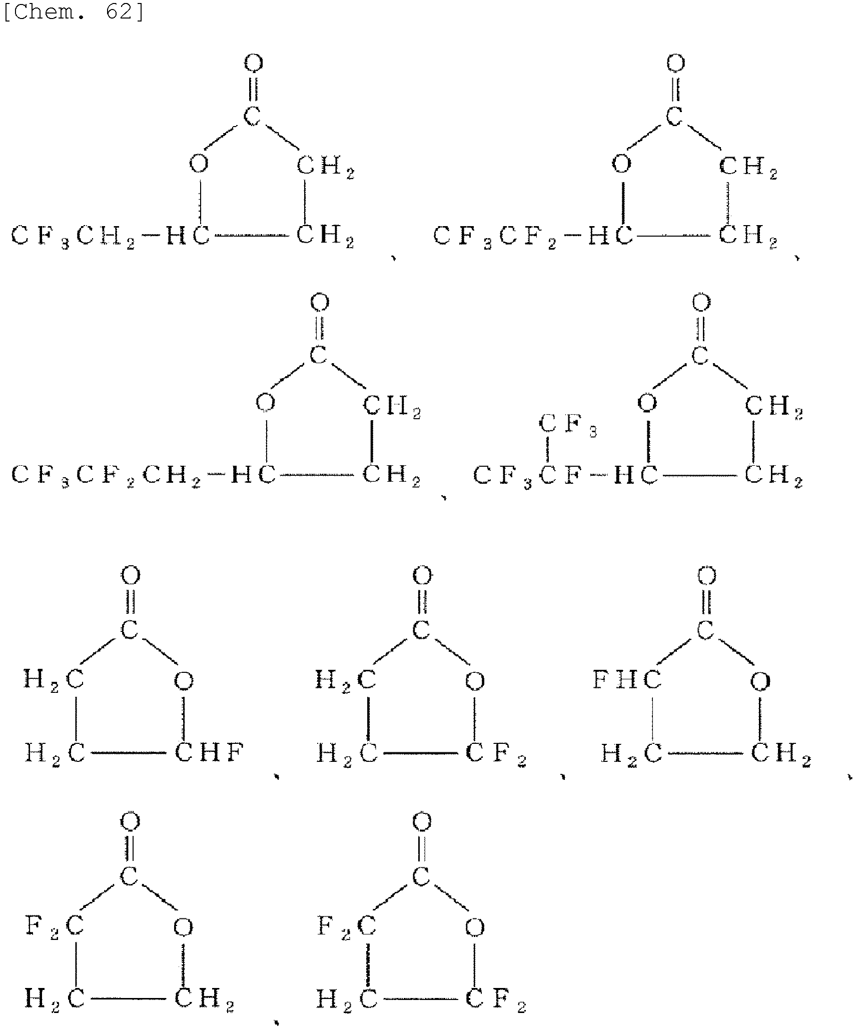 may be mentioned.

The presence of a fluorinated cyclic carboxylate can lead to, for example, effects of improving the ion conductivity, improving the safety, and improving the stability at high temperature.

Examples of the acyclic carboxylate include those having a carbon number of 3 to 7 in total in the structural formula thereof. Specific examples thereof include methyl acetate, ethyl acetate, n-propyl acetate, isopropyl acetate, n-butyl acetate, isobutyl acetate, t-butyl acetate, methyl propionate, ethyl propionate, n-propyl propionate, isobutyl propionate, n-butyl propionate, methyl butyrate, isobutyl propionate, t-butyl propionate, methyl butyrate, ethyl butyrate, n-propyl butyrate, isopropyl butyrate, methyl isobutyrate, ethyl isobutyrate, n-propyl isobutyrate, and isopropyl isobutyrate.

In order to improve the ion conductivity owing to viscosity reduction, preferred among these are methyl acetate, ethyl acetate, n-propyl acetate, n-butyl acetate, methyl propionate, ethyl propionate, n-propyl propionate, isopropyl propionate, methyl butyrate, and ethyl butyrate.

The ether compound is preferably a C2-C10 acyclic ether or a C3-C6 cyclic ether.

Examples of the C2-C10 acyclic ether include dimethyl ether, diethyl ether, di-n-butyl ether, dimethoxymethane, methoxyethoxymethane, diethoxymethane, dimethoxyethane, methoxyethoxyethane, diethoxyethane, ethylene glycol di-n-propyl ether, ethylene glycol di-n-butyl ether, diethylene glycol, diethylene glycol dimethyl ether, pentaethylene glycol, triethylene glycol dimethyl ether, triethylene glycol, tetraethylene glycol, tetraethylene glycol dimethyl ether, and diisopropyl ether.

Examples of the C3-C6 cyclic ether include 1,2-dioxane, 1,3-dioxane, 2-methyl-1,3-dioxane, 4-methyl-1,3-dioxane, 1,4-dioxane, metaformaldehyde, 2-methyl-1,3-dioxolane, 1,3-dioxolane, 4-methyl-1,3-dioxolane, 2-(trifluoroethyl)dioxolane, 2,2,-bis(trifluoromethyl)-1,3-dioxolane, and fluorinated compounds thereof. In order to achieve a high ability to solvate with lithium ions and improve the degree of ion dissociation, preferred are dimethoxymethane, diethoxymethane, ethoxymethoxymethane, ethylene glycol n-propyl ether, ethylene glycol di-n-butyl ether, diethylene glycol dimethyl ether, and crown ethers. In order to achieve low viscosity and to give a high ion conductivity, particularly preferred are dimethoxymethane, diethoxymethane, and ethoxymethoxymethane.

Examples of the nitrogen-containing compound include nitrile, fluorine-containing nitrile, carboxylic acid amide, fluorine-containing carboxylic acid amide, sulfonic acid amide, fluorine-containing sulfonic acid amide, acetamide, and formamide. Also, 1-methyl-2-pyrrolidinone, 1-methyl-2-piperidone, 3-methyl-2-oxazolidinone, 1,3-dimethyl-2-imidazolidinone, and N-methylsuccinimide may be used. The nitrile compounds represented by the formulas (1a), (1b), and (1c) are not included in the above nitrogen-containing compounds.

Examples of the boron-containing compound include borates such as trimethyl borate and triethyl borate, boric acid ethers, and alkyl borates.

Examples of the organosilicon-containing compound include (CH₃)₄-Si, (CH₃)₃-Si-Si(CH₃)₃, and silicone oil.

Examples of the fireproof agent (flame retardant) include organophosphates and phosphazene-based compounds. Examples of the organophosphates include fluorine-containing alkyl phosphates, non-fluorine-containing alkyl phosphates, and aryl phosphates. In order to achieve a flame-retardant effect even in a small amount, fluorine-containing alkyl phosphates are particularly preferred.

Examples of the phosphazene-based compounds include methoxypentafluorocyclotriphosphazene, phenoxypentafluorocyclotriphosphazene, dimethylaminopentafluorocyclotriphosphazene, diethylaminopentafluorocyclotriphosphazene, ethoxypentafluorocyclotriphosphazene, and ethoxyheptafluorocyclotetraphosphazene.

Specific examples of the fluorine-containing alkyl phosphates include fluorine-containing dialkyl phosphates disclosed in JP H11-233141 A, cyclic alkyl phosphates disclosed in JP H11-283669 A, and fluorine-containing trialkyl phosphates.

Preferred examples of the fireproof agent (flame retardant) include (CH₃O)₃P=O, (CF₃CH₂O)₃P=O, (HCF₂CH₂O)₃P=O, (CF₃CF₂CH₂)₃P=O, and (HCF₂CF₂CH₂)₃P=O.

The surfactant may be any of cationic surfactants, anionic surfactants, nonionic surfactants, and amphoteric surfactants. In order to give good cycle characteristics and rate characteristics, the surfactant is preferably one containing a fluorine atom.

Preferred examples of such a surfactant containing a fluorine atom include fluorine-containing carboxylic acid salts represented by the following formula (30):

Rf⁵COO⁻M⁺ (30)

(wherein Rf⁵ is a C3-C10 fluorine-containing alkyl group optionally containing an ether bond; M⁺ is Li⁺, Na⁺, K⁺, or NHR'₃⁺, wherein R's are the same as or different from each other, and are each H or a C1-C3 alkyl group), and fluorine-containing sulfonic acid salts represented by the following formula (40):

Rf⁶SO₃⁻M⁺ (40)

(wherein Rf⁶ is a C3-C10 fluorine-containing alkyl group optionally containing an ether bond; M⁺ is Li⁺, Na⁺, K⁺, or NHR'₃⁺, wherein R's are the same as or different from each other, and are each H or a C1-C3 alkyl group).

In order to reduce the surface tension of the electrolyte solution without impairing the charge and discharge cycle characteristics, the surfactant is preferably present in an amount of 0.01 to 2% by mass of the electrolyte solution.

Examples of the additive for increasing the permittivity include sulfolane, methylsulfolane, γ-butyrolactone, and γ-valerolactone.

Examples of the improver for cycle characteristics and rate characteristics include methyl acetate, ethyl acetate, tetrahydrofuran, and 1,4-dioxane.

The electrolyte solution may be combined with a polymer material and thereby formed into a gel-like (plasticized), gel electrolyte solution.

Examples of such a polymer material include conventionally known polyethylene oxide and polypropylene oxide, and modified products thereof (see JP H08-222270 A, JP 2002-100405 A); polyacrylate-based polymers, polyacrylonitrile, and fluororesins such as polyvinylidene fluoride and vinylidene fluoride-hexafluoropropylene copolymers (see JP H04-506726 T, JP H08-507407 T, JP H10-294131 A); and composites of any of these fluororesins and any hydrocarbon resin (see JP H11-35765 A, JP H11-86630 A). In particular, polyvinylidene fluoride or a vinylidene fluoride-hexafluoropropylene copolymer is preferably used as a polymer material for a gel electrolyte.

The electrolyte solution may also contain an ion conductive compound disclosed in Japanese Patent Application No. 2004-301934.

This ion conductive compound is an amorphous fluorine-containing polyether compound having a fluorine-containing group in a side chain and is represented by the following formula (101):

A-(D)-B (101)

wherein D is represented by the following formula (201):

-(D1)ₙ-(FAE)ₘ-(AE)ₚ-(Y)_{q}- (201)

[wherein D1 is an ether unit having a fluorine-containing ether group in a side chain and is represented by the following formula (2a): 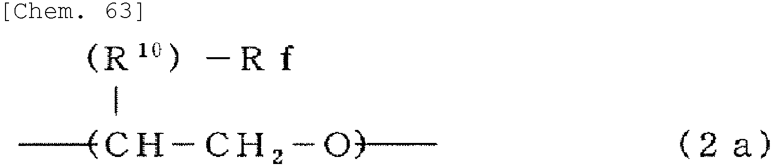 (wherein Rf is a fluorine-containing ether group optionally containing a crosslinkable functional group; and R¹⁰ is a group or a bond that links Rf and the main chain);
FAE is an ether unit having a fluorinated alkyl group in a side chain and is represented by the following formula (2b): 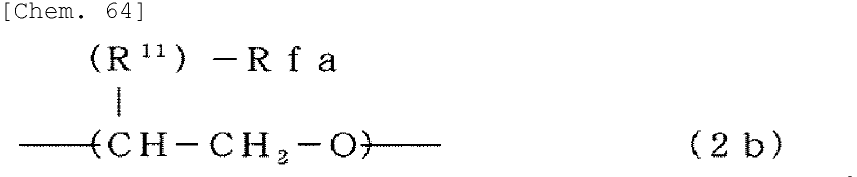 (wherein Rfa is a hydrogen atom or a fluorinated alkyl group optionally containing a crosslinkable functional group; and R¹¹ is a group or a bond that links Rfa and the main chain);
AE is an ether unit represented by the following formula (2c): 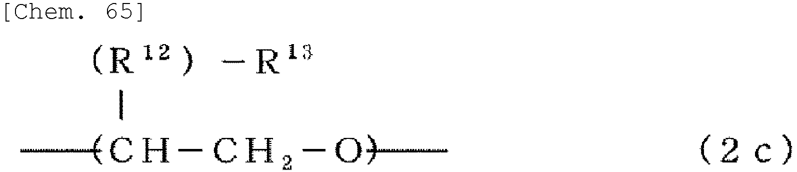 (wherein R¹³ is a hydrogen atom, an alkyl group optionally containing a crosslinkable functional group, an aliphatic cyclic hydrocarbon group optionally containing a crosslinkable functional group, or an aromatic hydrocarbon group optionally containing a crosslinkable functional group; and R¹² is a group or a bond that links R¹³ and the main chain);
Y is a unit containing at least one selected from the following formulas (2d-1) to (2d-3): 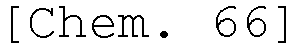 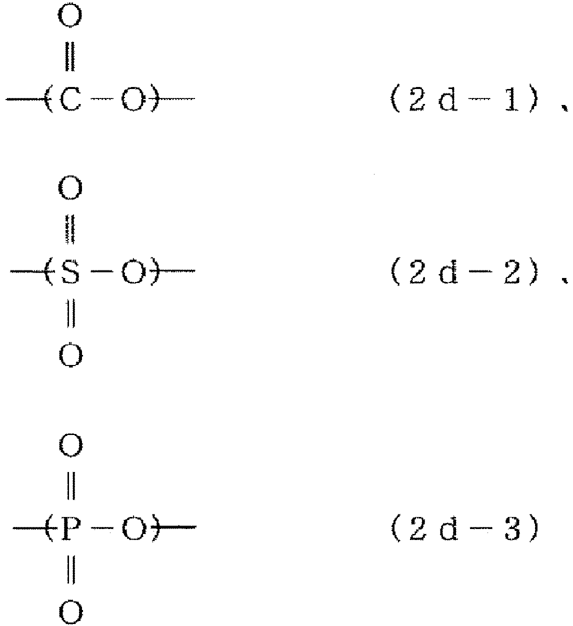
n is an integer of 0 to 200;
m is an integer of 0 to 200;
p is an integer of 0 to 10000;
q is an integer of 1 to 100;
n + m is not 0; and
the bonding order of D1, FAE, AE, and Y is not specified]; and
A and B are the same as or different from each other, and are each a hydrogen atom, an alkyl group optionally containing a fluorine atom and/or a crosslinkable functional group, a phenyl group optionally containing a fluorine atom and/or a crosslinkable functional group, a - COOH group, -OR (where R is a hydrogen atom or an alkyl group optionally containing a fluorine atom and/or a crosslinkable functional group), an ester group, or a carbonate group, and when an end of D is an oxygen atom, A and B are each none of a -COOH group, -OR, an ester group, and a carbonate group.

The electrolyte solution may contain a sulfone-based compound. Preferred as the sulfone-based compound are a C3-C6 cyclic sulfone and a C2-C6 acyclic sulfone. The number of sulfonyl groups in one molecule is preferably 1 or 2.

Examples of the cyclic sulfone include monosulfone compounds such as trimethylene sulfones, tetramethylene sulfones, and hexamethylene sulfones; disulfone compounds such as trimethylene disulfones, tetramethylene disulfones, and hexamethylene disulfones. In order to give good permittivity and viscosity, more preferred among these are tetramethylene sulfones, tetramethylene disulfones, hexamethylene sulfones, and hexamethylene disulfones, particularly preferred are tetramethylene sulfones (sulfolanes).

The sulfolanes are preferably sulfolane and/or sulfolane derivatives (hereinafter, also abbreviated as "sulfolanes" including sulfolane). The sulfolane derivatives are preferably those in which one or more hydrogen atoms binding to any carbon atom constituting the sulfolane ring is replaced by a fluorine atom or an alkyl group.

In order to achieve high ion conductivity and high input and output, preferred among these are 2-methylsulfolane, 3-methylsulfolane, 2-fluorosulfolane, 3-fluorosulfolane, 2,2-difluorosulfolane, 2,3-difluorosulfolane, 2,4-difluorosulfolane, 2,5-difluorosulfolane, 3,4-difluorosulfolane, 2-fluoro-3-methylsulfolane, 2-fluoro-2-methylsulfolane, 3-fluoro-3-methylsulfolane, 3-fluoro-2-methylsulfolane, 4-fluoro-3-methylsulfolane, 4-fluoro-2-methylsulfolane, 5-fluoro-3-methylsulfolane, 5-fluoro-2-methylsulfolane, 2-fluoromethylsulfolane, 3-fluoromethylsulfolane, 2-difluoromethylsulfolane, 3-difluoromethylsulfolane, 2-trifluoromethylsulfolane, 3-trifluoromethylsulfolane, 2-fluoro-3-(trifluoromethyl)sulfolane, 3-fluoro-3-(trifluoromethyl)sulfolane, 4-fluoro-3-(trifluoromethyl)sulfolane, 3-sulfolene, 5-fluoro-3-(trifluoromethyl)sulfolane, and the like.

Examples of the acyclic sulfone include dimethyl sulfone, ethyl methyl sulfone, diethyl sulfone, n-propyl methyl sulfone, n-propyl ethyl sulfone, di-n-propyl sulfone, isopropyl methyl sulfone, isopropyl ethyl sulfone, diisopropyl sulfone, n-butyl methyl sulfone, n-butyl ethyl sulfone, t-butyl methyl sulfone, t-butyl ethyl sulfone, monofluoromethyl methyl sulfone, difluoromethyl methyl sulfone, trifluoromethyl methyl sulfone, monofluoroethyl methyl sulfone, difluoroethyl methyl sulfone, trifluoroethyl methyl sulfone, pentafluoroethyl methyl sulfone, ethyl monofluoromethyl sulfone, ethyl difluoromethyl sulfone, ethyl trifluoromethyl sulfone, perfluoroethyl methyl sulfone, ethyl trifluoroethyl sulfone, ethyl pentafluoroethyl sulfone, di(trifluoroethyl)sulfone, perfluorodiethyl sulfone, fluoromethyl-n-propyl sulfone, difluoromethyl-n-propyl sulfone, trifluoromethyl-n-propyl sulfone, fluoromethyl isopropyl sulfone, difluoromethyl isopropyl sulfone, trifluoromethyl isopropyl sulfone, trifluoroethyl-n-propyl sulfone, trifluoroethyl isopropyl sulfone, pentafluoroethyl-n-propyl sulfone, pentafluoroethyl isopropyl sulfone, trifluoroethyl-n-butyl sulfone, trifluoroethyl-t-butyl sulfone, pentafluoroethyl-n-butyl sulfone, and pentafluoroethyl-t-butyl sulfone.

In order to achieve high ion conductivity and high input and output, preferred among these are dimethyl sulfone, ethyl methyl sulfone, diethyl sulfone, n-propyl methyl sulfone, isopropyl methyl sulfone, n-butyl methyl sulfone, t-butyl methyl sulfone, monofluoromethyl methyl sulfone, difluoromethyl methyl sulfone, trifluoromethyl methyl sulfone, monofluoroethyl methyl sulfone, difluoroethyl methyl sulfone, trifluoroethyl methyl sulfone, pentafluoroethyl methyl sulfone, ethyl monofluoromethyl sulfone, ethyl difluoromethyl sulfone, ethyl trifluoromethyl sulfone, ethyl trifluoroethyl sulfone, ethyl pentafluoroethyl sulfone, trifluoromethyl-n-propyl sulfone, trifluoromethyl isopropyl sulfone, trifluoroethyl-n-butyl sulfone, trifluoroethyl-t-butyl sulfone, trifluoromethyl-n-butyl sulfone, trifluoromethyl-t-butyl sulfone, and the like.

The sulfone-based compound may be present in any amount that does not significantly impair the effects of the disclosure. The amount is usually 0.3% by volume or more, preferably 0.5% by volume or more, more preferably 1% by volume or more, while usually 40% by volume or less, preferably 35% by volume or less, more preferably 30% by volume or less, in 100% by volume of the solvent. The sulfone-based compound in an amount within the above range can easily achieve an effect of improving the cycle characteristics and durability such as storage characteristics, can lead to an appropriate range of the viscosity of a non-aqueous electrolyte solution, can eliminate a reduction in electric conductivity, and can lead to appropriate ranges of the input and output characteristics and charge and discharge rate characteristics of a non-aqueous electrolyte secondary battery.

In order to improve the output characteristics, the electrolyte solution also preferably contains as an additive a compound (7) that includes at least one selected from the group consisting of lithium fluorophosphate salts (other than LiPF₆) and lithium salts containing a S=O group.

When the compound (7) is used as an additive, the above-described electrolyte salt is preferably a compound other than the compound (7).

Examples of the lithium fluorophosphate salts include lithium monofluorophosphate (LiPO₃F) and lithium difluorophosphate (LiPO₂F₂).

Examples of the lithium salts containing a S=O group include lithium monofluorosulfonate (FSO₃Li), lithium methyl sulfate (CH₃OSO₃Li), lithium ethyl sulfate (C₂H₅OSO₃Li), and lithium 2,2,2-trifluoroethyl sulfate.

Preferred among these as the compound (7) are LiPO₂F₂, FSO₃Li, and C₂H₅OSO₃Li.

The compound (7) is preferably present in an amount of 0.001 to 20% by mass, more preferably 0.01 to 15% by mass, still more preferably 0.1 to 10% by mass, particularly preferably 0.1 to 7% by mass, relative to the electrolyte solution.

The electrolyte solution may further contain a different additive, if necessary. Examples of the different additives include metal oxides and glass.

The electrolyte solution preferably contains 1 to 1000 ppm of hydrogen fluoride (HF). The presence of HF can promote formation of a film of the aforementioned additive. Too small an amount of HF tends to impair the ability to form a film on the negative electrode, impairing the characteristics of an electrochemical device. Too large an amount of HF tends to impair the oxidation resistance of the electrolyte solution due to the influence by HF. The composition of the disclosure, even when containing HF in an amount within the above range, causes no reduction in capacity recovery of an electrochemical device after high-temperature storage.

The amount of HF is more preferably 5 ppm or more, still more preferably 10 ppm or more, particularly preferably 20 ppm or more. The amount of HF is also more preferably 200 ppm or less, still more preferably 100 ppm or less, still further more preferably 80 ppm or less, particularly preferably 50 ppm or less.

The amount of HF can be determined by neutralization titration.

The electrolyte solution is preferably prepared by any method using the aforementioned components.

### <Separator>

The separator may be formed from any known material and may have any known shape as long as the resulting separator is stable to the electrolyte solution and is excellent in a liquid-retaining ability. The separator is preferably in the form of a porous sheet or nonwoven fabric which is formed from a material stable to the composition (electrolyte solution) of the disclosure, such as resin, glass fiber, or inorganic matter, and which has an excellent liquid-retaining ability.

Examples of the material of a resin or glass-fiber separator include polyolefins such as polyethylene and polypropylene, aromatic polyamide, polytetrafluoroethylene, polyether sulfone, and glass filters. One of these materials may be used alone or two or more thereof may be used in any combination at any ratio, for example, in the form of a polypropylene/polyethylene bilayer film or a polypropylene/polyethylene/polypropylene trilayer film. In order to achieve good permeability of the electrolyte solution and a good shut-down effect, the separator is preferably a porous sheet or nonwoven fabric formed from a polyolefin such as polyethylene or polypropylene.

The separator may have any thickness, and the thickness is usually 1 µm or greater, preferably 5 µm or greater, more preferably 8 µm or greater, while usually 50 µm or smaller, preferably 40 µm or smaller, more preferably 30 µm or smaller. The separator thinner than the above range may have poor insulation and mechanical strength. The separator thicker than the above range may cause not only poor battery performance such as poor rate characteristics but also a low energy density of the whole electrolyte battery.

The separator which is a porous one such as a porous sheet or a nonwoven fabric may have any porosity. The porosity is usually 20% or higher, preferably 35% or higher, more preferably 45% or higher, while usually 90% or lower, preferably 85% or lower, more preferably 75% or lower. The separator having a porosity lower than the above range tends to have high film resistance, causing poor rate characteristics. The separator having a porosity higher than the above range tends to have low mechanical strength, causing poor insulation.

The separator may also have any average pore size. The average pore size is usually 0.5 µm or smaller, preferably 0.2 µm or smaller, while usually 0.05 µm or larger. The separator having an average pore size larger than the above range may easily cause short circuits. The separator having an average pore size smaller than the above range may have high film resistance, causing poor rate characteristics.

Examples of the inorganic matter include oxides such as alumina and silicon dioxide, nitrides such as aluminum nitride and silicon nitride, and sulfates such as barium sulfate and calcium sulfate, each in the form of particles or fibers.

The separator is in the form of a thin film such as a nonwoven fabric, a woven fabric, or a microporous film. The thin film favorably has a pore size of 0.01 to 1 µm and a thickness of 5 to 50 µm. Instead of the above separate thin film, the separator may have a structure in which a composite porous layer containing particles of the above inorganic matter is disposed on a surface of one or each of the positive and negative electrodes using a resin binder. For example, alumina particles having a 90% particle size of smaller than 1 µm may be applied to the respective surfaces of the positive electrode with fluororesin used as a binder to form a porous layer.

### <Battery design>

The electrode group may be either a laminate structure including the above positive and negative electrode plates with the above separator in between, or a wound structure including the above positive and negative electrode plates in spiral with the above separator in between. The proportion of the volume of the electrode group in the battery internal volume (hereinafter, referred to as an electrode group proportion) is usually 40% or higher, preferably 50% or higher, while usually 90% or lower, preferably 80% or lower.

The electrode group proportion lower than the above range may cause a low battery capacity. The electrode group proportion higher than the above range may cause small void space in the battery. Thus, if the battery temperature rises to high temperature and thereby the components swell and the liquid fraction of the electrolyte solution exhibits high vapor pressure to raise the internal pressure, the battery characteristics such as charge and discharge repeatability and high-temperature storageability may be impaired and a gas-releasing valve for releasing the internal pressure toward the outside may be actuated.

The current collecting structure may be any structure. In order to more effectively improve the high-current-density charge and discharge performance, the current collecting structure is preferably a structure which reduces the resistances at wiring portions and jointing portions.

In an electrode group having the laminate structure, the metal core portions of the respective electrode layers are preferably bundled and welded to a terminal. If an electrode has a large area, the internal resistance is high. Thus, multiple terminals may preferably be disposed in the electrode so as to reduce the resistance. In an electrode group having the wound structure, multiple lead structures may be disposed on each of the positive electrode and the negative electrode and bundled to a terminal. This can reduce the internal resistance.

The external case may be made of any material that is stable to an electrolyte solution to be used. Specific examples thereof include metals such as nickel-plated steel plates, stainless steel, aluminum and aluminum alloys, and magnesium alloys, and a layered film (laminate film) of resin and aluminum foil. In order to reduce weight, a metal such as aluminum or an aluminum alloy or a laminate film is favorably used.

An external case made of metal may have a sealed-up structure formed by welding the metal by laser welding, resistance welding, or ultrasonic welding, or a caulking structure using the metal with a resin gasket in between. An external case made of a laminate film may have a sealed-up structure formed by hot-melting resin layers. In order to improve the sealability, a resin which is different from the resin of the laminate film may be disposed between the resin layers. Especially, in the case of forming a sealed-up structure by hot-melting the resin layers with current collecting terminals in between, metal and resin are to be bonded. Thus, the resin to be disposed between the resin layers is favorably a resin having a polar group or a modified resin having a polar group introduced therein.

The lithium-ion secondary battery may have any shape, such as a cylindrical shape, a square shape, a laminate shape, a coin shape, or a large-size shape. The shapes and the structures of the positive electrode, the negative electrode, and the separator may be changed in accordance with the shape of the battery.

In a preferred embodiment, the lithium-ion secondary battery includes a positive electrode, a negative electrode, and the aforementioned electrolyte solution, the positive electrode including a positive electrode current collector and a positive electrode active material layer containing a positive electrode active material, the positive electrode active material containing Mn. The lithium-ion secondary battery including a positive electrode active material layer that contains a positive electrode active material containing Mn can have much better high-temperature storage characteristics.

In order to provide a high-power lithium-ion secondary battery having a high energy density, preferred as the positive electrode active material containing Mn are LiMn_{1.5}Ni_{0.5}O₄, LiNi_{0.5}Co_{0.2}Mn_{0.3}O₂, and LiNi_{0.6}Co_{0.2}Mn_{0.2}O₂.

In order to provide a high-capacity lithium-ion secondary battery, the positive electrode active material containing Mn is preferably LiNi_{0.6}Co_{0.2}Mn_{0.2}O₂ or LiNi_{0.8}Co_{0.1}Mn_{0.1}O₂.

In order to further improve the high-temperature storage characteristics of the lithium-ion secondary battery, a portion in contact with the electrolyte solution among portions electrically coupled with the positive electrode current collector is also preferably formed from a valve metal or an alloy thereof. In particular, the external case of the battery and a portion that is electrically coupled with the positive electrode current collector and is in contact with the non-aqueous electrolyte solution among components accommodated in the external case of the battery, such as leads and a safety valve, are preferably formed from a valve metal or an alloy thereof. Stainless steel coated with a valve metal or an alloy thereof may also be used.

The positive electrode may be produced by the aforementioned method. An example of the production method is a method in which the positive electrode active material is mixed with the aforementioned binder, thickening agent, conductive material, solvent, and other components to form a slurry-like positive electrode mixture, and then this mixture is applied to the positive electrode current collector, dried, and pressed so as to be densified.

The structure of the negative electrode is as described above.

The lithium-ion secondary battery can be also used in the form of a module.

### <Electric double-layer capacitor>

As described above, the current collector foil and the electrode of the disclosure can be used in an electric double-layer capacitor.

The electric double-layer capacitor may include a positive electrode, a negative electrode, and an electrolyte solution.

The electrolyte solution of the electric double-layer capacitor contains a solvent and an electrolyte salt. The electrolyte solution may contain additives other than these.

The solvent and additives usable in the electrolyte solution for an electric double-layer capacitor are the same as those described for the electrolyte solution for a battery.

The electrolyte salt in the electrolyte solution for an electric double-layer capacitor is preferably an ammonium salt.

Examples of the ammonium salt include the following salts (IIa) to (IIe).

### (IIa) Tetraalkyl quaternary ammonium salts

Preferred examples thereof include tetraalkyl quaternary ammonium salts represented by the following formula (IIa): 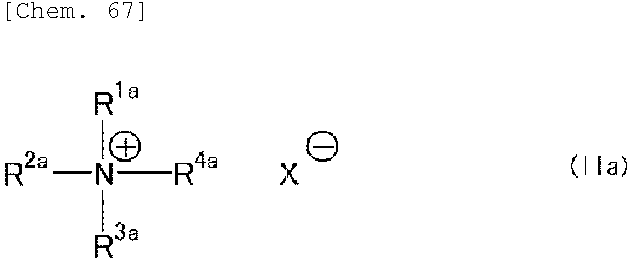 (wherein R^{1a}, R^{2a}, R^{3a}, and R^{4a} are the same as or different from each other, and are each a C1-C6 alkyl group optionally containing an ether bond; and X⁻ is an anion).

In order to improve the oxidation resistance, any or all of the hydrogen atoms in the ammonium salt are also preferably replaced by a fluorine atom and/or a C1-C4 fluorine-containing alkyl group.

Preferred specific examples thereof include
tetraalkyl quaternary ammonium salts represented by the following formula (IIa-1):

   [Chem. 68] (R^{1a})ₓ(R^{2a})_{y}N^{⊕} x^{⊖} (IIa - 1)

   (wherein R^{1a}, R^{2a}, and X⁻ are defined as described above; x and y are the same as or different from each other, and are each an integer of 0 to 4 with x + y = 4), and
alkyl ether group-containing trialkyl ammonium salts represented by the following formula (IIa-2): 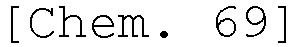 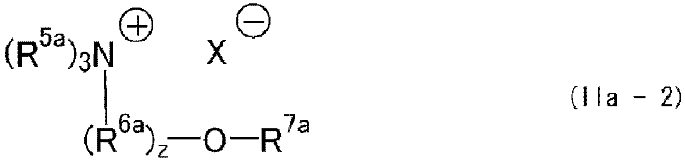 (wherein R^{5a} is a C1-C6 alkyl group; R^{6a} is a C1-C6 divalent hydrocarbon group; R^{7a} is a C1-C4 alkyl group; z is 1 or 2; and X⁻ is an anion) .

Introduction of an alkyl ether group enables reduction in viscosity.

The anion X⁻ may be either an inorganic anion or an organic anion. Examples of the inorganic anion include AlCl₄⁻, BF₄⁻, PF₆⁻, AsF₆⁻, TaF₆⁻, I⁻, and SbF₆⁻. Examples of the organic anion include a bisoxalatoborate anion, a difluorooxalatoborate anion, a tetrafluorooxalatophosphate anion, a difluorobisoxalatophosphate anion, CF₃COO⁻, CF₃SO₃⁻, (CF₃SO₂)₂N⁻, and (C₂F₅SO₂)₂N⁻.

In order to achieve good oxidation resistance and ionic dissociation, preferred are BF₄⁻, PF₆⁻, AsF₆⁻, and SbF₆⁻.

Preferred specific examples of the tetraalkyl quaternary ammonium salts to be used include Et₄NBF₄, Et₄NclO₄, Et₄NPF₆, Et₄NAsF₆, Et₄NSbF₆, Et₄NCF₃SO₃, Et₄N(CF₃SO₂)₂N, Et₄NC₄F₉SO₃, Et₃MeNBF₄, Et₃MeNClO₄, Et₃MeNPF₆, Et₃MeNAsF₆, Et₃MeNSbF₆, Et₃MeNCF₃SO₃, Et₃MeN(CF₃SO₂)₂N, and Et₃MeNC₄F₉SO₃. In particular, Et₄NBF₄, Et₄NPF₆, Et₄NSbF₆, Et₄NAsF₆, Et₃MeNBF₄, and an N,N-diethyl-N-methyl-N-(2-methoxyethyl)ammonium salt may be mentioned as examples.

### (IIb) Spirocyclic bipyrrolidinium salts

Preferred examples thereof include
spirocyclic bipyrrolidinium salts represented by the following formula (IIb-1): 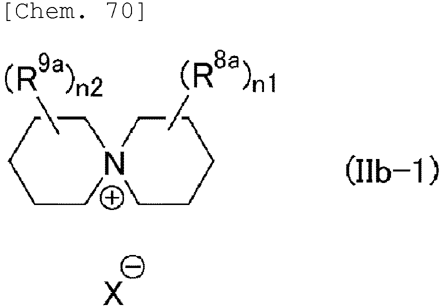 (wherein R^{8a} and R^{9a} are the same as or different from each other, and are each a C1-C4 alkyl group; X⁻ is an anion; n1 is an integer of 0 to 5; and n2 is an integer of 0 to 5),
spirocyclic bipyrrolidinium salts represented by the following formula (IIb-2): 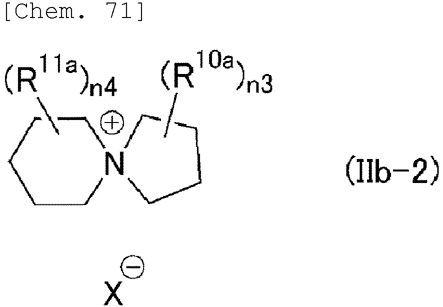 (wherein R^{10a} and R^{11a} are the same as or different from each other, and are each a C1-C4 alkyl group; X⁻ is an anion; n3 is an integer of 0 to 5; and n4 is an integer of 0 to 5), and
spirocyclic bipyrrolidinium salts represented by the following formula (IIb-3): 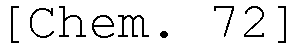 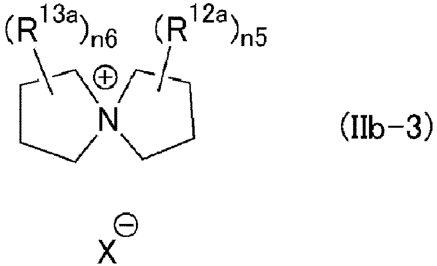 (wherein R^{12a} and R^{13a} are the same as or different from each other, and are each a C1-C4 alkyl group; X⁻ is an anion; n5 is an integer of 0 to 5; and n6 is an integer of 0 to 5). In order to improve the oxidation resistance, any or all of the hydrogen atoms in the spirocyclic bipyrrolidinium salt are also preferably replaced by a fluorine atom and/or a C1-C4 fluorine-containing alkyl group.

Preferred specific examples of the anion X⁻ are the same as those mentioned for the salts (IIa). In order to achieve good dissociation and a low internal resistance under high voltage, preferred among these is BF₄⁻, PF₆⁻, (CF₃SO₂)₂N-, or (C₂F₅SO₂)₂N-.

For example, those represented by the following formulas: 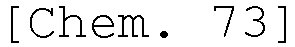 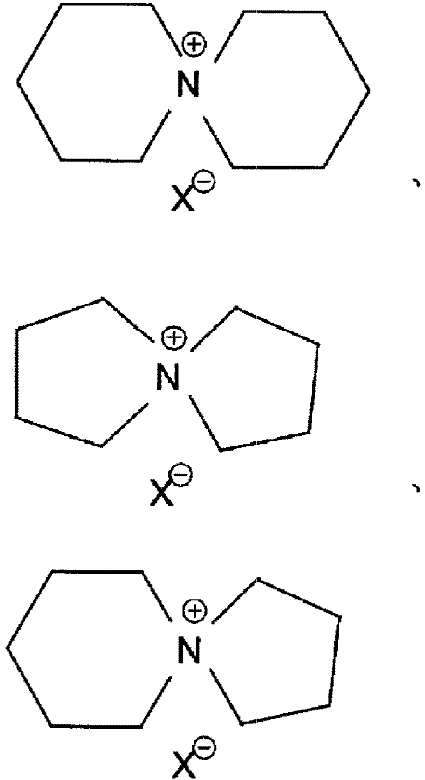 may be mentioned as preferred specific examples of the spirocyclic bipyrrolidinium salts.

These spirocyclic bipyrrolidinium salts are excellent in solubility in a solvent, oxidation resistance, and ion conductivity.

### (IIc) Imidazolium salts

Preferred examples thereof include imidazolium salts represented by the following formula (IIc): 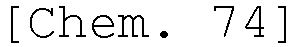 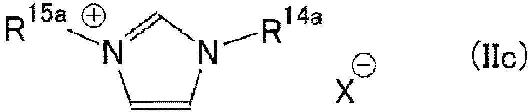 wherein R^{14a} and R^{15a} are the same as or different from each other, and are each a C1-C6 alkyl group; and X⁻ is an anion.

In order to improve the oxidation resistance, any or all of the hydrogen atoms in the imidazolium salt are also preferably replaced by a fluorine atom and/or a C1-C4 fluorine-containing alkyl group.

Preferred specific examples of the anion X⁻ are the same as those mentioned for the salts (IIa).

For example, those represented by the following formulas: 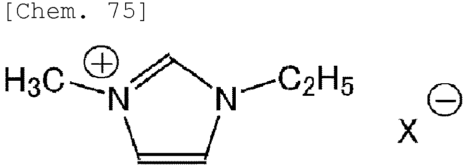 may be mentioned as preferred specific examples thereof.

This imidazolium salt is excellent in that it has low viscosity and good solubility.

### (IId) N-alkylpyridinium salts

Preferred examples thereof include N-alkylpyridinium salts represented by the following formula (IId): 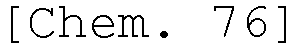 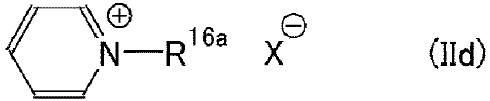 wherein R^{16a} is a C1-C6 alkyl group; and X⁻ is an anion. In order to improve the oxidation resistance, any or all of the hydrogen atoms in the N-alkylpyridinium salt are also preferably replaced by a fluorine atom and/or a C1-C4 fluorine-containing alkyl group.

Preferred specific examples of the anion X⁻ are the same as those mentioned for the salts (IIa).

For example, those represented by the following formulas: 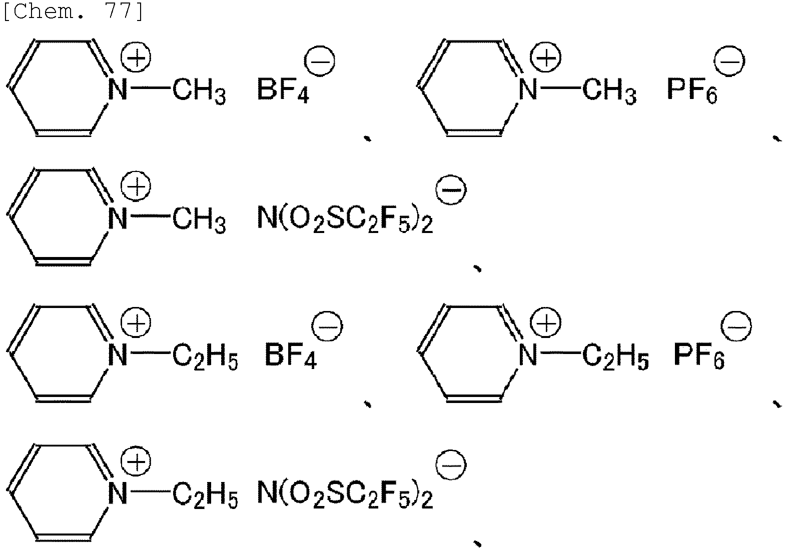 may be mentioned as preferred specific examples thereof.

These N-alkylpyridinium salts are excellent in that they have low viscosity and good solubility.

### (IIe) N,N-dialkylpyrrolidinium salts

Preferred examples thereof include N,N-dialkylpyrrolidinium salts represented by the following formula (IIe): 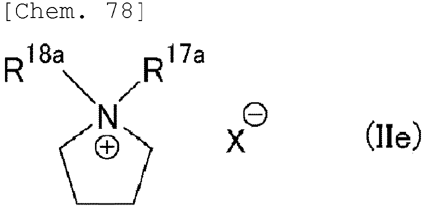 wherein R^{17a} and R^{18a} are the same as or different from each other, and are each a C1-C6 alkyl group; and X⁻ is an anion. In order to improve the oxidation resistance, any or all of the hydrogen atoms in the N,N-dialkylpyrrolidinium salt are also preferably replaced by a fluorine atom and/or a C1-C4 fluorine-containing alkyl group.

Preferred specific examples of the anion X⁻ are the same as those mentioned for the salts (IIa).

For example, those represented by the following formulas: 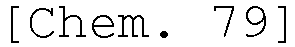 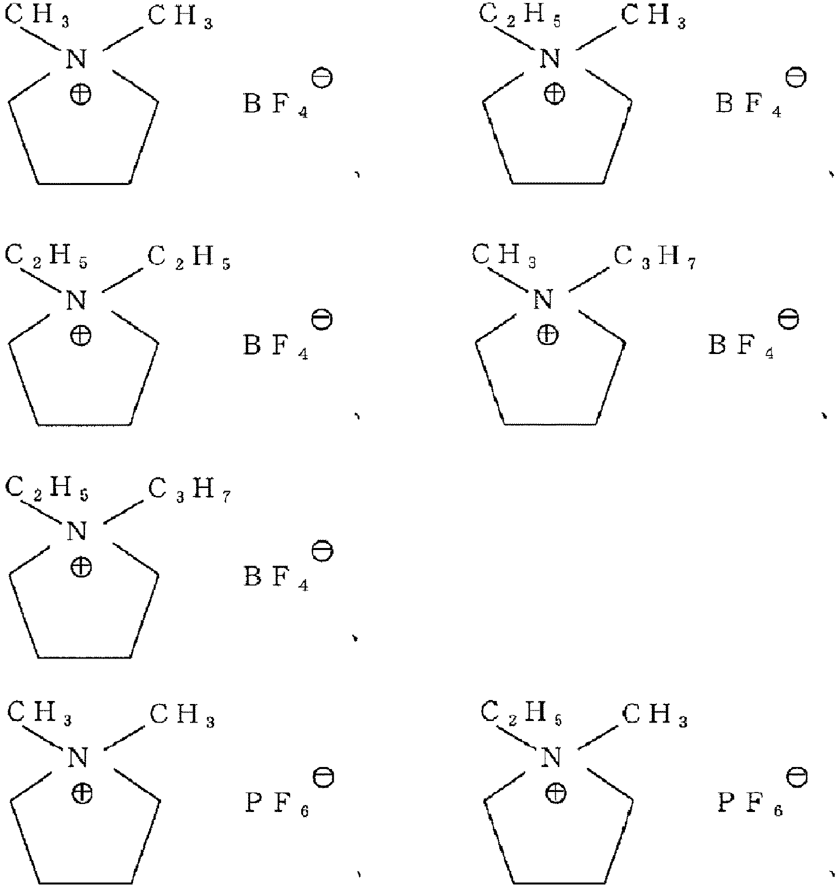 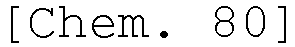 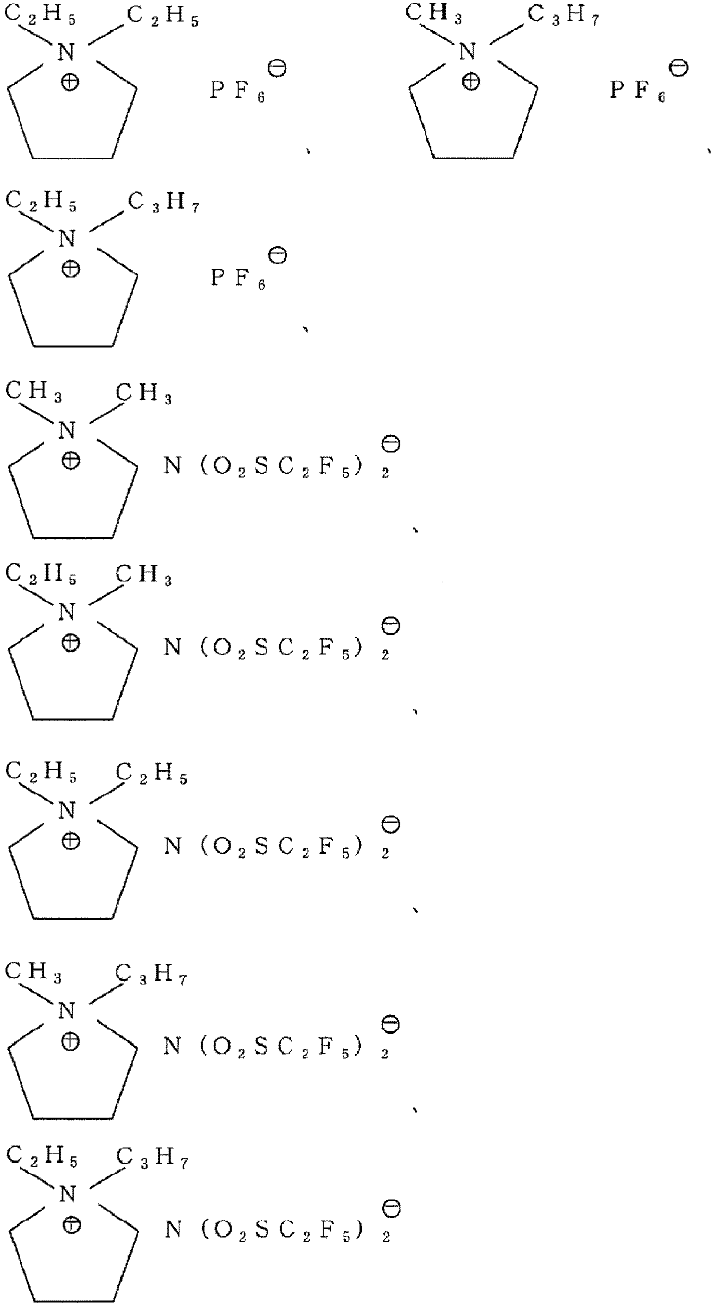 may be mentioned as preferred specific examples thereof.

These N,N-dialkylpyrrolidinium salts are excellent in that they have low viscosity and good solubility.

Preferred among these ammonium salts are those represented by the formula (IIa), (IIb), or (IIc) because they can have good solubility, oxidation resistance, and ion conductivity. More preferred are those represented by the following formulas: 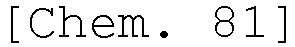 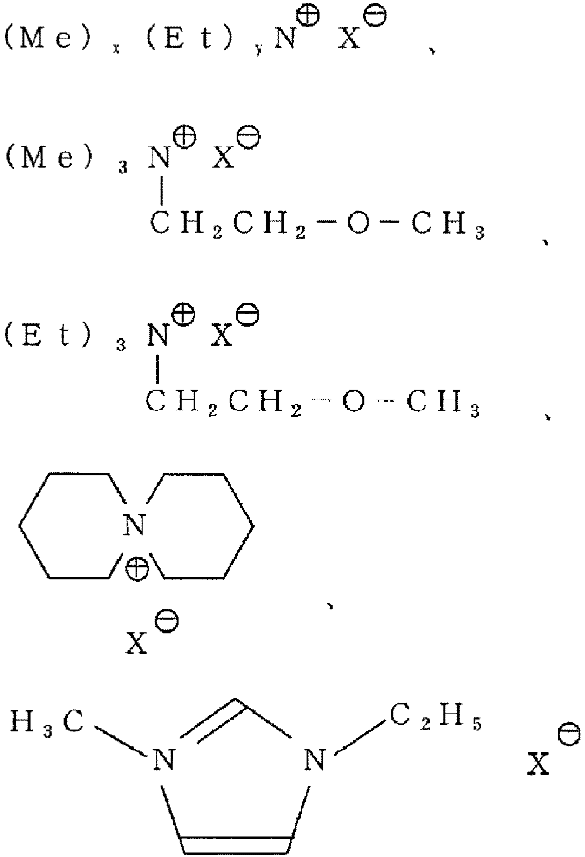 wherein Me is a methyl group; Et is an ethyl group; and X⁻, x, and y are defined as in the formula (IIa-1).

A lithium salt may be used as an electrolyte salt for an electric double-layer capacitor. Preferred examples thereof include LiPF₆, LiBF₄, LiN(FSO₂)₂, LiAsF₆, LiSbF₆, and LiN(SO₂C₂H₅)₂.

In order to further increase the capacity, a magnesium salt may be used. Preferred examples of the magnesium salt include Mg(ClO₄)₂ and Mg(OOC₂H₅)₂.

The ammonium salt serving as an electrolyte salt is preferably used at a concentration of 0.7 mol/L or higher. The ammonium salt at a concentration lower than 0.7 mol/L may cause not only poor low-temperature characteristics but also high initial internal resistance. The concentration of the electrolyte salt is more preferably 0.9 mol/L or higher.

In order to give good low-temperature characteristics, the upper limit of the concentration is preferably 2.0 mol/L or lower, more preferably 1.5 mol/L or lower.

When the ammonium salt is triethyl methyl ammonium tetrafluoroborate (TEMABF₄), the concentration is preferably 0.7 to 1.5 mol/L to give excellent low-temperature characteristics.

When the ammonium salt is spirobipyrrolidinium tetrafluoroborate (SBPBF₄) the concentration is preferably 0.7 to 2.0 mol/L.

At least one of the positive electrode or the negative electrode is a polarizable electrode in the electric double-layer capacitor. Examples of the polarizable electrode and a non-polarizable electrode include the following electrodes specifically disclosed in JP H09-7896 A.

The polarizable electrode mainly containing activated carbon to be used in the disclosure preferably contains inactivated carbon having a large specific surface area and a conductive material, such as carbon black, providing electronic conductivity. The polarizable electrode may be formed by a variety of methods. For example, a polarizable electrode including activated carbon and carbon black can be produced by mixing activated carbon powder, carbon black, and phenolic resin, press-molding the mixture, and then sintering and activating the mixture in an inert gas atmosphere and water vapor atmosphere. Preferably, this polarizable electrode is bonded to a current collector using a conductive adhesive, for example.

Alternatively, a polarizable electrode can also be formed by kneading activated carbon powder, carbon black, and a binder in the presence of an alcohol, forming the mixture into a sheet, and then drying the sheet. The binder to be used may be polytetrafluoroethylene, for example. Alternatively, a polarizable electrode integrated with a current collector can be produced by mixing activated carbon powder, carbon black, a binder, and a solvent to form slurry, applying this slurry to metal foil of a current collector, and then drying the slurry.

The electric double-layer capacitor may have polarizable electrodes mainly containing activated carbon as the respective electrodes. Still, the electric double-layer capacitor may have a structure in which a non-polarizable electrode is used on one side. Examples of such a structure include a structure in which a positive electrode mainly containing an electrode active material such as a metal oxide is combined with a polarizable negative electrode mainly containing activated carbon; and a structure in which a negative electrode mainly containing a carbon material that can reversibly occlude and release lithium ions or a negative electrode of lithium metal or lithium alloy is combined with a polarizable positive electrode mainly containing activated carbon.

In place of or in combination with activated carbon, any carbonaceous material may be used, such as carbon black, graphite, expanded graphite, porous carbon, carbon nanotube, carbon nanohorn, and Ketjenblack.

The non-polarizable electrode is preferably an electrode mainly containing a carbon material that can reversibly occlude and release lithium ions, with this carbon material made to occlude lithium ions in advance. In this case, the electrolyte used is a lithium salt. The electric double-layer capacitor having such a structure can achieve a much higher withstand voltage exceeding 4 V.

The solvent used in preparation of the slurry in production of electrodes is preferably one that dissolves a binder. In accordance with the type of a binder, the solvent is appropriately selected from N-methylpyrrolidone, dimethyl formamide, toluene, xylene, isophorone, methyl ethyl ketone, ethyl acetate, methyl acetate, dimethyl phthalate, ethanol, methanol, butanol, and water.

Examples of the activated carbon used for the polarizable electrode include phenol resin-type activated carbon, coconut shell-type activated carbon, and petroleum coke-type activated carbon. In order to achieve a large capacity, petroleum coke-type activated carbon or phenol resin-type activated carbon is preferably used. Examples of methods of activating the activated carbon include steam activation and molten KOH activation. In order to achieve a larger capacity, activated carbon prepared by molten KOH activation is preferably used.

Preferred examples of the conductive agent used for the polarizable electrode include carbon black, Ketjenblack, acetylene black, natural graphite, artificial graphite, metal fiber, conductive titanium oxide, and ruthenium oxide. In order to achieve good conductivity (i.e., low internal resistance), and because too large an amount thereof may lead to a decreased capacity of the product, the amount of the conductive agent such as carbon black used for the polarizable electrode is preferably 1 to 50% by mass in the sum of the amounts of the activated carbon and the conductive agent.

In order to provide an electric double-layer capacitor having a large capacity and low internal resistance, the activated carbon used for the polarizable electrode preferably has an average particle size of 20 µm or smaller and a specific surface area of 1500 to 3000 m²/g. Preferred examples of the carbon material for providing an electrode mainly containing a carbon material that can reversibly occlude and release lithium ions include natural graphite, artificial graphite, graphitized mesocarbon microsphere, graphitized whisker, vapor-grown carbon fiber, sintered furfuryl alcohol resin, and sintered novolak resin.

The current collector may be any chemically and electrochemically corrosion-resistant one. Preferred examples of the current collector used for the polarizable electrode mainly containing activated carbon include stainless steel, aluminum, titanium, and tantalum. Particularly preferred materials in terms of the characteristics and cost of the resulting electric double-layer capacitor are stainless steel and aluminum. Preferred examples of the current collector used for the electrode mainly containing a carbon material that can reversibly occlude and release lithium ions include stainless steel, copper, and nickel.

Examples of methods of allowing the carbon material that can reversibly occlude and release lithium ions to occlude lithium ions in advance include: (1) a method of mixing powdery lithium with a carbon material that can reversibly occlude and release lithium ions; (2) a method of placing lithium foil on an electrode including a carbon material that can reversibly occlude and release lithium ions and a binder so as to bring the lithium foil to be in electrical contact with the electrode, immersing this electrode in an electrolyte solution containing a lithium salt dissolved therein so as to ionize the lithium, and allowing the carbon material to take in the lithium ions; and (3) a method of placing an electrode including a carbon material that can reversibly occlude and release lithium ions and a binder on the minus side and placing a lithium metal on the plus side, immersing the electrodes in a non-aqueous electrolyte solution containing a lithium salt as an electrolyte, and supplying a current so that the carbon material is allowed to electrochemically take in the ionized lithium.

Examples of known electric double-layer capacitors include wound electric double-layer capacitors, laminated electric double-layer capacitors, and coin-type electric double-layer capacitors. The electric double-layer capacitor may also be any of these types.

For example, a wound electric double-layer capacitor may be assembled as follows. A positive electrode and a negative electrode each of which includes a laminate (electrode) of a current collector and an electrode layer are wound with a separator in between to provide a wound element. This wound element is put into a case made of aluminum, for example. The case is filled with an electrolyte solution, preferably a non-aqueous electrolyte solution, and then sealed with a rubber sealant.

A separator formed from a conventionally known material and having a conventionally known structure may be used. Examples thereof include polyethylene porous membranes, and nonwoven fabric of polytetrafluoroethylene, polypropylene fiber, glass fiber, or cellulose fiber.

In accordance with any known method, the electric double-layer capacitor may be prepared in the form of a laminated electric double-layer capacitor in which sheet-like positive and negative electrodes are stacked with an electrolyte solution and a separator in between or a coin-type electric double-layer capacitor in which positive and negative electrodes are fixed in a coin shape by a gasket with an electrolyte solution and a separator in between.

### EXAMPLES

The disclosure is described with reference to examples, but the disclosure is not intended to be limited by these examples.

### <Preparation of treatment liquid>

Treatment agent A: 1,1'-difluoro-2,2-bipyridinium bis(tetrafluoroborate) or treatment agent B: an IF₅-pyridine-HF complex, as a surface-treating agent, was mixed with acetonitrile as a solvent, whereby a treatment liquid was prepared. The mixing ratio was 3 mg of the surface-treating agent to 1 ml (786 mg) of acetonitrile.

In the tables below, the treatment liquid containing the treatment agent A is denoted as Treatment agent A solution and the treatment liquid containing the treatment agent B is denoted as Treatment agent B solution.

### <Production of positive electrode>

First, 85% by mass of LiNi_{0.5}Mn_{1.5}O₄ (LNMO) serving as a positive electrode active material, 10% by mass of Super C45 (carbon black) serving as a conductive aid, and 5% by mass of carboxymethyl cellulose (CMC) serving as a binder were mixed in distilled water to form slurry. The resulting slurry was applied to one surface of 20-µm-thick aluminum foil with a conductive aid applied thereto in advance, and dried. The workpiece was then roll-pressed using a press and cut to provide a circular piece including an active material layer having a diameter of 12 mm. This piece was used as a positive electrode.

### <Production of negative electrode>

First, 97.3 parts by mass of a carbonaceous material (graphite) as a negative electrode active material was mixed with 1.2 parts by mass of an aqueous dispersion of sodium carboxymethyl cellulose (concentration of sodium carboxymethyl cellulose: 1% by mass) and 1.5 parts by mass of an aqueous dispersion of styrene-butadiene rubber (concentration of styrene-butadiene rubber: 50% by mass) respectively serving as a thickening agent and a binder. They were mixed using a disperser to form slurry. The resulting slurry was applied to 10-um-thick copper foil and dried. The workpiece was rolled using a press and cut to provide a circular piece including an active material layer having a diameter of 12 mm. This piece was used as a negative electrode.

### <Preparation of electrolyte solution>

LiN(CF₃SO₂)₂ (LiTFSI) was added to a mixture of ethylene carbonate (EC) and dimethyl carbonate (DMC) (volume ratio = 50:50) such that the concentration of LiN(CF₃SO₂)₂ was 1.0 mol/L, whereby an electrolyte solution was prepared.

### (Production of coin-type lithium-ion secondary battery)

The positive electrode and the negative electrode were placed to face each other with a 20-um-thick porous polyethylene film (separator) in between. The electrolyte solution was filled thereinto and the electrolyte solution was made to sufficiently permeate into the components such as the separator. The workpiece was then sealed, precharged, and aged, whereby a lithium-ion secondary battery was produced.

### <EDX measurement>

The percentage (atom%) of fluorine was measured by energy dispersive X-ray spectroscopy using a scanning electron microscope (SEM-EDX).

### <Cycle characteristics test>

The coin-type lithium-ion secondary battery was subjected to constant current/constant voltage charge (hereinafter, referred to as CC/CV charge) (0.1 C cut off) to 4.95 V at a current corresponding to 1 C at 20°C, and then discharged to 3.5 V at a constant current of 1 C. This was counted as one cycle, and the discharge capacity at the third cycle was used to determine the initial discharge capacity. Here, 1 C means the current value at which the reference capacity of the battery is discharged in one hour. For example, 0.2 C means 1/5 of this current value. The cycles were again performed, and the discharge capacity after the 1000th cycle was determined. The ratio of the discharge capacity after the 1000th cycle to the initial discharge capacity was determined. This value was defined as the cycle retention (%). (Discharge capacity after 1000th cycle)/(Initial discharge capacity) × 100 = Cycle retention (%)

### <Experimental examples of positive electrode active materials>

### (Examples A1 to A4)

LNMO was immersed in the treatment liquid at 25°C under the conditions shown in Table 1, taken out of the treatment liquid, and vacuum-dried. Then, the percentage (atom%) of the fluorine on the surface of the LNMO was measured by the EDX measurement. Based on the difference in mass of the active material before and after the treatment, the amount (% by mass) of the unreacted/reacted surface-treating agent remaining on the surface of the LNMO was calculated.

A positive electrode was produced by the above-described method, except that the treated LNMO was used as the positive electrode active material, and combined with lithium metal foil, whereby a coin-type lithium-ion secondary battery was produced. Then, the cycle characteristics test was performed. The amount (% by mass) of the surface-treating agent in the positive electrode mixture was calculated from the amounts of the materials charged.

### (Comparative Example A1)

A lithium-ion secondary battery was produced as in Examples A1 to A4, except that LNMO was treated with fluorine gas, and evaluated. The treatment with fluorine gas was performed by the same method as that in "(3) Fluorination of positive electrode active material" in Example 3 in Patent Literature 1.

### (Comparative Example A2)

A lithium-ion secondary battery was produced as in Examples A1 to A4, except that the positive electrode active material was not treated, and evaluated.

**[Table 1]**

| | Treatment method | Percentage of fluorine | Amount of surface-treating agent remaining on LNMO surface | Amount of surface-treating agent in positive electrode mixture | Cycle retention |
|---|---|---|---|---|---|
| Example A1 | Treatment agent A solution for 10 min | 3.50% | 0.20% | 0.17% | 55% |
| Example A2 | Treatment agent A solution for 30 min | 3.90% | 0.22% | 0.19% | 63% |
| Example A3 | Treatment agent B solution for 10 min | 3.38% | 0.19% | 0.16% | 54% |
| Example A4 | Treatment agent B solution for 30 min | 3.62% | 0.20% | 0.17% | 61% |
| Comparative Example A1 | Fluorine gas | 3.20% | 0% | 0% | 50% |
| Comparative Example A2 | - (No treatment) | 0% | 0% | 0% | 49% |

### <Experimental examples of current collector foils>

### (Examples B1 and B2)

Aluminum foil was immersed in the treatment liquid at 25°C under the conditions shown in Table 2, taken out of the treatment liquid, and vacuum-dried. The percentages (atom%) of fluorine and aluminum on the surface of the aluminum foil were measured by the EDX measurement.

Using the treated aluminum foil, the CV measurement was performed. For the CV measurement, a three-electrode cell containing an electrolyte solution was produced, in which the aluminum foil was used as a working electrode and metallic lithium was used as a reference electrode and a counter electrode. The measurement was performed 10 cycles at 20°C, at a scanning speed of 0.1 mV/sec, and within a scanning range of 3.5 V to 5 V.

The percentages (atom%) of fluorine and aluminum on the surface of the aluminum foil after the CV measurement were measured by the EDX measurement.

### (Comparative Example B1)

A lithium-ion secondary battery was produced as in Examples B1 and B2, except that aluminum foil was treated with fluorine gas, and evaluated. The treatment with fluorine gas was performed by the same method as that in "(1) Preparation of fluorinated aluminum foil" in Example 1 in Patent Literature 1.

### (Comparative Example B2)

A lithium-ion secondary battery was produced as in Examples B1 and B2, except that the aluminum foil was not treated, and evaluated.

**[Table 2]**

| | Treatment method | Before cycles | | After cycles | |
|---|---|---|---|---|---|
| | | Al | F | Al | F |
| Example B1 | Treatment agent A solution for 10 min | 89.2% | 1.1% | 88.8% | 0.9% |
| Example B2 | Treatment agent B solution for 10 min | 89.2% | 1.1% | 88.6% | 10% |
| Comparative Example B1 | Fluorine gas | 89.4% | 0.9% | 85.1% | 1.4% |
| Comparative Example B2 | - (No treatment) | 88.2% | 0% | 82.8% | 1.8% |

### <Experimental examples of positive electrodes>

### (Examples C1 to C6)

The positive electrode was immersed in the treatment liquid under the conditions shown in Table 3, taken out of the treatment liquid, and vacuum-dried. The percentage (atom%) of fluorine on the surface of the positive electrode was measured by the EDX measurement.

The positive electrode was combined with lithium metal foil, whereby a coin-type lithium-ion secondary battery was produced. Then, the cycle characteristics test was performed.

### (Comparative Example C1)

A lithium-ion secondary battery was produced as in Examples C1 to C6, except that the positive electrode was treated with fluorine gas, and evaluated. The treatment with fluorine gas was performed by the same method as that in "(1) Preparation of fluorinated aluminum foil" in Example 1 in Patent Literature 1.

### (Comparative Example C2)

A lithium-ion secondary battery was produced as in Examples C1 to C6, except that the positive electrode was not treated, and evaluated.

**[Table 3]**

| | Treatment method | Percentage of fluorine | Cycle retention |
|---|---|---|---|
| Example C1 | Treatment agent A solution for 10 min | 3.47% | 55% |
| Example C2 | Treatment agent A solution for 30 min | 3.86% | 60% |
| Example C3 | Treatment agent A solution for 60 min | 4.20% | 68% |
| Example C4 | Treatment agent B solution for 10 min | 3.45% | 52% |
| Example C5 | Treatment agent B solution for 30 min | 3.82% | 58% |
| Example C6 | Treatment agent B solution for 60 min | 4.18% | 66% |
| Comparative Example C1 | Fluorine gas | 4.12% | 50% |
| Comparative Example C2 | - (No treatment) | 0% | 49% |

### <Experimental examples of negative electrode active materials>

### (Examples D1 to D4)

Graphite was immersed in the treatment liquid at 25°C under the conditions shown in Table 4, taken out of the treatment liquid, and vacuum-dried. Then, the percentage (atom%) of the fluorine on the surface of the graphite was measured by the EDX measurement. Based on the difference in mass of the graphite before and after the treatment, the amount (% by mass) of the unreacted/reacted surface-treating agent remaining on the surface of the graphite was calculated.

A negative electrode was produced by the above-described method, except that the treated graphite was used as the negative electrode active material, and combined with the positive electrode, whereby a coin-type lithium-ion secondary battery was produced. Then, the cycle characteristics test was performed. The amount (% by mass) of the surface-treating agent in the negative electrode mixture was calculated from the amounts of the materials charged.

### (Comparative Example D1)

A lithium-ion secondary battery was produced as in Examples D1 to D4, except that the graphite was treated with fluorine gas, and evaluated. The treatment with fluorine gas was performed by the same method as that in "(3) Fluorination of positive electrode active material" in Example 3 in Patent Literature 1, except that graphite was the treatment target.

### (Comparative Example D2)

A lithium-ion secondary battery was produced as in Examples D1 to D4, except that graphite was not treated, and evaluated.

**[Table 4]**

| | Treatment method | Percentage of fluorine | Amount of surface-treating agent remaining on graphite surface | Amount of surface-treating agent in negative electrode mixture | Cycle retention |
|---|---|---|---|---|---|
| Example D1 | Treatment agent A solution for 30 min | 3.90% | 0.20% | 0.19% | 60% |
| Example D2 | Treatment agent A solution for 60 min | 5.70% | 0.29% | 0.28% | 62% |
| Example D3 | Treatment agent B solution for 30 min | 3.62% | 0.19% | 0.18% | 58% |
| Example D4 | Treatment agent B solution for 60 min | 5.40% | 0.28% | 0.27% | 61% |
| Comparative Example D1 | Fluorine gas | 3.20% | 0% | 0% | 50% |
| Comparative Example D2 | - (No treatment) | 0% | 0% | 0% | 49% |

### <Experimental examples of conductive aids>

### (Examples E1 to E4)

Multi-walled carbon nanotube (CNT) was immersed in the treatment liquid at 25°C under the conditions shown in Table 5, taken out of the treatment liquid, and vacuum-dried. Then, the percentage (atom%) of the fluorine on the surface of the multi-walled carbon nanotube was measured by the EDX measurement. Based on the difference in mass of the multi-walled carbon nanotube before and after the treatment, the amount (% by mass) of the unreacted/reacted surface-treating agent remaining on the surface of the multi-walled carbon nanotube was calculated.

A positive electrode was produced by the above-described method, except that the treated multi-walled carbon nanotube was used as the conductive aid, and combined with the negative electrode, whereby a coin-type lithium-ion secondary battery was produced. Then, the cycle characteristics test was performed. The amount (% by mass) of the surface-treating agent in the positive electrode mixture was calculated from the amounts of the materials charged.

### (Comparative Example E1)

A lithium-ion secondary battery was produced as in Examples E1 to E4, except that the multi-walled carbon nanotube was treated with fluorine gas, and evaluated. The treatment with fluorine gas was performed by the same method as that in "(3) Fluorination of positive electrode active material" in Example 3 in Patent Literature 1, except that multi-walled carbon nanotube was the treatment target.

### (Comparative Example E2)

A lithium-ion secondary battery was produced as in Examples E1 to E4, except that the multi-walled carbon nanotube was not treated, and evaluated.

**[Table 5]**

| | Treatment method | Percentage of fluorine | Amount of surface-treating agent remaining on multi-walled CNT surface | Amount of surface-treating agent in positive electrode mixture | Cycle retention |
|---|---|---|---|---|---|
| Example E1 | Treatment agent A solution for 30 min | 4.10% | 0.19% | 0.01% | 59% |
| Example E2 | Treatment agent A solution for 60 min | 6.20% | 0.29% | 0.02% | 62% |
| Example E3 | Treatment agent B solution for 30 min | 3.80% | 0.18% | 0.01% | 57% |
| Example E4 | Treatment agent B solution for 60 min | 5.90% | 0.28% | 0.01% | 59% |
| Comparative Example E1 | Fluorine gas | 3.60% | 0% | 0% | 51% |
| Comparative Example E2 | - (No treatment) | 0% | 0% | 0% | 50% |

## Claims

1. A surface-treating agent for an electrode material, comprising a pyridine skeleton and fluorine.

2. The surface-treating agent for an electrode material according to claim 1, which is an N-fluoropyridinium compound or a fluorinated halogen-pyridine-HF complex.

3. The surface-treating agent for an electrode material according to claim 2,
wherein the N-fluoropyridinium compound is 1,1'-difluoro-2,2'-bipyridinium bis(tetrafluoroborate) and the fluorinated halogen-pyridine-HF complex is an IF₅-pyridine-HF complex.

4. The surface-treating agent for an electrode material according to any one of claims 1 to 3, which is used for a positive electrode active material, a current collector foil, a negative electrode active material, a conductive aid, or an electrode.

5. A positive electrode active material treated with the surface-treating agent for an electrode material according to any one of claims 1 to 4.

6. A current collector foil treated with the surface-treating agent for an electrode material according to any one of claims 1 to 4.

7. A negative electrode active material treated with the surface-treating agent for an electrode material according to any one of claims 1 to 4.

8. A conductive aid treated with the surface-treating agent for an electrode material according to any one of claims 1 to 4.

9. An electrode comprising at least one selected from the group consisting of the positive electrode active material according to claim 5, the current collector foil according to claim 6, and the conductive aid according to claim 8.

10. An electrode comprising at least one selected from the group consisting of the negative electrode active material according to claim 7, the current collector foil according to claim 6, and the conductive aid according to claim 8.

11. A battery comprising the electrode according to claim 9 or 10.

12. A method for producing the positive electrode active material according to claim 5, the method comprising
bringing a positive electrode active material into contact with a solution containing the surface-treating agent for an electrode material according to any one of claims 1 to 4.

13. A method for producing the current collector foil according to claim 6, the method comprising
bringing a current collector foil into contact with a solution containing the surface-treating agent for an electrode material according to any one of claims 1 to 4.

14. A method for producing the negative electrode active material according to claim 7, the method comprising
bringing a negative electrode active material into contact with a solution containing the surface-treating agent for an electrode material according to any one of claims 1 to 4.

15. A method for producing the conductive aid according to claim 8, the method comprising
bringing a conductive aid into contact with a solution containing the surface-treating agent for an electrode material according to any one of claims 1 to 4.

16. A method for producing an electrode, the method comprising
bringing an electrode including a current collector foil and a positive electrode active material or a negative electrode active material into contact with a solution containing the surface-treating agent for an electrode material according to any one of claims 1 to 4.
